# FASCICULE DE BREVET EUROPEEN

(11) **EP 2 250 270 B1**
(45) Date de publication et mention de la délivrance du brevet: **19.08.2015**
(21) Numéro de dépôt: 09709170.6
(22) Date de dépôt: 05.02.2009
(51) Int. Cl.: C12N 15/81

(54) **PROCÉDÉ D'INTÉGRATION CIBLÉE DE MULTICOPIES D'UN GÈNE D'INTÉRÊT DANS UNE SOUCHE DE YARROWIA**
VERFAHREN ZUR GEZIELTEN INTEGRATION MEHRERER KOPIEN EINES INTERESSIERENDEN GENS IN EINEN YARROWIA-STAMM
METHOD FOR THE TARGETED INTEGRATION OF MULTIPLE COPIES OF A GENE OF INTEREST IN A YARROWIA STRAIN

(30) Priorité: 05.02.2008 FR 0850736
(43) Date de publication de la demande: 17.11.2010
(73) Titulaire: Institut National de la Recherche Agronomique, 75007 Paris (FR)
(72) Inventeur: NICAUD, Jean-Marc, 78190 Trappes (FR); FUDALEJ, Franck, 92500 Rueil-Malmaison (FR); NEUVEGLISE, Cécile, 78610 Auffargis (FR); BECKERICH, Jean-Marie, 92340 Bourg-la-Reine (FR)
(74) Mandataire: Regimbeau
(86) Numéro de dépôt international: PCT/EP2009/051332
(87) Numéro de publication internationale: WO 2009/098263

(56) Documents cités:
- EP-A- 0 138 508
- MADZAK C ET AL: "Heterologous Protein Expression and Secretion in the Non-conventional Yeast Yarrowia lipolytica: A Review" JOURNAL OF BIOTECHNOLOGY, ELSEVIER SCIENCE PUBLISHERS, AMSTERDAM, NL, vol. 109, no. 1-2, 1 avril 2004 (2004-04-01), pages 63-81, XP002995176 ISSN: 0168-1656
- BORDES ET AL: "A new recombinant protein expression system for high-throughput screening in the yeast Yarrowia lipolytica" JOURNAL OF MICROBIOLOGICAL METHODS, ELSEVIER, AMSTERDAM, NL, vol. 70, no. 3, 25 août 2007 (2007-08-25), pages 493-502, XP022212058 ISSN: 0167-7012
- JURETZEK T ET AL: "VECTORS FOR GENE EXPRESSION AND AMPLIFICATION IN THE YEAST XARROWIA LIPOLYTICA" YEAST, CHICHESTER, SUSSEX, GB, vol. 18, no. 2, 30 janvier 2001 (2001-01-30), pages 97-113, XP008025954 ISSN: 0749-503X
- FICKERS P ET AL: "New disruption cassettes for rapid gene disruption and marker rescue in the yeast Yarrowia lipolytica" JOURNAL OF MICROBIOLOGICAL METHODS, ELSEVIER, AMSTERDAM, NL, vol. 55, no. 3, 1 décembre 2003 (2003-12-01), pages 727-737, XP002336154 ISSN: 0167-7012 cité dans la demande

## Description

La présente invention concerne un procédé d'intégration ciblée d'au moins trois copies d'un gène d'intérêt dans le génome d'une souche de *Yarrowia* comprenant les étapes de (a) mise en culture d'une souche de *Yarrowia,* laquelle souche comprend une délétion parmi au moins trois délétions choisies parmi Ura 3-302, Leu 2-270, Gut 2-744 et Ade 2-844, le phénotype associé à chacune de ces délétions correspondant à une auxotrophie pour ladite souche, indépendamment l'un de l'autre des au moins trois gènes; (b) transformation de ladite souche de *Yarrowia* ainsi obtenue avec au moins trois vecteurs recombinants qui comprennent des marqueurs de sélection permettant, pour ladite souche de *Yarrowia,* la complémentation de l'auxotrophie résultant de chacune desdites délétions; et (c) sélection sur un milieu minimal, des levures ayant intégré lesdits au moins trois vecteurs recombinants. La présente invention comprend également un procédé de production de polypeptide d'intérêt mettant en oeuvre un tel procédé ainsi qu'un procédé d'obtention de souche de *Yarrowia* modifiée comprenant une délétion parmi au moins trois gènes, le phénotype associé à chacune de ces délétions correspondant à une auxotrophie ou à un caractère dominant pour ladite souche.

La levure *Yarrowia lipolytica* est aujourd'hui de plus en plus employée comme hôte d'expression de gènes d'intérêts. Cette levure présente en effet la spécificité de produire et de sécréter avec une grande efficacité dans le milieu de culture des protéines de haut poids moléculaire, comme les protéases alcalines, les protéases acides ou la RNase, lesdites protéines pouvant alors être facilement récupérées à partir du milieu de culture. En vue de permettre l'expression de ces gènes d'intérêt, différentes technologies ont été développées précocement comme celles décrites notamment dans le brevet EP 0 138 508 B1. ou EP 0 220 864 B1. Ces différentes technologies ont en commun d'utiliser des vecteurs dits « intégratifs » qui permettent l'insertion d'un segment d'ADN portant le gène d'intérêt dans l'ADN chromosomique, lequel gène d'intérêt est exprimé par la suite. Pour autant, aucune de ces technologies n'a pu satisfaire complètement les impératifs de la production industrielle, à savoir un rendement et une stabilité dans le temps importants.En vue de mieux satisfaire ces impératifs industriels, de nouvelles technologies ont été développées.

Ainsi, la demande PCT WO 00/12729 décrit ainsi une technologie qui utilise des vecteurs intégratifs comprenant une séquence d'intérêt flanquée de séquences zéta correspondant aux LTRs du rétrotransposon Yltl de *Yarrowia lipolytica.* Ces vecteurs intégratifs, lorsqu'ils sont utilisés dans des souches de *Yarrowia dipolytica* dépourvues de séquences zéta, permettent des intégrations multicopies et aléatoires dans le génome desdites souches Cette technologie particulière a notamment été utilisée pour la production de la lipase extracellulaire acidorésistante LIP2 (voir demande PCT WO 01/83773).

Toutefois, il est apparu que les souches de *Yarrowia lipolytica* transformées par LIP2 présentaient pour la majorité d'entre elles une médiocre stabilité génétique, généralement inférieure à 50%. De surcroît, ces souches transformées étant obtenues à la suite d'événements d'intégration aléatoires, le suivi de la stabilité génétique des souches par séquençage des sites d'insertion est extrêmement fastidieux.

A ces problèmes de stabilité des souches obtenues et de difficulté à identifier le site d'intégration précis de la séquence hexogène s'ajoutent des difficultés complémentaires associées à l'absence de souches de *Yarrowia lipolytica* déficientes pour de multiples marqueurs de sélection du fait de l'instabilité de ces souches.

Parmi les méthodes d'intégration de cassettes d'expressions, on peut citer celles mettant en oeuvre les méthodes de transformation homologue, mais ces dernières utilisent peu de marqueur (LEU2, URA3, LYS5) et peu de souches multimarquées et avec des délétions permettant une forte diminution des taux de conversion. Les souches ont souvent des marqueurs génomiques correspondant à des mutations, peu de marqueur correspondant a des délétions. Les seul marqueurs génomiques connus avec des délétions sont : ura3-302 (délétion de 695 pb), ura3-41 (délétion de 41 pb), leu2-270 (délétion StuI de 681 pb). MADZAK et al. (J. of Biotech., vol.109, p :63-8T, 2004) décrit ainsi les marqueurs ura3-302 et leu2-270.

Parmi les vecteurs, on peut citer ceux qui permettent une intégration ciblée, mais en intégrant la partie plasmidique (i.e., intégration des plasmides a une plateforme pBR322 Madzak et al, Fems yeast research, vol. 5, p : 635-646, 2005) ou zeta (Bordes et al., J Microbiol Methods Vol. 70, p. 493-502, 2007). Il existe des vecteurs d'autoclonages pour l'intégration et l'amplification. Mais celles ci sont dispersées et aléatoires.

Pour la production de protéine thérapeutique il ne faut pas d'ADN plasmidique et il faut une intégration ciblée pour identifier l'insertion et vérifier sa séquence. Il faut aussi pouvoir augmenter le nombre de copie du gène pour augmenter l'expression.

Les inventeurs ont conçu une méthode qui permet une intégration ciblée grâce à des « vecteurs d'intégration », qui ont été construits pour libérer la cassette d'intégration (site rare 1) et éliminer la partie vecteur, qui ont des site rares pour introduire les marqueurs de sélection rapidement (site rare 2) et pour cloner les cassettes d'expression (site rare 3). Des combinaisons de vecteurs d'intégration avec différente combinaison de locus/marqueur.

Les inventeurs ont également :
- optimisé des conditions de transformation pour augmenter les taux de transformation, notamment pour les marqueurs ADE2 et GUT2 ; et
- réalisé l'association de l'intégration et d'un phénotype pour identifier rapidement les clones intéressant ayant intégré au locus désiré les cassettes d'intégration.

Ainsi, les inventeurs ont réalisé ici une méthode d'intégration d'une cassette d'expression à un locus qui permet d'introduire en même temps un nouveau marqueur de sélection (eg, nouveau marqueur génomique, nouvelle auxotrophie) pour permettre une nouvelle intégration avec une nouvelle cassette d'intégration possédant le marqueur de sélection correspondant.

Due à la possibilité de bonne efficacité de transformation, de faible taux de conversion, de crible d'identification des insertions (lipase pour LIP2, marron pour ADE2, SUC- pour URA3), il est ainsi possible de réaliser la cointégration (transformer avec deux cassettes d'intégration). Ceci permet de diminuer le temps nécessaire pour l'obtention de la souche de production. De mêmes, l'insertion à des sites définies permet une caractérisation rapide des souches de production. La souche finale étant prototrophe

La souche finale n'exprime plus les protéines majeurs secrétées (protéase AEP, lipase Lip2p), elle présente des délétions stables, une faible lyse cellulaire et un surnageant propre (peu de protéines sécrétées).

Les inventeurs ont mis en évidence également la possibilité d'obtenir avec ces marqueurs excisables, l'excision générale des marqueurs permettant de régénérer les marqueurs et ainsi de pouvoir à nouveau intégrer de nouvelles copies.

Pour l'obtention de souches multi-marquées, on peut introduire un marqueur génomique par différentes méthodes.

Par exemple, la méthode appelée POP IN/ POP OUT utilisée chez *Yarrowia lipolytica* pour la construction des marqueurs leu2-270, ura3-302 et xpr2-322 décrits dans la revue de G. Barth et al. : Yarrowia lipolytica. in: Nonconvéntional Yeasts in Biotechnology A Handbook (Wolf, K., Ed.), Vol. 1, 1996, pp. 313-388. Springer-Verlag. Elle consiste à intégrer un vecteur possédant une délétion du gène au locus puis à sélectionner l'excision de ce vecteur et d'identifier un clone qui par recombinaison à éliminé le gène sauvage et conservé le gène muté. C'est une méthode longue, peu efficace et qui nécessite aussi l'utilisation d'un marqueur permettant une contre sélection. Par exemple, la contre sélection des clones Ura+ avec le 5FOA. Cette contre sélection peut introduire des mutations secondaires.

On peut aussi utiliser la méthode SEP (Maftahi M., Nicaud J-M., Gaillardin C. ,1996. Sticky-end polymerase chain reaction method for systematic gene disruption in Saccharomyces cerevisiae. Yeast, vol. 12, p:859-868) qui a été adaptée chez *Yarrowia lipolytica* pour la disruption successive des gènes POX (Wang H. J., Le Dali M-T, Waché Y, Belin J-M, Gaillardin C, Nicaud J-M ,1999. Evaluation of Acyl CoA oxidase (Aox) isozymes function in the n-alkanes-assimilating yeast Yarrowia lipolytica. J. Bacteriol., vol. 181, p.5140-5148). Cette méthode est plus rapide, mais nécessite toujours l'utilisation d'un marqueur permettant une contre sélection.

1 On peut aussi utiliser la méthode SEP/cre développée par Fickers et collaborateurs (Fickers P., Le Dall M.T., Gaillardin C. Thonart P. Nicaud J-M., New disruption cassettes for rapid gene disruption and marker rescue in the yeast Yarrowia lipolytica. J. Microbiol. Methods vol. 55, p:727-737, 2003). C'est une méthode rapide et qui ne nécessite pas l'utilisation d'un marqueur permettant une contre sélection. Cette méthode consiste a 1) sélectionner le gène d'intérêt que l'on veut déléter, 2) construire une cassette de disruption par PCR (« Polymerase Chain Reaction) ou par clonage, 3) d'introduire un marqueur de sélection contenant de part et d'autre une séquence de recombinaison (avantageusement une séquence loxP ou loxR ou dérivée) permettant une recombinaison entre elle pour l'élimination du marqueur (avantageusement une séquence de type loxP qui permet la recombinaison sous l'action de la recombinase cre), 4) de sélectionner les souches avec le gène d'intérêt délété (transformation et sélection des transformants) et de vérifier la disruption, 5) de transformer avec un vecteur permettant l'expression de la recombinase (avantageusement la recombinase cre qui permet la recombinaison des séquences loxP/loxR et l'élimination du marqueur) 6) l'isolement d'un clone présentant la délétion du gène d'intérêt et ayant perdu le plasmide d'expression de la recombinase. Mais il faut optimiser les délétions et les marqueurs afin de minimiser les conversions géniques au cours de la transformation.

Il résulte de ce qui précède qu'il n'existe pas de technologies de transformation permettant d'intégrer, spécifiquement et stablement dans le génome de *Yarrowia lipolytica,* la séquence d'un gène d'intérêt de sorte d'obtenir une expression importante de celui-ci.

Les inventeurs ont maintenant mis en évidence que la stabilité des souches de *Yarrowia* obtenues par transformation était fortement dépendante du nombre de copies intégrées. Ainsi, une souche présentant 8 copies intégrées du gène LIP2 présente 70% de stabilité, laquelle stabilité monte à 98% pour 7 copies et à 100% pour 6 copies.

De même, les inventeurs ont réussi à développer de nouvelles souches viables de *Yarrowia lipolytica* présentant une auxotrophie et, le cas échéant, un caractère dominant associée à au moins trois marqueurs de sélection distincts, notamment choisis parmi les marqueurs de sélection Ura3, Leu2, Gut2 et Ade2. Les souches développées et viables présentent en outre de larges délétions (Ura3-302, Leu2-270, Gut2-744 et Ade2-884), avec notamment les nouvelles délétions Gut2-744 et Ade2-844, lesquelles dlétions permettent de limiter considérablement la fréquence de conversion génique (cf. exemples).

L'utilisation de telles souches dans des procédés d'intégration, en particulier ciblée, permet d'obtenir beaucoup plus rapidement et simplement des souches transformées comprenant de multiples intégrations d'un gène d'intérêt.

Finalement, les inventeurs ont pu développer un nouveau procédé de transformation de souches de *Yarrowia lipolytica* permettant d'obtenir des souches transformées stables et présentant un niveau intéressant de production d'un polypeptide d'intérêt.

### Objet de l'invention

L'objet de l'invention est de remédier aux inconvénients de l'art antérieur et d'obtenir rapidement des souches surproductrices d'un gène ou dé gènes d'intérêt(s) pour la production de protéines hétérologues et de pourvoir rapidement vérifier les cassettes d'expressions et caractériser la souche de production.

On utilise comme microorganismes des souches possédant des marqueurs de sélection permettant un niveau faible de conversion et une grande efficacité de transformation.

On utilise un ensemble de vecteurs permettant la construction de vecteurs contenant les cassettes d'expression et de vecteurs contenant les cassettes d'intégration.

On utilisé également des méthodes rapides d'identification des transformants contenants l'intégration des cassettes d'intégrations aux loci définis.

Finalement, la méthode selon l'invention permet la production d'une protéine hétérologue.

De manière plus détaillée, invention propose un ensemble de souches, de vecteurs, de marqueurs de sélection, de tests de phénotype et de caractérisation de l'insertion des cassettes d'insertion pour l'obtention rapide des souches surproductrices d'un gène ou de gènes d'intérêt(s) pour la production de protéines hétérologues et de pouvoir rapidement vérifier les cassettes d'expressions et caractériser la souche de production qui comprend :
(a) Des souches réceptrices contenant des délétions de gènes conférant une auxotrophie ou un défaut de croissance sur certains milieux;
(b) Un ensemble de vecteurs de clonage permettant la construction de « vecteurs d'expression » qui contiennent les cassettes d'expression; et un ensemble de « vecteurs d'intégration » permettant la construction de vecteurs d'intégration ;
(c) Des méthodes d'obtention des transformants, d'identification des transformants contenants l'intégration des cassettes d'intégration au locus définis ; et
(d) Une méthode de production de la protéine hétérologue.
En conséquence, un premier objet de l'invention concerne un procédé d'intégration ciblée d'au moins trois copies d'un gène d'intérêt dans le génome d'une souche de *Yarrowia* comprenant les étapes de :
a) mise en culture d'une souche de *Yarrowia* comprenant au moins trois délétions choisies parmi Ura3-302, Leu2-270, Gut2-744 et Ade2-844, le phénotype associé à chacune de ces délétions correspond à une auxotrophie pour ladite souche, indépendamment l'un de l'autre de ces au moins trois gènes ;
b) transformation de ladite souche de *Yarrowia* auxotrophe avec au moins trois vecteurs recombinants qui comprennent des marqueurs de sélection permettant, pour ladite souche de *Yarrowia,* la complémentation de l'auxotrophie résultant de chacune desdites délétions, lesquels vecteurs recombinants comprenant chacun:
   i) la séquence dudit gène d'intérêt,
   ii) un marqueur de sélèction, et
   iii) deux séquences d'ADN encadrant la séquence dudit gène d'intérêt et ledit marqueur de sélection, lesquelles deux séquences d'ADN sont homologues aux séquences correspondant aux extrémités du site d'intégration ciblé dans le génome de ladite souche de *Yarrowia* de sorte de permettre l'intégration ciblée dudit vecteur recombinant par recombinaison homologue ;
   sélection sur un milieu minimal, des levures ayant intégré lesdits au moins trois vecteurs recombinants.

Il est aussi décrit un procédé d'intégration ciblée et caractérisé en ce que les gènes présentant une délétion associés à un phénotype d'auxotrophie ou de caractère dominant choisis parmi :
- pour les marqueurs d'auxotrophie, les gènes URA3, LEU2, GUT2, ADE2, HIS et LYS5 ; et
- pour les caractères dominants, le gène de résistance à l'hygromycine HYG4, et les gènes MDR3, KanMX, HPH et Tn5ble.

Dans un mode de réalisation préféré, ladite souche comprend en outre au moins une délétion d'un gène associé à un caractère dominant choisi parmi le gène de résistance à l'hygromycine HPH (HYG), et les gènes MDR3. KanMX, et Tn5ble, le gène HYG étant le plus préféré.

Dans un mode de réalisation préféré, l'étape b), le site d'intégration ciblé dans le génome de ladite souche de *Yarrowia* est choisi parmi les gènes URA3, LEU2, ADE2, LIP2, LIP7, LIP8, AXP, GUT2 et XPR2.

Avantageusement, la délétion URA3 correspond à la délétion Ura3-302 bien connue de l'homme du métier et notamment décrite dans MADZAK *et al.* (2004).

Avantageusement, la délétion LEU2 correspond à la délétion Leu2-270 également bien connue de l'homme du métier et notamment décrite dans MADZAK *et al.* (2004). Avantageusement, la délétion GUT2 correspond à la délétion Gut2-744 décrite dans les exemples. Gut2-744 correspond à une délétion complète de l'ORF du gène GUT2 (SEQ ID NO :4), laquelle délétion est présente dans les souches mutantes FF-Lug (CNCM-3911) et FF-luga (CNCM-3913). Après excision, il reste sur le génome de *Yarrowia,* entre les séquences promotrices et germinatrices de GUT2, la séquence SEQ ID NO : 11.

Avantageusement, la délétion ADE2 correspond à la délétion Ade2-844 décrite dans les exemples. Ade2-844 correspond à une délétion complète de l'ORF du gène ADE2 (SEQ ID NO:3), laquelle délétion est présente dans les souches FF-Lua (CNCM-3912) et FF-luga (CNCM-3913). Après excision, il reste sur le génome de *Yarrowia,* entre les séquences promotrices et terminatrices de ADE2, la séquence SEQ 1D NO : 12.

Par milieu minimal, on entend ici un milieu ne comportant pas les éléments pour lesquels la souche de *Yarrowia* est auxotrophe, ou permettant, le cas échéant, la sélection de la souche.

Des souches de *Yarrowia* sont bien connues de l'homme du métier. A titre d'exemples préférés de telles souches, on peut citer les souches de *Yarrowia lipolytica.*

La technique de transformation d'une levure par intégration ciblée d'un gène est une technique de biologie moléculaire fréquemment utilisée. Dans cette technique, un fragment d'ADN est cloné dans un vecteur, introduit dans la cellule à transformer, lequel fragment d'ADN s'intègre alors par recombinaison homologue dans une région ciblée du génome receveur (ORR-WEAVER et al., Proc. Natl. Acad. Sci. USA, vol.78, p : 6354-6358, 1981). De tels procédés de transformation sont bien connus de l'homme du métier et sont décrits, notamment, dans ITO et al. (J. Bacteriol., viol. 153, p :163-168, 1983) dans KLEBE et al. (Gene, vol.25, p :333-341, 1983) et dans GYSLER et al. (Biotechn. Techn., vol.4, p : 285-290, 1990). Dans la mesure où cet événement de recombinaison est rare, des marqueurs de sélection sont insérés entre les séquences assurant la recombinaison afin de permettre, après transformation, d'isoler les cellules où l'intégration du fragment s'est produite par mise en évidence des marqueurs correspondant.

Les marqueurs de sélection permettant la complémentation d'une auxotrophie, également communément appelés marqueurs d'auxotrophie, sont bien connus de l'homme du métier.

Le marqueur de sélection MURA3 est bien connu de l'homme du métier. Plus spécifiquement, une souche de *Yarrowia* dont le gène URA3 (SEQ ID N°1 pour *Yarrowia lipolytica,* ladite séquence est aussi accessible par le numéro d'accession Yali0E26719g à l'adresse « http://cbi.labri.fr/Genolevures/index.php# »), codant pour l'orotidine-5'-phosphate décarboxylase, est inactivé (par exemple par délétion), ne sera pas capable de pousser sur un milieu non supplémenté en uracile. L'intégration du marqueur de sélection URA3 dans cette souche de *Yarrowia* permettra alors de restaurer la croissance de cette souche sur un milieu dépourvu d'uracile.

Le marqueur de sélection LEU2 décrit notamment dans le brevet US 4,937,189 est également bien connu de l'homme du métier. Plus spécifiquement, une souche de *Yarrowia* dont le gène LEU2 (Yali0E26719g ; SEQ ID N°2 pour *Yarrowia lipolytica*), codant pour la β-isopropylmalate déhydrogenase, est inactivé (par exemple par délétion), ne sera pas capable de pousser sur un milieu non supplémenté en leucine. Comme précédemment. L'intégration du marqueur de sélection LEU2 dans cette souche de *Yarrowia* permettra alors de restaurer la croissance de cette souche sur un milieu non supplémenté en leucine.

Le marqueur de sélection ADE2 est également bien connu de l'homme du métier dans le domaine de la transformation de la levure. Une souche de *Yarrowia* dont le gène ADE2 (SEQ ID N°3 pour *Yarrowia lipolytica,* YALI0B23188g), codant pour la phosphoribosylaminoimidazole carboxylase, est inactivé (par exemple par délétion), ne sera pas capable de pousser sur un milieu non supplémenté en adénine. Là encore, l'intégration du marqueur de sélection ADE2 dans cette souche de *Yarrowia* permettra alors de restaurer la croissance de cette souche sur un milieu non supplémenté en adénine.

Le gène GUT2 code pour la glycerol-3-phosphate déhydrogenase (SEQ ID N°4, pour *Yarrowia lipolytica,* YALI0B13970g) et son inactivation (par exemple par délétion) entraîne l'incapacité pour le mutant résultant à pousser sur un milieu non fermentable dont la source de carbone correspond à du glycérol.

Parmi les gènes de résistance qui pourront être utilisés comme marqueur de sélection pour conférer à la souche de *Yarrowia* un phénotype présentant un caractère dominant, on peut citer :
- Le gène MDR3 originaire de la souris code pour la P-glycoprotéine et, lorsqu'il est exprimé dans une souche de Yarrowia, confère une résistance au FK520 (agent antifongique et immunosuppresseur; RAYMOND et al., Mol. Cell. Biol., viol.14, p :277-286, 1994).
- Le modulé KanMX, qui contient la séquence kan^{r} issue du transposon Tn903 d'*Escherichia coli* fusionnée aux séquences transcriptionnelles du gène TEF du champignon filamenteux *Ashbya gossypii,* ou à tout promoteur et terminateur de transcription fonctionnels, confère à une souche de *Yarrowia* la résistance à la généticine (G418 ; WACH et al., Yeast, vol.10, p :1793-1808, 1994).
- Le gène HPH (HYG), originaire d'un plasmide d'*Escherichia coli,* permet la résistance à l'hygromycineB (GRITZ & DAVIES, Yeast, vol.8, p : 667-668, 1992 ; CORDERO OTERO & GAILLAIRDIN, Applied Microbiol and Biotechnologie, vol. 46, p : 143-148, 2004).
- Le gène Tn5ble, issu de *Escherichia coli,* permet à la levure transformée d'acquérir une résistance à la phléomycine (WENZEL et al., Yeast, vol.8, p :667-668, 1992).

D'autres marqueurs de sélection utilisables dans le procédé selon l'invention sont également décrits par BARTH & GAILLARDIN (The dimoorphic fungus Yarrowia lipolytica. In : Non conventional yeasts in biotechnology (Wolf K. Ed.). Springer-Verlag, Bef-lin, p : 313-388, 1996)*.*

Dans un mode de réalisation également préféré, le procédé d'intégration ciblée selon l'invention est caractérisé en ce qu'à l'étape b), le site d'intégration ciblé dans le génome de ladite souche de *Yarrowia* est choisi parmi les gènes URA3, LEU2, ADE2, LIP2, LIP7, LIP8, AXP, GUT2 et XPR2.

De préférence, le procédé d'intégration ciblée est caractérisé en ce qu'à l'étape b), le site d'intégration dans le génome de ladite souche de Yarrowia est choisi parmi les gènes donc la disruption confère un phénotype facilement détectable (visualisable, mesurable), notamment parmi les gènes URA3, ADE2, LIP2, LIP8, AXP.

Ainsi, selon un mode de réalisation préféré, l'étape b), le site d'intégration ciblé dans le génome de ladite souche de *Yarrowia* est choisi parmi les gènes URA3, LEU2, ADE2, LIP2, LIP8 et AXP.

Le gène LIP2 (SEQ ID N°5 pour *Yarrowia lipolytica)* peut être utilisé comme locus d'insertion. Le gène LIP2 code pour une lipase extracellulaire (PIGNEDE et al., J. Bacteriol., vol. 182(10), p:2802-10, 2000) qui hydrolyse préférentiellement les triglycérides à longue chaîne des résidus oléiques. Une souche de *Yarrowia* dont le gène LIP2 est inactivé (par exemple par délétion), présentera une activité lipase reduite (faible activité lipase extracellulaire, faible halo d'hydrolyse r boite te tributyrine).

Le gène LIP8 (SEQ ID N°6 pour *Yarrowia lipolytica)* peut être utilisé comme locus d'insertion. Le gène LIP8 code pour une triacylglycérol hydrolase. Une souche de *Yarrowia* dont le gène LIP8 est inactivé (après avoir inactivé LIP2) ne présentera pas de halo d'hydrolyse de la tributyrine sur boite YNBT et pas activité lipase (FICKERS et al., Fungal Genetics and Biology, vol. 42, p : 264-274, 2005).

Le gène AXP (SEQ ID N°7 pour *Yarrowia lipolytica*) peut être utilisé comme locus d'insertion. Le gène AXP code pour une protéase acide extracellulaire normalement exprimée lorsque le pH du milieu est inférieur à 4. Une souche de *Yarrowia* dont le gène AXP est inactivé (par exemple par délétion), présentera sur boite YNBcaseine à pH acide un halo d'hydrolyse de la caséine diminué et une activité protéase diminué. (GLOVER et al., Microbiology, vol. 143, p.: 3045-54, 1997).

Le gène XPR2 code pour une protéase alcaline extracellulaire exprimée lorsque le pH du milieu est supérieur à 6. Une souche de *Yarrowia* dont le gène XPR2 (SEQ ID N°8 pour *Yarrowia lipolytica)* est inactivé (par exemple par délétion), présentera sur boite YNBcaseine à pH6 un halo d'hydrolyse de la caséine diminué et une activité protéase diminué (DAVIDOW et al., J. Bacteriol., vol. 169, p. 4621-9, 1987).

A titre d'exemple, on peut citer la souche de *Yarrowia lipolytica* déposée le 4 février 2008 conformément au traité de Budapest auprès de la Collection nationale de Culture de Microorganismes (CNCM), INSTITUT PASTEUR, 25 rue du Docteur Roux, 75724 PARIS CEDEX 15, France sous le numéro CNCM-3912.

A titre d'exemple, on peut citer la souche de *Yarrowia lipolytica* déposée le 4 février 2008 conformément au traité de Budapest auprès de la Collection nationale de Culture de Microorganismes (CNCM), INSTITUT PASTEUR, 25 rue du Docteur Roux, 75724 PARIS CEDEX 15, France sous le numéro CNCM-3911.

Avantageusement, ladite souche de *Yarrowia* comprend quatre gènes présentant indépendamment l'un de l'autre une délétion associée à un phénotype d'auxotrophie ou de caractère dominant, de préférence choisis parmi les gènes URA3, LEU2, GUT2 et ADE2.

La présente demande se rapporte également à la souche de *Yarrowia lipolytica* déposée le 4 février 2008 conformément au traité de Budapest auprès de la Collection nationale de Culture de Microorganismes (CNCM), INSTITUT PASTEUR, 25 rue du Docteur Roux, 75724 PARIS CEDEX 15, France sous le numéro CNCM-3913.

Selon un mode de réalisation préféré du procédé selon l'invention, celui-ci permet l'intégration ciblée de 3 à 10 copies du gène d'intérêt, de préférence de 4 à 8 copies et, de manière particulièrement préférée, de 5 à 7 copies dudit gène d'intérêt.

Avantageusement, le procédé selon l'invention comprend la transformation de ladite souche de *Yarrowia* auxotrophe avec trois à dix vecteurs recombinants, de préférence de 4 à 8 vecteurs recombinants et, de manière particulièrement préférée, de 5 à 7 vecteurs recombinants.

Les vecteurs recombinants utilisés à l'étape b) pourront comprendre en outre un ou plusieurs marqueurs de sélection distincts de ceux permettant, pour ladite souche de *Yarrowia,* la complémentation de l'auxotrophie et, le cas échéant du caractère dominant résultant des délétions parmi les au moins trois gènes.

Les séquences codant pour le marqueur de sélection et celles du gène d'intérêt comprennent en outre les éléments nécessaires à leur expression dans une souche de *Yarrowia.*

De tels éléments correspondent notamment à des séquences promotrices et terminatrices actives chez une souche de *Yarrowia.* De préférence, les séquences promotrices et terminatrices utilisées appartiennent à des gènes différents de manière à minimiser les risques de recombinaison non désirée dans le génome de la souche de *Yarrowia.*

De telles séquences promotrices sont bien connues de l'homme du métier et peuvent correspondre notamment à des promoteurs inductibles ou constitutifs. A titre d'exemple de promoteurs utilisables dans le procédé selon l'invention, on peut citer notamment le promoteur d'un gène de *Yarrowia lipolytica* qui est fortement réprimé par le glucose et qui est inductible par les acides gras ou les triglycerides tel que le promoteur POX2 du gène de l'acyl CoA oxydase 2 de *Yarrowia lipolytica* et le promoteur du gène LIP2 décrit dans la demande PCT WO 01/83773. On peut aussi utiliser le promoteur du gène FBA1 du gène de la fructose-bisphosphate aldolase (US 2005/0130280), le promoteur du gène YAT1 du gène du transporteur de l'ammonium (US 2006/0094102 A1), le promoteur du gène GPAT du gène de la glycérol-3-phosphate O-acyltransferase (US 2006/0057690 A1), le promoteur du gene TEF (US 2001/6265185) et le promoteur hybride hp4d (WO9641889).

De telles séquences terminatrices sont également bien connues de l'homme du métier et on peut citer, à titre d'exemple de séquences terminatrices utilisables dans le procédé selon l'invention, la séquence terminatrice du gène PGK1, la séquence terminatrice du gène LIP2 décrit dans la demande PCT WO 01/83773.

Lorsque le produit du gène d'intérêt est destiné à être sécrété par la cellule hôte, ledit insert comprend en outre des signaux de contrôle de la sécrétion dudit produit. On peut utiliser dans ce but des séquences signal fonctionnelles chez les souches de *Yarrowia,* par exemple tout ou partie de la séquence prépro du gène LIP2 décrit dans la demande PCT WO 01/83773.

Le gène d'intérêt code pour un polypeptide que l'on souhaite produire en grande quantité dans la souche de *Yarrowia* transformée. Ledit polypeptide correspond de préférence à un polypeptide hétérologue, comme par exemple la L-asparaginase de *Erwinia chrysanthemi* (SEQ ID N° 9) ou de *Escherichia coli* (SEQ ID N°10), mais il peut également correspondre à un polypeptide autologue comme la lipase LIP2 de *Yarrowia lipolytica* décrite précédemment.

Selon un mode de réalisation particulier du procédé selon l'invention, les étapes b) de transformation de la souche de *Yarrowia* et c) de sélection sur milieu minimal sont effectuées séparément pour chacun desdits au moins trois vecteurs recombinants.

Selon un mode de réalisation plus préféré, le procédé d'intégration ciblée selon l'invention est caractérisé en ce qu'à l'étape b), les transformations avec au moins les trois vecteurs recombinants sont réalisées de manière indépendante et successivement avec chacun des vecteurs recombinants, ou simultanément avec l'ensembles desdits vecteurs recombinants, et en ce que l'ensemble de ces transformations est suivie d'une étape c) de sélection sur un milieu minimal des levures ayant intégré l'ensemble desdits vecteurs recombinants.

Selon un mode de réalisation également préféré, le procédé selon l'invention est caractérisé en ce que le marqueur de sélection est encadré par des séquences permettant son excision après l'intégration ciblée dudit vecteur recombinant.

Il est aussi décrit un procédé selon l'invention, dans lequel le marqueur de sélection est encadré par des séquences permettant son excision après l'intégration ciblée dudit vecteur recombinant.

Le procédé selon l'invention permet alors de réutiliser un même marqueur de sélection, notamment un marqueur d'auxotrophie, pour une autre intégration ciblée lors d'une nouvelle étape de transformation.

De telles séquences permettant l'excision et l'élimination, chez une souche de *Yarrowia,* d'une séquence encadrée sont bien connues de l'homme du métier.

A titre d'exemple, on peut citer l'utilisation de deux séquences identiques ou similaires, dites séquences répétées directes (SRD), en vue d'obtenir un événement de recombinaison comme décrit dans le brevet EP 0994192 B1 ou dans le brevet EP 0635574 B1.

A titre d'exemple, on peut également citer le système « Cre-lox » tel que décrit dans le brevet EP 0220009 B1, dans lequel les séquences équivalentes aux séquences répétées directes sont dans ce cas des séquences spécifiques dites « lox » et l'excision de la séquence d'ADN comprise entre les deux séquences « lox » s'effectue en présence d'une recombinase « Cre » exprimée par un vecteur d'expression. D'autres systèmes équivalent et utilisant d'autres recombinases spécifiques sont également connus de l'homme du métier notamment le procédé décrit dans le brevet EP 0814165 B1.

Sous un autre aspect, la présente invention a pour objet un procédé de production d'un polypeptide codé par un gène d'intérêt, caractérisé en ce qu'il comprend :
A) un procédé d'intégration ciblée selon l'invention;
B) une étape d) de culture de la souche de *Yarrowia* modifiée obtenue par le procédé selon l'une quelconque des revendications 1 à 10 dans un milieu de culture liquide comprenant des sources assimilables de carbone, d'azote et de sels inorganiques de sorte de permettre la croissance de ladite souche transformée de *Yarrowia* et
C) la récupération dudit polypeptide d'intérêt exprimé à partir des cellules *Yarrowia* ou du milieu de culture obtenus à l'étape B).

Avantageusement, le procédé selon l'invention comprend une étape e), après l'étape C), de purification dudit polypeptide codé par le gène d'intérêt.

Cette étape e) de purification peut être effectuée soit après lyse cellulaire, soit, avantageusement, à partir du milieu de culture dans lequel il est sécrété par les cellules, par des techniques classiques, connues en elle mêmes, par exemple par précipitation fractionnée suivie d'une ou plusieurs étapes de chromatographie.

Les cellules de *Yarrowia* transformées susceptibles d'être obtenues par le procédé conforme à l'invention font également partie de la présente invention.

Ainsi, la présente invention se rapporte également à une souche de *Yarrowia* auxotrophe susceptible d'être obtenue par le procédé selon l'inventionlaquelle souche comprend au moins trois délétions choisies parmi Ura3-302, Leu2-270, Gut2-744 et Ade2-844 , le phénotype associé à chacune de ces délétions correspondant à une auxotrophie dominant pour ladite souche, indépendamment l'un de l'autre de ces au moins trois gènes.

Selon un autre aspect, la présente invention est relative à un procédé d'obtention d'une souche modifiée de *Yarrowia,* caractérisée en ce qu'il comprend une étape dans laquelle on transforme ladite souche de *Yarrowia* avec au moins trois vecteurs permettant d'aboutir à une souche de *Yarrowia* comprenant au moins trois délétions choisies parmi Ura 3-302, Leu 2-270. Gut 2-744 et Ade 2-844 et caractérisée en ce qu'indépendamment l'un de l'autre de ces au moins trois gènes, le phénotype associé à chacune de ces délétions correspond à une auxotrophie ou à ûn caractère dominant pour ladite souche.

De préférence ici, ladite souche comprend en outre au moins un gène associé à un phénotype de caractère dominant choisis parmi : le gène de résistance à l'hygromycine HPH (HYG), et les gènes MDR3, KanMX et Tn5ble, le gène HYG étant aussi le plus préféré des gènes de résistance.

Il est également décrit une souche de *Yarrowia* auxotrophe et, le cas échéant, présentant un caractère dominant acquis, susceptible d'être obtenue par le procédé d'obtention d'une souche modifiée de *Yarrowia* selon l'invention, laquelle souche comprend une délétion dans au moins trois gènes de son génome et caractérisée en ce qu'indépendamment l'un de l'autre de ces au moins trois gènes, le phénotype associé à chacune de ces délétions correspond à une auxotrophie ou à un caractère dominant pour ladite souche. Chacune des préférences définies pour le nombre de gènes délétés, les gènes choisis pour la délétion telles que définies pour l'étape a) du procédé d'intégration selon la présente invention est également préférée pour ce procédé.

Avantageusement, ladite souche de *Yarrowia* obtenue par ce procédé est une souche de *Yarrowia lipolytica.*

De préférence, ladite souche de *Yarrowia* auxotrophe comprendra au moins une mutation parmi les gènes URA3, LEU2 et ADE2 ou URA3, LEU2 et GUT2.

A titre d'exemple, on peut citer les souches de *Yarrowia lipolytica* déposées le 4 février 2008 conformément au traité de Budapest auprès de la Collection nationale de Culture de Microorganismes (CNCM), INSTITUT PASTEUR, 25 rue du Docteur Roux, 75724 PARIS CEDEX 15, France sous les numéros CNCMI-3911 et CNCM 1-3912.

Selon un deuxième mode de réalisation préféré, la souche de *Yarrowia* auxotrophe utilisée dans le procédé selon l'invention comprend une mutation parmi quatre gènes nécessaires à la croissance de ladite souche, lesquels trois gènes sont choisis dans le groupe comprenant URA3, LEU2, ADE2, LIP2, LIP7, LIP8, AXP, GUT2 et XPR2.

De préférence, ladite souche de *Yarrowia* auxotrophe comprendra au moins une mutation parmi les gènes URA3, LEU2, ADE2 et GUT2.

A titre d'exemple on peut citer la souche de *Yarrowia lipolytica* déposée le 4 février 2008 conformément au traité de Budapest auprès de la Collection nationale de Culture de Microorganismes (CNCM), INSTITUT PASTEUR, 25 rue du Docteur Roux, 75724 PARIS CEDEX 15, France sous le numéro CNCMI-3913.

La présente invention sera mieux comprise à l'aide des figures et de leur légéende et du complément de description qui va suivre, qui se réfère à des exemples non-limitatifs d'obtention de souches transformées de *Yarrowia lipolytica* conforme à l'invention.

### Légendes des figures

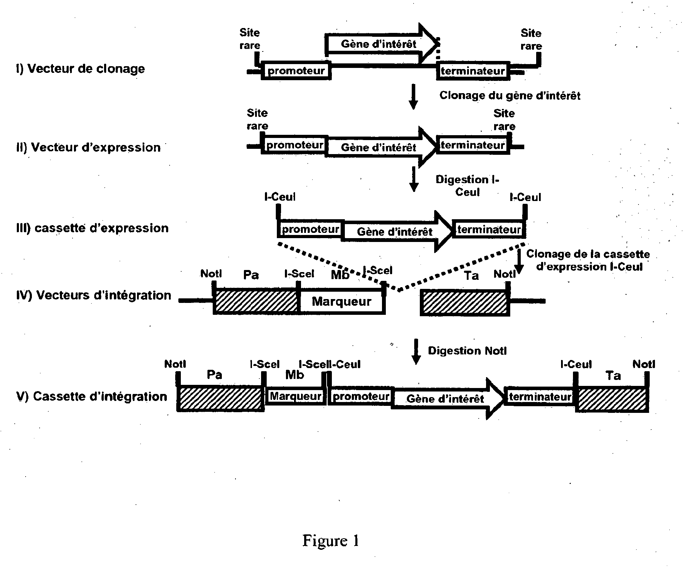

**Figure 1** **:** Représentation schématique de la construction de « cassette d'intégration » ciblée de « cassette d'expression ». 1) Le gène d'intérêt est cloné dans un vecteur d'expression, 2) la cassette d'expression est libérée par une digestion I-Scel, 3) puis clonée dans différents vecteurs d'intégration, 4) les cassettes d'intégration sont libérées par une digestion NotI, purifiées et utilisées pour transformer une souche d'intégration.

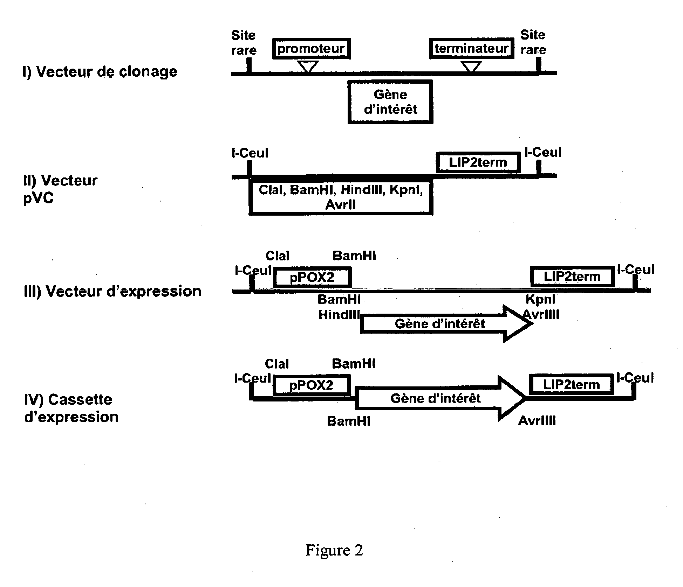

**Figure 2** **:** Représentation schématique du vecteur de clonage. 1) Le vecteur de clonage comprend un promoteur fort suivi de sites de restriction unique pour le clonage du gène d'intérêt, d'un terminateur de transcription, et de part et d'autre un site rare pour permettre l'excision de la cassette d'expression. 2) le vecteur pVC de base contient un multi sites composé des sites ClaI; BamHI, KpnI et AvrII, , la séquence termiriatrice du gène LIP2 (LIP2term) avec de part et d'autre le site rare I-CeuI, 3) le vecteur d'expression contient le promoteur pPOX2 cloné aux sites ClaI et BamHI, 4) La cassette d'expression est obtenue par clonage au miltisites du gène d'intérêt, par exemple BamHI-AvrII, puis une digestion du vecteur d'expression par I-CeuI.

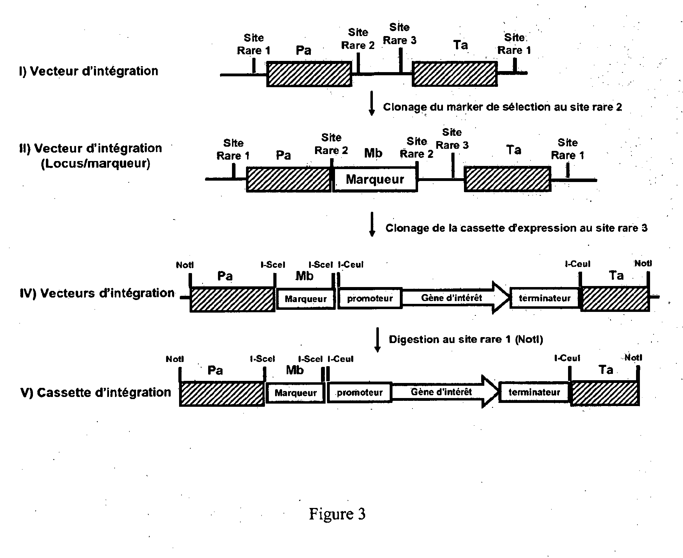

**Figure 3** **:** Représentation schématique du vecteur d'intégration et obtention de la cassette d'intégration ciblée. 1) Le vecteur d'intégration contient une région amont du locus d'insertion (région P, pour promotrice) et une région aval du locus d'insertions (région T, pour terminatrice) avec de part et d'autre un site rare 1 pour l'excision de la cassette d'intégration. Entre les régions P et T sont présent deux sites rares, le site rare 2 pour l'insertion du marqueur de sélection, et le site rare 3 pour l'insertion de la cassette d'expression.

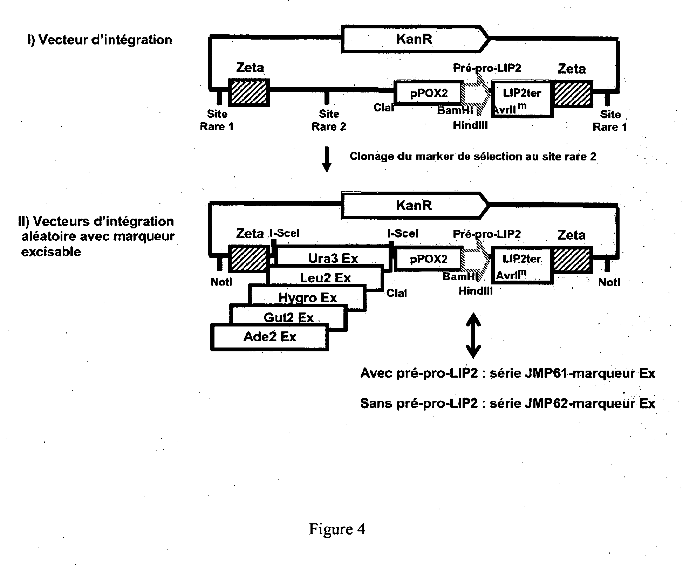

**Figure 4** **:** Représentation schématique des vecteurs d'intégration pour une intégration aléatoire. I) représentation schématique du vecteur qui comprend un site rare 1 utilisable pour libérer la cassette d'expression de la partie plasmide, un site rare 2 pour le clonage du marqueur de sélection pour *Y. lipolytica.* Il contient un promoteur entre les sites ClaI et BamHI (ici le promoteur pPOX2), le terminateur du gène LIP2 et les régions Zeta pour une intégration aléatoire (voir brevet amplification). La série JMP61 présente la séquence d'adressage de la lipase Lip2. II) Représentation schématique des vecteurs d'intégration pour une intégration aléatoire construit avec le site NotI comme site rare 1 et le site I-SceI comme site rare 2 utilisé pour l'insertion des marqueurs URA3. LEU2. Hyg, GUT2 et ADE2.

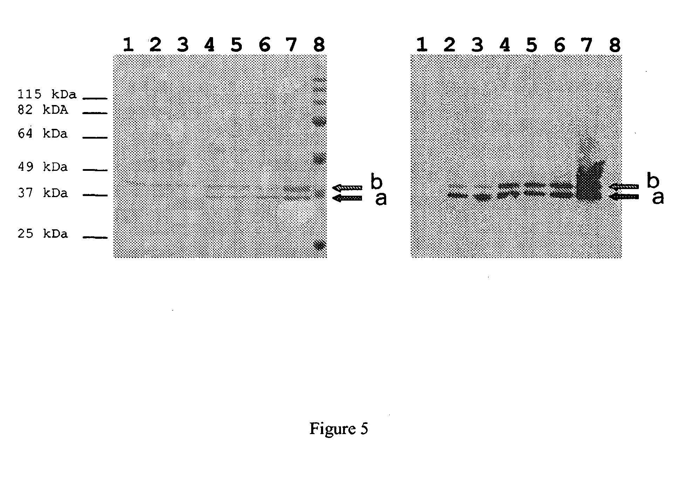

**Figure 5** **:** Expression de l'asparaginase dans les souches contenant différent nombre de copies du gène. A) Analyse sur gel d'électrophorèse NuPAGE 10% et coloration au bleu colloïdal (équivalent à 30 µl de surnageant), B) Analyse sur gel d'électrophorèse NuPAGE 10%, transfert et détection par Western blot avec un anticorps anti Asparag.inase (équivalent à 10 µl de surnageant). La bande a correspond à la protéine non glycosylée et la bande b correspond à la protéine mono glycosylée. L1, Souche réceptrice FF-luga ; L2, souche 5LAsp1 : ade2 :: GUT2-Asp, 1 copie; L3, souche 6LAsp1 :ade2 :: GUT2-Asp, 1 copie; L4, souche 15LAsp2 : ade2 :: GUT2-Asp lip2 ::URA3-Asp, 2 copies ; L5, souche 21LAsp2 : ade2 :: GUT2-Asp lip2 ::URA3-Asp, : 2 copies ; L6, souche ILAsp3 : ade2 :: GUT2-Asp lip2 ::URA3-Asp leu2 ::ADE2-Asp : 3 copies ; L7, souche 2LAsp4 : ade2 :: GUT2-Asp lip2 ::URA3-Asp leu2 ::ADE2-Asp ura3 ::LEU2-Asp : 4 copies.

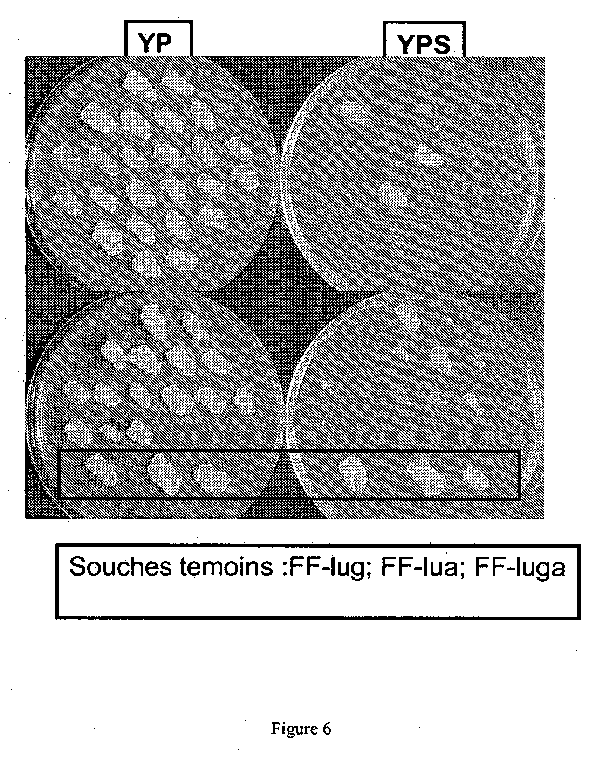

**Figure 6** **:** Analyse phénotypique Suc+/ Suc- pour vérifier l'insertion au locus URA3. Les transformants obtenus avec la cassette d'intégration ciblée au locus URA3 sont testés par strie sur boite de milieu riche glucose (YPD) et saccharose (YPS). Les souches réceptrices Suc+ FF-lug, FF-lua et FF-luga sont utilisées comme contrôle.

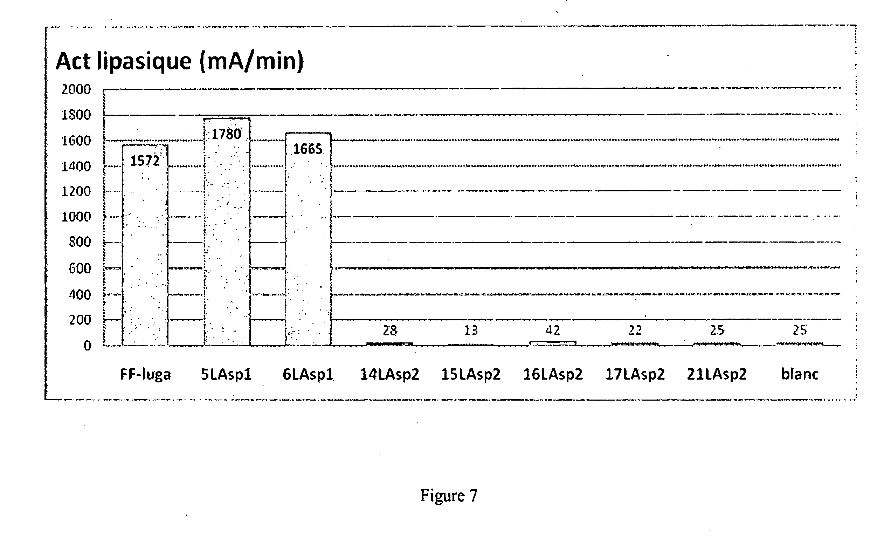

**Figure 7** : Analyse phénotypique Lip+/ Lip- pour vérifier l'insertion au locus LIP2. Les transformants obtenus avec la cassette d'intégration ciblée au locus LIP2 sont cultivés en milieu riche acide oléique (YPD2O2). L'activité lipase dans le surnageant des souches contenant différentes copies de l'asparaginase. La souche réceptrice Lip+ FF-luga est utilisée comme contrôle. Souches avec 1 copie au locus ADE2 (ade2 :: GUT2-Asp) : 5LAsp1 et 6LAsp1 ; souches avec 1 copie au locus ADE2 et 1 copie au locus LIP2 (ade2 :: GUT2-Asp, lip2 ::URA3-Asp) : 14LAsp2, 15LAsp2, 16LAsp2, 17LAsp2 et 21 LAsp2.

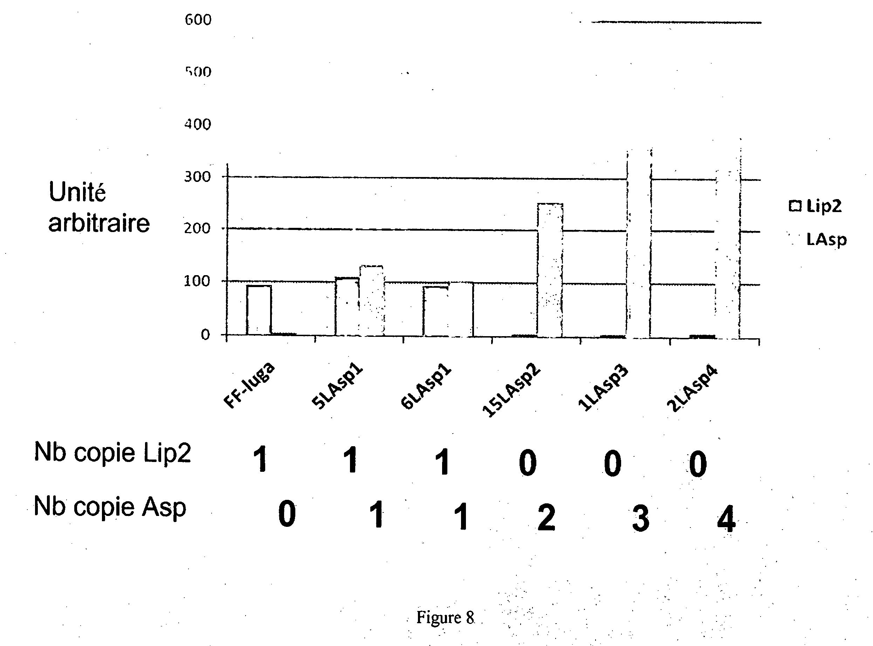

**Figure 8** : Détermination du nombre de copie des gène LIP2 et Asp par RT-qPCR dans les transformants.

### EXEMPLES

### Exemple 1 : Souches utilisées dans les exemples

Les souches utilisées dans le procédé de l'invention dérivent d'une souche sauvage de *Yarrowia lipolytica,* en particulier de la souche sauvage *Yarrowia lipolytica* W29 (ATCC 20460, répertoriée sous CLIB89 dans la Collection du CIRM (Centre International de Ressources Microbiennes).

Ainsi, on peut utiliser de nouvelles souches mutantes dérivant de la souche *Yarrowia lipolytica* ATCC 20460 par l'intermédiaire de la souche Po1d [souche auxotrophe pour la leucine (Leu-) et l'uracile (Ura-)], décrite dans la revue de G. Barth et al. : Yarrowia lipolytica. in: Nonconventional Yeasts in Biotechnology A Handbook (Wolf, K., Ed.), Vol. 1, 1996, pp. 313-388. Springer-Verlag. Elle est répertoriée sous CLIB139 dans la CIRM.

L'obtention de ces nouvelles souches receptrices (FF-lu, FF-lug (CNCM I-3911), FF-lua (CNCM I-3912), FF-luga (CNCM I-3913)) sera décrite plus loin.

### Obtention des souches

L'obtention des souches mutantes réceptrices utilisables dans le procédé de l'invention peut se faire à partir de la souche Po1d, qui dérive de la souche sauvage Yarrowia lipolytica W29. La souche Po1d est une souche auxotrophe pour la leucine (leu-) et l'uracile (ura-). Elle est décrite dans la revue de G. Barth et al. : Yarrowia lipolytica in: Nonconventional Yeasts in Biotechnology A Handbook (Wolf, K., Ed.), Vol. 1, 1996, pp. 313-388. Springer-Verlag, Berlin, Heidelberg, New York. Elle est répertoriée sous CLIB139 dans la collection du CIRM. L'obtention des souches mutantes utilisables dans le procédé de l'invention peut se faire par l'insertion de délétion de gène conférant une auxotrophie ou conférant un défaut de croissance sur certain milieux, dont le gène présente une délétion, si possible une délétion qui ne puisse par reverser, avantageusement correspond a une délétion complète du gène. Dont le gène correspondant puisse être utilisé comme marqueurs de sélection pour la sélection des transformants ayant intégrée les cassettes d'intégration. Les délétions présenteront une délétion importante par rapport à la cassette correspondante utilisée pour la construction des markers de sélection, afin de diminuer le taux de conversion.

### 1 . Délétion du gène GUT2 (Δgut2-744), construction du marqueur GUT2 et obtention de la souche mutante FF-lug (CNCM I-3911).

### 1.1 Délétion du gène GUT2 (Δgut2-744)

Pour obtenir cette souche, on peut procéder comme suit :
1) on sélectionne le gène d'intérêt que l'on veut déléter ;
2) on construire une cassette de disruption par clonage ou par PCR (« Polymerase Chain Reaction) en amplifiant la région amont du gène (région « promotrice », symbolisé par P) et la région aval du gène (région « terminatrice », symbolisé par T) ;
3) on introduit un marqueur de sélection contenant de part et d'autre une séquence de recombinaison (avantageusement une séquence loxP ou loxR ou dérivée) permettant une recombinaison entre elle pour l'élimination du marqueur (avantageusement une séquence de type loxP qui permet la recombinaison sous l'action de la recombinase cre);
4) on transforme avec la cassette de disruption et on sélectionne les souches avec le gène d'intérêt délété (transformation et sélection des transformants) et on vérifie la disruption du gène ;
5) on transforme avec un vecteur permettant l'expression de la recombinase (avantageusement la recombinase cre qui permet la recombinaison des séquences loxP/loxR et l'élimination du marqueur) ;
6) on isole un clone présentant la délétion du gène d'intérêt et ayant perdu le plasmide d'expression de la recombinase.

À partir du mutant FF-lu auxotrophe (Leu-, Ura-), on a construit une souche Y. lipolytica mutante FF-lug auxotrophe pour la leucine et l'uracile (Leu-, Ura- et incapable d'utiliser le glycérol comme source de carbone (gly-). La première étape est la construction de la souche FF-lug ::URA3 prototrophe pour l'uracile (Leu-, Ura+) par délétion du gène GUT2 par la cassette de disruption gut2 ::URA3ex en transformant le fragment de PCR GUT2-PUexT (JME744) et en sélectionnant les Ura+ sur YNBcasa. On sélectionne les auxotrophes pour l'uracile (Leu+, Ura-) par excision du marqueur URA3ex en transformant avec le vecteur réplicatif pRRQ2 contenant la recombinase Cre et le marqueur LEU2 (Cre-LEU2) et en sélectionnant les transformants auxotrophes pour l'uracile, Leu+. La perte du plasmide pRRQ2 est réalisée par une culture sur milieu riche YPD et par l'isolement d'un clone (Leu-, Ura-, Gly-).
La représentation schématique de la construction de ces mutants est résumée dans le Tableau 1 ci-dessous.

**Tableau 1 : Étapes de transformation requises pour la production du mutant FF-lug**

| Étape | Opérations de transformation | | |
|---|---|---|---|
| | Mutant à transformer | Cassette de transformation | Mutant transformé |
| 1 | FF-lu, PO1d, Leu-, Ura- | Gut2-PUexT | JMY1202, Leu-, Ura+, Glygut2=PUexT |
| 2 | JMY1202, Leu-, Ura+, Gly-, gut2-PUexT | Vecteur pRRQ2 (pKS Cre ARS68 LEU2) | FF-lug JMY1346, Leu-, Ura-, Glygut2-744 |

La cassette de disruption pour le gène GUT2 a été construite par amplification de la région amont (région P ; YaliOB:1873518..1872400 complémentaire, 1119bp) et de la région aval (région T ; Yali0B:1870468.. 1869652 complémentaire, 817bp) ce qui permet la délétion de l'ORF du gène GUT2 (YaliOB1870469..1872401) et laisse une homologie avec le marqueur de sélection GUT2ex-0.5 (485 bp avec la région P et 56 bp dans la région T) afin de minimiser les conversions géniques. Les marqueurs de sélection ex sont présentés dans le Tableau 11.

Les régions P et T ont été amplifiées avec les primers G3PD-P1, G3PD-P2, G3PD-T1 et G3PD-T2 (Tableau 9).

Les primers G3PD-P2 et G3PD-T1 contiennent le site rare I-SceI pour l'insertion du marqueur de sélection excisable URA3ex. Le fragment PT a été cloné dans pGEM-T (Invitrogen) pour donner le plasmide pGEM-GUT2-PT (JME743). Le plasmide de disruption pGEM-GUT2-PUexT (JME744) a été obtenu par insertion du marqueur URA3ex au site I-SceI provenant du plasmide JME507.

La cassette de disruption (3245 pb) a été obtenue par PCR avec les primers G3PD-P1 et G3PD-T2. Le produit de PCR a été purifié sur gel d'agarose à l'aide du kit gel extraction Qiagen. Les souches ont été transformée par la méthode à l'acétate de lithium Les transformants Ura+ ont étés sélectionnés sur milieu YNBcasa.

Les transformants Ura+ sont obtenus avec un taux de transformation typique (Fickers, 2003). La vérification de la disruption du gène GUT2 a été vérifiée par PCR avec les primers en 5' de la région P (G3PDver1) et en 3' de la région T (G3PDver2). Le mutant JMY1202 gut2::URA3ex a été ensuite transformé par le plasmide replicatif pRRQ2 (pKS Cre ARS68 LEU2 ; JME461). Les transformants obtenus sont criblés pour l'excission du marqueur URA3 et la perte du plasmide réplicatif d'expression de la recombinase cre est réalisée par une culture sur milieu riche YPD et par l'isolement d'un clone (Leu-, Ura-, Gly-).. Le mutant JMY1346 (FF-lug) a été sélectionné.

La séquence du locus gut2-844 du mutant JMY1346 a été amplifiée avec les primers G3PDver1 et G3PDver2. Le produit de PCR de taille de 2415 pb a été séquencé. La séquence obtenue montre l'absence de la séquence de marqueur Ura3. Après excision, il reste sur le génome les séquences suivantes entre P et T du locus GUT2: TGCAGCTTTCGAGAACCGACGCCTGGGACCTGTGTCTGTAGGGATAACAGG GTAATTCGCTTCGGATAACTCCTGCTATAACGAAGTTATGTAGGGATAACAG GGTAAT (SEQ ID NO : 11 ; soit site I-SceI - séquence LoxR - site I-SceI).

### 1.2 Construction du marqueur GUT2ex

Pour la construction du marqueur GUT2ex, la séquence génomique du locus GUT2 a été amplifié avec les primers G3PTS et G3PTR (Tableau 8) et cloné dans le vecteur pKS-LPR (Fickers et al., 2003, Journal of Microbiological Methods, vol. 55, p : 727-737) au site EcoRI. Il contient 492 bp de promoteur, l'ORF du gène GUT2, (ORF Yali0B:1870546..1872384) et 132 bp du terminateur et qui correspond à la séquence Yali0B:1872876..1870417. Cette ORF contient 2 sites BamHI et 1 site EcoRI qui ont été éliminés par mutagénèse dirigée en introduisant des mutations silencieuses. Nous avons utilisé les primers suivants : G3PB1S et G3PB1R pour le premier site BamHI (ggAtcc en ggCtcc), G3PB2S et G3PB2R pour le deuxième site BamHI (ggatcC en ggatcT) et G3PE1S et G3PE1R pour le site EcoRI (gaAttc en gaGttc) (Tableau 9).

Le marqueur peut être inséré dans les deux sens. Pour ce marqueur GUT2ex0.5, seul le sens 1 a été obtenu dans le pKS-LPR. Après vérification par séquençage, le plasmide contenant le marqueur GUT2ex0.5 modifié est JME792.

Deux autres marqueurs GUT2ex1.0 et GUT2ex 1.5 contenant 1 kb et 1,5 kb de promoteur respectivement ont été construits suite à la difficulté d'obtenir des transformants à partir du marqueur GUT2ex0.5 (paragraphe 1.3). Ces promoteurs ont été amplifiés à partir de l'ADNg (souche E150 Yarrowia lipolytica) avec les primers P1KB et PNHE1 et P15KB et PNHE1, respectivement. Le clonage a été réalisé par ligature 3 voies entre le pKS-LPR (EcoRI déphosphorylé) le produit de PCR (EcoRI et NheI) et le reste du marqueur GUT2ex modifié issu de JME792 (NheI et EcoRI). Le marqueur GUT2ex1.0 dans le sens 1 correspond à JME919 et dans le sens 2 correspond à JME920. Le marqueur GUT2ex1.5 dans le sens 1 correspond à JME921 et dans le sens 2 correspond à JME922.

### 1.3 Transformation avec les marqueurs GUT2ex0.5, GUT2ex1.0 et GUT2ex1.5.

Pour valider l'utilisation du marqueur GUT2ex, le marqueur GUT2ex0.5 a été isolé du plasmide JME792 (pKS-LPR-GUT2ex0.5) par digestion I-SceI et inséré au site I-SceI du plasmide JMP62 I-SceI (JME801) pour donner le plasmide JMP62Gut2-ex0.5 (JME805).

Le plasmide JME805 a été digéré par NotI et utilisé pour transformer la souche FF-lug par la méthode au lithium acétate. Les transformants on été sélectionnés sur milieu YNBcasa Glycérol 1% supplémenté avec de l'uracile (100 mg/L). Après plusieurs tentatives, ce marqueur n'a pas permis d'obtenir des transformants.

Dans un premier temps, nous avons émis l'hypothèse d'une défectivité trop importante de la partie promotrice (492 pb). Nous avons donc construits 2 autres marqueurs contenant des parties promotrices plus longues (cf paragraphe 1.2) soit le marqueur GUT2ex1.0 et GUT2ex1.5. Les mêmes résultats ont été obtenus.

Il a été décrit chez la levure *Saccharomyces cerevisiae* une répression de l'utilisation du glucose en présence de glycérol pour des souches Δgut2. Effectivement, la souche FF-lug est incapable de pousser en présence des 2 sources de carbones pré-citées. En modifiant notre méthode de transformation, nous avons obtenu des transformants. Cette nouvelle méthode de transformation reste identique à la méthode à l'acétate de lithium et au polyéthylène glycol (Gaillardin et al. 1985) auquel on ajoute une étape de phase d'expression avant l'étalement de la transformation sur milieux sélectifs. Cette phase d'expression est réalisée ainsi : après le choc thermique a 42°C pendant 10 minutes. On centrifuge le produit de la transformation à 2000 rpm pendant 3 min et on lave les cellules avec 2 mL de tampon TE pH8. Ce lavage est répété 2 fois. Les cellules sont ensuite reprises avec le milieu nutritif Yeast Extract 1% Peptones 1% et dextrose 0,2%. On effectue une incubation de 16 heures à 28 degrés et sans agitation. De nouveau, on centrifuge à 2000 rpm pendant 3 min et on lave les cellules avec 2 mL de tampon TE pH8. Ce lavage est répété 2 fois. Les cellules sont ensuite reprises avec 2 ml de TE et sont étalées sur milieux sélectifs contenant du glycérol 1% comme seul source de carbone.

Les résultats de la transformation du marqueur GUT2ex1.0 dans la souche FF-lug sont présentés dans le tableau 2 suivant :

**Tableau 2 : Efficacité de transformation selon la méthode de transformation.**

| | Méthode classique sans phase d'expression | | Nouvelle méthode avec phase d'expression | |
|---|---|---|---|---|
| Type d'intégration | aléatoire | ciblée | aléatoire | ciblée |
| Nombre de transformantes | 0 | 14 | 133 | 615 |
| Taux de transformation par µg d'ADN | 0 | 187 | 266 | 8215 |
| % d'intégration | ND | 75% | ND | 100% |

### 2. Délétion du gène ADE2 (Δade2-844), construction du marqueur ADE2 et obtention des souches mutantes FF-lua (CNCM I-3912) et FF-luga (CNCM I-3913).

### 2.1 Délétion du gène ADE2 (Δade2-844)

Pour obtenir ces souches, on peut procéder comme suit : a partir du mutant FF-lu, on a construit la souche FF-lua Leu-, Ura-, Ade- et a partir du mutant FF-lug, on a construit la souche FF-luga Leu-, Ura-, Ade-, Gly-.
2.1a. À partir du mutant FF-lu auxotrophe (Leu-, Ura-), on a construit une souche Y. *lipolytica* mutante FF-lua auxotrophe pour la leucine, l'uracile et l'adénine (Leu-, Ura-, Ade-). La première étape est la construction de la souche FF-lua ::URA3 prototrophe pour l'uracile (Leu-, Ura+, Ade-) par deletion du gène ADE2 par la cassette de disruption Ade2 ::URA3ex en transformant la cassette NotI PTAde2-Ura3Ex (JME844) et en sélectionnant les Ura+ sur YNBcasa, supplémenté avec de l'adénine (800mg/L). On sélectionne les auxotrophes pour l'uracile (Leu+, Ura-, ade-) par excision du marqueur URA3ex en transformant avec le vecteur réplicatif pRRQ2 contenant la recombinase Cre et le marqueur LEU2 (Cre-LEU2) et en sélectionnant les transformants auxotrophes pour l'uracile, Leu+. La perte du plasmide replicatif pRRQ2 d'expression de la recombinase cre est réalisée par une culture sur milieu riche YPD et par l'isolement d'un clone (Leu-, Ura-, Ade-). Le mutant JMY1409 (FF-lua) a été sélectionné.
2.1.b. À partir du mutant FF-lug auxotrophe (Leu-, Ura-) et incapable d'utiliser le glycérol comme source de carbone (Gly-), on a construit une souche *Y. lipolytica* mutante FF-luga auxotrophe pour la leucine, l'uracile et l'adénine (Leu-, Ura-, Ade-) et incapable d'utiliser le glycérol comme source de carbone (Gly-). La première étape est la construction de la souche FF-luga ::URA3 auxotrophe pour la leucine et l'adénine (Leu-, Ade-, Ura+) par délétion du gène ADE2 par la cassette de disruption ade2 ::URA3ex en transformant la cassette NotI PTAde2-PUra3ExT (JME844) et en sélectionnant les Ura+ sur YNBcasa supplémenté avec de l'adénine (800 mg/L). On sélectionne les auxotrophes pour l'uracile (Leu+, Ura-) par excision du marqueur URA3ex en transformant avec le vecteur réplicatif pRRQ2 contenant la recombinase Cre et le marqueur LEU2 (Cre-LEU2) et en sélectionnant les transformants auxotrophe pour l'uracile, Leu+. La perte du plasmide pRRQ2 est réalisée par une culture sur milieu riche YPD et par l'isolement d'un clone (Leu-, Ura-, Ade-, Gly-). Le mutant JMY1404 (FF-luga) a été sélectionné.

La représentation schématique de la construction de ces mutants est résumée dans le Tableau 3 ci-dessous.

**Tableau 3 : Étapes de transformation requises pour la production des mutants FF-lua et FF-luga**

| **Etape** | **Opérations de transformation** | | |
|---|---|---|---|
| | **Mutant à transformer** | **Cassette de transformation** | **Mutant transformé** |
| 1 | PO1d, Leu-, Ura- | PTade2-Ura3Ex | JMY1407, Leu-, Ura+ ade2-PUexT |
| 2 | JMY1407, Leu-, Ura+ ade2-PUexT | Vecteur pRRQ2, sélection Leu+, vérification Gly-, perte du plasmide pRRQ2 sur YPD, isolement de Leu- | JMY1409, Leu-, Ura-, Aide-ade2-844 FF-lua |
| | | | |
| 1b | JMY1346, Leu-, Ura-, Gly-gut2-744 | PTade2-Ura3Ex | JMY1396, Leu-, Ura+ Gly- ade2-PUexT |
| 2b | JMY1396, Leu-, Ura+, Gly-ade2-844 | Vecteur pRRQ2, sélection Leu+, vérification Gly-, perte du plasmide pRRQ2 sur YPD, isolement de Leu- | JMY1404, Leu-, Ura-, Ade-, Gly-, ade2-844 FF-luga |

La cassette de disruption pour le gène ADE2 (Yali0B23188g ; highly similar to tr|Q9P4V1 *Candida boidinii),* code pour la phosphoribosyl-5-aminoimidazole carboxylase, protéine déduite de 565 aa (Yali0B:3030460..3032157, sens) a été construite par amplification de la région amont (région P ; Yali0B:3029011..3029931, 920 bp) et de la région aval (région T ; 792 pb, Yali0B:3031871..3032662) ce qui permet la délétion de l'ORF du gène ADE2 Yali0B:3030460..3032157). La région éliminée est de 1939 pb (Yali0B:3029932..3031870) et qui ne laisse qu'une très faible homologie avec le marqueur de sélection ADE2ex (O bp avec la région P et 415 bp dans la région T) afin de minimiser les conversions géniques. Le marqueur ADE2ex correspond à la région Yali0B:3029961..3032280 fragment de 2319 pb. Les marqueurs de sélection ex sont présentés dans le Tableau 11.

Les régions P et T ont été amplifiées avec les primers suivant P1ADE2, P2ADE2, T1ADE2, T2ADE2 (Tableau 9).

Les primers P2 et T1 contiennent le site rare I-SceI pour l'insertion du marqueur de sélection excisable URA3ex. Le primer T1 contient aussi le site rare I-CeuI pour l'insertion de la cassette d'expression. Les primers P1 et T2 contiennent le site rare NotI pour le clonage dans pHSS6-NotI (JME800). Le fragment PT a été cloné dans le JME800 digéré par NotI et déphosphorylé pour donner le plasmide pADE2-PT dans l'orientation 1 (sens 1, JME813) ou dans l'orientation 2 (sens 2, JME814). Le Sens est défini suivant l'orientation du site I-SceI : soit le sens 1 avec le site I-SceI en sens, soit le sens 2 avec le site I-SceI en complémentaire. Le plasmide de disruption pPTAde2-Ura3Ex (JME844) a été obtenu par insertion du marqueur URA3ex au site I-SceI du plasmide JME813.

La cassette de disruption a été obtenue par digestion NotI et purification de la bande de 3 089 pb. La souche Pold a été transformée par la méthode à l'acétate de lithium Les transformants Ura+ ont étés sélectionnés sur milieu YNBcasa supplémenté avec de l'adénine (100 mg/L). Les concentrations typiques nécessaires pour la complémentation d'une auxotrophie qui sont utilisées sont généralement comprises entre 100 à 200 mg/L. Les transformants Ura+ sont obtenus avec un taux de transformation faible, de l'ordre de 50 transformants par microgramme d'ADN, l'analyse des transformants a révélé qu'ils contenaient une conversion du marqueur ura3-302 et pas la délétion du gène ADE2.

Nous avons réalisé plusieurs essais de disruption avec des concentrations croissance d'adénine. Pour obtenir des taux de transformation supérieur à 103 transformants par µg d'ADN, il est nécessaire d'avoir une concentration supérieure à 400 mg/L d'adénine dans les milieux. La concentration utilisée pour la complémentation est de 800 mg/L. Ce n'est qu'avec ces conditions que nous avons obtenu des transformants présentant une disruption du gène ADE2. Contrairement à *S. cerevisiae,* les souches Ade- ne présentent pas de couleur rose. Par contre, les clones Ade- de *Y. lipolytica* présentent une couleur marron après plusieurs jours sur boite et un surnageant marron en milieu liquide: Phénotype utilisable pour identifier les transformants ou l'intégration de la cassette d'expression s'est bien intégrée au locus ADE2.

Vérification de la séquence du locus ade2-844. La séquence du locus ade2-844 a été amplifiée avec les primers ver1ade2 et ver2ade2 (Tableau 9). Le produit de PCR de taille de 2204 pb a été séquencé. La séquence obtenue montre l'absence de la séquence de marqueur URA3. De plus, cette vérification montre l'absence de l'intégration des sites NotI en 5' et 3' de la cassette de disruption PUexT. Après excision, il reste sur le génome les séquences suivantes entre P et T du locus ade2 : TAGGGATAACAGGGTAATTATCGCTTCGGATAACTCCTGCTATAGGAAGTTA TGTAGGGATAACAGGGTAATTAACTATAACGGTCCTAAGGTAGCGA (SEQ ID NO :12) soit les sites suivants I-SceI - LoxR - I-SceI - I-CeuI.

### 2.2 Construction du marqueur ADE2ex

Pour la construction du marqueur ADE2ex, la séquence génomique du locus ADE2 a été amplifié en utilisant les primers ADE2S et ADE2R et cloné dans le vecteur pKS-LPR (Fickers et al., 2003, Journal of Microbiological Methods, vol. 55, p : 727-737) au site EcoRI. Il contient 500 bp de promoteur, l'ORF du gène ADE2 (Yali0B23188g) et 125 bp du terminateur et qui correspond à la séquence Yali0B:3029961..3032280 soit 2319 pb. Le site EcoRI présent dans l'ORF du gène ADE2 a été éliminé en modifiant la gaatTc en gaatCc par mutagénèse dirigée avec les primers ADE2ES et ADE2ER (Tableau 9). Cette base modifiée crée une mutation silencieuse ATT en ATC.

Le marqueur peut être inséré dans les deux sens. Le marqueur ADE2ex dans le sens 1 correspond à JME798 et dans le sens 2 à JME799.

### 2.3 Transformation avec le marqueur ADE2ex

Pour tester l'utilisation du marqueur ADE2ex, le marqueur ADE2ex a été isolé du plasmide JME798 (pKS-LPR-ADE2) par digestion I-SceI et inséré au site I-SceI du plasmide JMP62 I-SceI (JME793) pour obtenir le plasmide JMP62 ADE2ex (JME862). Le plasmide JME862 a été digéré par NotI et utilisé pour transformer la souche FF-lua par la méthode au lithium acétate. Les transformants ont été sélectionnés sur milieu YNBcasa supplémenté avec de l'uracile (100 mg/L) et de l'adénine. Dans la bibliographie, il est couramment décrit chez des mutants Ade- de différentes espèces de levures les milieux sélectifs sont supplémentés par de l'adénine a une concentration de 100 a 200 mg/L. Dans notre cas pour les mutants Ade- précités, la concentration nécessaire pour une croissance correcte des transformants est de 800 mg/L. Dans ces conditions, nous obtenons des taux de transformations similaires aux taux de transformation obtenu avec le marqueur URA3. 3. Délétion du gène LEU2 (marquer Δleu2-958), obtention des souches mutantes FF2-lu, FF-2lug, FF2-lua et FF2-luag et construction du marqueur LEU2ΔBamHI.

### 3.1 Délétion du gène LEU2 (Δleu2-958)

Pour obtenir ces souches, on peut procéder comme suit : a partir du mutant FF-lu, on a construit la souche FF2-lu Leu-, Ura-. D'une manière similaire on peut construire des souches dérivée de FF-lua, FF-lug, FF-luag présentant la délétion Δleu2-958. A partir du mutant FF-lug, on peut construire la souche FF2-lug Leu-, Ura-, gly-, a partir du mutant FF-lua, on peut construire la souche FF2-lua Leu-, Ura-, ade- et a partir du mutant FF-luag, on peut construire la souche FF2-luag Leu-, Ura-, ade-, gly-.

Par exemple, a partir du mutant FF-lu on a construit la souche *Y. lipolytica* mutante correspondante présentant une délétion du gène LEU2 plus importante que pour le marqueur génétique leu2-270. La première étape est la construction de la souche FF2-lu ::URA3 prototrophe pour l'uracile (Leu-, Ura+) par délétion du gène leu2-270 par la cassette de disruption leu2 ::URA3ex en transformant la cassette NotI issue du plasmide pPTLeu2-Ura3Ex (JME958) contenant ce marqueur et en sélectionnant les Ura+ sur YNBcasa. On sélectionne les auxotrophes pour l'uracile (Leu+, Ura-) par excision du marqueur URA3ex en transformant avec le vecteur réplicatif pRRQ2 contenant la recombinase Cre et le marqueur LEU2 (Cre-LEU2) et en sélectionnant les transformants auxotrophes pour l'uracile, Leu+. La perte du plasmide pRRQ2 est réalisée par une culture sur milieu riche YPD et par l'isolement d'un clone (Leu-, Ura-). La même méthode peut être utilisée pour obtenir les mutants FF2-lua, FF2-lug et FF2-luga.

La représentation schématique de la construction du mutant FF2-lu est résumée dans le Tableau 4 ci-dessous.

**Tableau 4 : Étapes de transformation requises pour la production du mutant FF2-lu**

| Étape | Opérations de transformation | | |
|---|---|---|---|
| | Mutant à transformer | Cassette de transformation | Mutant transformé |
| a1 | FF-lu, Leu-, Ura-JMY195, Po1d | PTLeu2-Ura3Ex | FF-IU+, leu-, Ura+ leu2-PUexT |
| a2 | FF-IU+, Leu-, Ura+, leu2-URA3 | Vecteur pRRQ2, sélection Leu+, vérification Ura-, perte du plasmide pRRQ2 sur YPD, isolement de Leu- | JMY1516, Lieu-, Ura-, leu2-958 FF2-lu |

La cassette de disruption pour le gène LEU2 a été construite par amplification de la région amont (région P ; Yali0C44989..Yali0C46081, 1092 pb) et de la région aval (région T ; Yali0C47036..Yali0C47932, 897 pb) ce qui permet la délétion de l'ORF du gène LEU2 (Yali0C46082..Yali0C47035, 954 pb) et qui ne laisse plus qu'une très faible homologie avec le marqueur de sélection LEU2ex (O bp avec la région P et 207 bp dans la région T) afin de minimiser les conversions géniques. Alors que le marqueur de sélection Leu2Ex présentait une forte homologie avec le marqueur génétique leu2-270 (401 bp avec la région P et 710 bp dans la région T) car celui ci correspond à une simple délétion StuI de 681 bp du gène LEU2. Les marqueurs de sélection ex sont présentés dans le Tableau 11.

Les régions P et T ont été amplifiées avec les primers PILeu2, P2Leu2, TILeu2 et T2Leu2 (Tableau 9).

Les primers P2 et T1 contiennent le site rare I-SceI pour l'insertion du marqueur de sélection excisable Ura3ex. Le primer T1 contient aussi le site rare I-CeuI pour l'insertion de la cassette d'expression. Les primers P1 et T2 contiennent le site rare NotI pour le clonage dans pHSS6-NotI (JME800). Le fragment PT a été cloné dans le JME800 digéré par NotI et déphosphorylé pour donner le plasmide pLeu2-PT dans l'orientation 1 (sens 1, JME811) ou dans l'orientation 2 (sens 2, JME812). Le Sens est défini suivant l'orientation du site I-SceI : soit le sens 1 avec le site I-SceI en sens, soit le sens 2 avec le site I-SceI , en complémentaire. Le plasmide de disruption pPTLeu2-Ura3Ex (JME958) a été obtenu par insertion du marqueur Ura3ex au site I-SceI du plasmide JME811.

La cassette de disruption a été obtenue par digestion NotI et purification de la bande de 3351 pb. Les souches ont été transformées par la méthode à l'acétate de lithium Les transformants Ura+ ont étés sélectionnés sur milieu YNBcasa.

### 3.2 Construction du marqueur LEU2n (LEU2exABamHI)

Pour la construction des cassettes d'expression et des cassettes d'intégration, les marqueurs de sélection ne doivent pas contenir les sites uniques utilisés pour le clonage des gènes d'intèrets (BamHI, HindIII, KpnI et AvrII). Pour la construction du marqueurLEU2n (LEU2exABamHI), les primers Leu2-LI et Leu2-L2 ont été utilisés pour créer un linker apportant la modification ggaatT en ggaatC et possédant le site ApaI (Tableau 9). Le plasmide pKS-LPR-Leu2 (JME509) a été digéré d'une part par ApaI et ScaI et d'autre part par BamHI et ScaI. Les Bandes ApaI-ScaI de 1471 pb et BamHI-ScaI de 3300 pb ont été purifie sur gel d'agarose. Une ligation 3 voies a été effectuée avec ces 2 bandes plus le linker Leu2L1/Leu2L2. Nous avons obtenu un plasmide contenant le nouveau marqueur LEU2n modifié LEU2exΔBamHI (JME790).

### 4. Développement du système d'intégration monocopie aléatoire avec différents marqueurs excisables

La construction a été réalisée a partir des différents marqueurs excisables existants et des marqueurs obtenus dans la présente invention (Tableau 11).

Dans un premier temps, nous avons construit les 2 vecteurs de base nommes I-SceI soient JMP61 I-SceI et le JMP62 I-SceI. A partir des vecteurs JMP61 leu2Ex et JMP62 leu2Ex contenant le marqueur excisable LEU2, nous avons effectué une PCR de la totalité du plasmide avec un couple de primers : 62claS et 62claR (Tableau 9).

Nous avons utilisé le kit « QuikChange® Site-Directed Mutagenesis Kit » de Stratagene qui permet d'effectuer des mutations dirigées. Le principe, basé sur l'amplification par PCR, utilise des amorces construites spécifiquement pour forcer la mutation du produit PCR. Habituellement utilisé pour de courtes insertions/délétions ou des mutations ponctuelles, nous avons utilisé ce principe afin d'éliminer le marqueur Leu2Ex par recircularisation du plasmide au site I-SceI. Ces 2 primers ont été construits aussi pour éliminer les sites BamHI et AvrII contenu dans le plasmide JMP61 Leu2Ex en amont du site I-SceI et de restaurer le site ClaI de façon à permettre l'échange du promoteur par ce site unique. Le produit de PCR obtenu est ensuite digère par l'enzyme de restriction DpnI qui permet d'éliminer le brin parentale. Ce dernier, provenant d'une souche d'*E*. *coli* dam+ est méthylé et, contrairement au brin nouvellement formé, il sera reconnu et digéré spécifiquement par l'enzyme DpnI. Apres transformation et criblage, nous avons obtenu les vecteurs JMP61 I-SceI et JMP62 I-SceI (Tableau 12).

Les autres constructions (Tableau 12) ont été réalisées à partir de ces 2 plasmides. Les ligations ont été effectuées avec les plasmides digérés par I-SceI et déphosphorylé et les bandes purifiées I-SceI des différents marqueurs excisables à partir des plasmides pKS-LPR correspondants (Tableau 11). Pour le marqueur excisable Leu2, nous avons utilise le plasmide JME790 contenant le marqueur LEU2n (Leu2ABamHI).

**Tableau 5 : Souches obtenus :**

| **Souche** | **N° collection** | **Génotype** |
|---|---|---|
| **FF-lu** | JMY 195, Po1d | xpr2-322, leu2-270, ura3-302 |
| **FF2-lu** | | xpr2-322, leu2-958, ura3-302 |
| | | |
| | JMY 1202 | xpr2-322, leu2-270, ura3-302, gut2-PUexT |
| **FF-lug** | JMY 1346 | xpr2-322, leu2-270, ura3-302, gut2-744 |
| | | |
| | JMY 1407 | xpr2-322, leu2-270, ura3-302, ade2-PUexT |
| **FF-lua** | JMY 1409 | xpr2-322, leu2-270, ura3-302, ade2-844 |
| | | |
| | JMY 1396 | xpr2-322, leu2-270, ura3-302, gut2-744, ade2-PUexT |
| **FF-luga** | JMY 1404 | xpr2-322, leu2-270, ura3-302, gut2-744, ade2-844 |
| | | |
| **5LAsp1** | 1 copie | ade2 :: GUT2-Asp |
| **6LAsp1** | 1 copie | ade2 :: GUT2-Asp |
| | | |
| **14LAsp2** | 2 copies | ade2 :: GUT2-Asp lip2 ::URA3-Asp |
| **15LAsp2** | 2 copies | ade2 :: GUT2-Asp lip2 ::URA3-Asp |
| **16LAsp2** | 2 copies | ade2 :: GUT2-Asp lip2 ::URA3-Asp |
| **17LAsp2** | 2 copies | ade2 :: GUT2-Asp lip2 ::URA3-Asp |
| **21LAsp2** | 2 copies | ade2 :: GUT2-Asp lip2 ::URA3-Asp |
| **1LAsp3** | 3 copies | ade2 :: GUT2-Asp lip2 ::URA3-Asp leu2 ::ADE2-Asp. |
| | | |
| **2LAsp4** | 4 copies | ade2 :: GUT2-Asp lip2 ::URA3-Asp leu2 ::ADE2-Asp ura3 ::LEU2-Asp |

### 5. Construction des vecteurs de base, de clonage et d'expression.

### 5.1 Vecteurs de base et de clonage.

La construction du vecteur de base a été réalisée a partir du vecteur JMP62 en conservant que la partie navette *E. coli* provenant du plasmide pHSS6. Après digestion par NotI du vecteur JMP62, la bande de 2210 pb contenant l'origine de réplication pour E. coli et la résistance à la Kanamycine a été isolée et purifiée puis religuée pour former ainsi le vecteur de base nommé pHSS6-NotI (JME800).

A partir du vecteur de base, nous avons construit le vecteur de clonage (figure 2) par ligation de 2 linkers et d'un fragment de PCR contenant les séquences de terminaisons de la lipase LIP2 de *Y. lipolytica* contenu dans le vecteur JMP62. Les linkers ont été fait par synthèse de couple de primer phosphoryle en 5' soit le linker 1 avec les primers link1 et link11 et le linker 2 avec les primers link2 et link22 (Tableau 10). Ces clinkers créent un multisite de clonage contenant respectivement les sites ClaI et BamHI pour l'insertion d'un promoteur, les sites BamHI/HindIII et SacII/KpnI/AvrII pour l'insertion du gène d'intérêt. Nous avons aussi introduit une séquence « consensus » en -4 et -1 de l'ATG : C⁻⁴ A C A⁻¹. Cette séquence est présente pour les gènes de *Y. lipolytica* qui codent pour des protéines fortement exprimées. Ainsi, pour une expression avec cette séquence le clonage du gène d'intérêt s'effectue par HindIII et sans cette séquence par BamHI en 5'. Le fragment de PCR (840 pb) contenant les séquences de terminaisons de LIP2 a été obtenu avec les primers POX2Xho et Lip2Thc. Ce fragment a été digéré par AvrII et NotI pour obtenir le fragment de 179 pb ne contenant que les séquences de terminaisons de LIP2. Nous avons ajouté un site unique HincII de façon à permettre l'échange de ces séquences de terminaison par AvrII et HincII. Nous avons introduit 2 sites rares de l'enzyme de restriction I-CeuI de part et d'autre du multisite et des séquences de terminaison. Ce double site I-CeuI permet d'isoler et de purifier la cassette d'expression pour un clonage dans des vecteurs d'intégration.

Une ligation 3 voies a été réalisée avec les 2 linkers, la bande de 179 pb AvrII/NotI et le vecteur de base pHSS6 NotI déphosphorylé pour obtenir le vecteur de clonage nommé pVC (JME829).

### 5.2 Vecteur d'expression

La construction du vecteur d'expression a été réalisée à partir du vecteur de clonage pVC digéré par ClaI et BamHI. Le promoteur pPOX2 réduit (1017 pb) a été isolé et purifié après digestion par ClaI et BamHI du vecteur JMP62 (Tableau 12). Une ligation du pVC et du promoteur pPOX2 a été réalisée pour obtenir le vecteur d'expression nommé pVC-pPOX2 (JME830), Tableau 6.

**Tableau 6 : Vecteurs utilisés pour la construction du vecteur d'expression.**

| **Descriptifs** | **Nom du vecteur** | **N° collection** |
|---|---|---|
| **Vecteur de base** | pHSS6-NotI | JME800 |
| **Vecteur de clonage** | pVC | JME829 |
| **Vecteur d'expression** | pVC-pPOX2 | JME830 |

### 6. Choix des loci d'insertion et principe de construction des vecteurs d'intégrations. Nous avons choisi les différents loci suivants:

### 6.1 Locus Leu2-270

Ce locus a souvent été utilisé comme cible d'intégration homologue par simple « crossing over » (eg. Bordes et al, 2007), mais avec ce marqueur génomique on obtient un taux élevé de conversion (elle correspond à une petite délétion StuI de 681 pb qui laisse une région 5' de 401 pb (Yali0C:46411..46537) et une région 3' de 710 pb (Yali0C:46528..46948) qui permet une double recombinaison avec le marqueur de sélection LEU2 utilisé dans nos vecteurs. Ainsi, un fort taux de conversion est généralement observé lors des transformations de cassette d'expression contenant ce marqueur. Pour diminuer le taux de conversion, nous avons choisi d'insérer dans une première étape une cassette d'expression au locus leu2-270, afin d'éliminer ces régions d'homologie en 5' et 3'. Ainsi, lors d'une insertion d'une nouvelle cassette d'expression contenant le marqueur LEU2 à un autre locus, le taux de conversion est fortement diminué. Alternativement on peut construire une souche avec le marqueur leu2-958 comme décrit dans le chapitre 3.

### 6.2 Locus Ura3-320

La délétion a été réalisée par double crossing-over ne laissant que 21 pb en position 5' (Yali0E:3171873..3171893) et 108 pb en position 3' (Yali0E:3175134..3175241). Le fragment délété (695 pb) a été remplacé par la cassette d'expression suivante : promoteur du gène XPR2 et le gène SUC2 codant pour l'invertase de *Saccharomyces cerevisiae* en sens inverse (3240 pb, Yali0E:3171894..3175133). L'intégration à ce locus Ura3-320 permet d'éliminer le gène SUC2 provenant de *S. cerevisiae* dans la souche de production et d'effectuer un criblage simple et rapide des transformants Suc-(absence de croissance sur milieu ne contenant que du saccharose comme seule source de carbone) comme décrit dans la Figure 6.

### 6.3 Locus Lip2

Le gène LIP2 code pour la lipase Lip2p de *Y. lipolytica.* Son expression est induite en présence acide gras et de triglycérides (Pignede et al., 2000). Dans la présente invention, l'expression des protéines d'intérêt est placée sous le promoteur fort et inductible pPOX2. Ce promoteur est aussi inductible par les acides gras comme l'acide oléique. L'intégration au locus LIP2 permet d'invalider le gène LIP2 et d'obtenir l'absence d'expression de la lipase Lip2p dans les surnageants de culture lors de la production. De plus, ceci permet d'effectuer un criblage simple et rapide des transformants Lip2-(absence de dégradation d'une émulsion de tributyrine visible sur milieu gélosé ou absence d'activité lipase dans le surnageant de culture comme décrit dans la Figure 7).

### 6.4 Locus Ade2

Dans la présente invention, de nouvelles souches Ade- ont été construites, ainsi que le marqueur de sélection Ade2ex. Pour la disruption du gène ADE2 les séquences P et T ont été amplifié. Ces mêmes séquences ont donc été utilisées dans les vecteurs d'intégration. Ainsi, aucune construction supplémentaire n'a été nécessaire pour ce locus.

Si la souche réceptrice utilisée est FF-lug, l'insertion au locus ADE2 rend la souche Ade-, ce qui crée une nouvelle auxotrophie, qui peut être utilisée pour introduire une copie supplémentaire du gène d'intérêt. Cette disruption est aussi associée à un phénotype rapidement identifiable (coloration marron des colonies disruptées sur milieu riche en boite de pétrie, coloration marron du milieu de culture).

Cette liste ou ces combinaisons de Locus / Marqueur n'est pas exhaustive. Toutes autres combinaisons sont possible, de préférence avec un locus dont la disruption crée un phénotype rapidement visualisable qui permet un crible rapide des transformant ayant intégrés correctement la cassette d'expression au locus. On peut choisir d'autre locus et d'autres marqueurs ou de réaliser d'autres combinaisons.

### 6.5 Principe de construction des vecteurs d'intégration

Le principe de la construction des vecteurs d'intégration est décrit dans la Figure 3. Les différentes cassettes d'intégration à des loci définis ont été construites par une méthodologie identique. A partir de l'ADNg de la souche E150, nous avons amplifié la partie promotrice nommée P et la partie terminatrice nommée T du gène à disrupter. Les fragments P et T doivent, de préférence, avoir une taille comprise entre 800 et 1000 pb pour obtenir une double recombinaison homologue efficace chez *Y. lipolytica.* Les primers utilisés sont nommée P1/P2 pour le fragment P et T1/T2 pour le fragment T suivi du nom du locus (Tableau 9). Les primers P1 et T2 contiennent un site rare 1, ici le site NotI, pour permettre le clonage dans le vecteur de base pHSS6-NotI et aussi pour libérer la cassette d'expression. Les primers P2 et T1 contiennent deux autres sites rares, les sites rare 2 et rare 3, ici les sites I-SceI et I-CeuI, respectivement, qui permet la fusion par PCR des 2 fragments et le clonage ultérieur du marqueur de sélection au site rare 2, ici le site I-SceI et de la cassette d'expression au site rare 3, ici le site I-CeuI. La fusion par PCR est donc réalisée à partir d'un mélange des 2 fragments P et T purifié et du couple de primers P1/T2. Le fragment PT obtenu est digéré par NotI puis est ensuite cloné par ligature dans le vecteur pHSS6 digéré par NotI et déphosphorylé. Les vecteurs d'intégration sont nommés pPT suivi du nom du locus (Tableau 7).

Description des fragments P et T des différents loci :
Locus Leu2-270 : Les régions amplifiées pour la double recombinaison homologue sont pour la région P de 1092 pb (Yali0C:44989..46081) et pour la région T de 897 pb (Yali0C:47036..47932). La région qui sera éliminée est de 954 pb (Yali0C:46082..47035). Cette région élimine la totalité de la région 5' du marqueur Leu2 (401pb) et une partie de la région 3' (503pb). Il reste une homologie de 207 pb de la région 3' du marqueur Leu2.
Locus Ura3-302 : Les régions amplifiées pour la double recombinaison homologue sont pour la région P de 1197 pb (Yali0E:3170698..3171894) et pour la région T de 1013 pb (Yali0E:3175412..3176424). La région qui sera éliminée est de 3516 pb (Yali0E:3171895..3175411). Cette région élimine la totalité de la cassette d'expression pXPR2+SUC2 et la région 3' du marqueur Ura3 (280 pb). Il reste une homologie de 200 pb de la région 5' du marqueur Ura3.
Locus Lip2 : Les régions amplifiées pour la double recombinaison homologué sont pour la région P de 1123 pb (Yali0A:2185897..2187019) et pour la région T de 961 pb (Yali0A:2187883..2188843). La région qui sera éliminée est de 863 pb (Yali0C:2187020..2187882). Cette région élimine presque l'intégralité du gène LIP2. Il reste une homologie de la région 3' de 148 pb du gène LIP2.
Locus Ade2 : Les régions amplifiées pour la double recombinaison homologue sont pour la région P de 920 pb (Yali0B:3029011..3029931) et pour la région T de 792 pb (Yali0B:3031871..3032662). La région qui sera éliminée est de 1939 pb (Yali0B:3029932..3031870) qui ne laisse qu'une très faible homologie avec le marqueur de sélection ADE2ex (O bp avec la région P et 415 bp dans la région T) afin de minimiser les conversions géniques.

Pour chaque locus, les régions P et T ont été amplifiées a l'aide de couple de primers nommé P1/P2 et T1/T2 suivi du nom du locus (Tableau 9). Puis la fusion PT a été réalisée avec le couple P1/T2.

A partir de ces vecteurs pPT, différents marqueurs excisables ont été introduits par clonage au site I-SceI. Ce site permet un clonage orienté du marqueur. Nous avons choisit d'orienter le marqueur en sens inverse de la cassette d'expression. Les vecteurs d'intégration obtenus sont nommés pPT suivi du nom du locus suivi du nom du marqueur excisable inséré (Tableau 7).

Les combinaisons Locus / Marqueur ne sont données qu'à titre d'exemples, différente combinaison sont possibles.

**Tableau 7 : liste des vecteurs d'intégration**

| ***Locus*** | **Nom du vecteur** | **Orientation 1-Scel** | **N° collection** | **Marqueur** | **Nom du vecteur** | **N° collection** |
|---|---|---|---|---|---|---|
| ***LIP2*** | pPTLip2 | Sens 1 | JME806 | URA3ex | pPTLip2-Ura3ex | JME815 |
| | | | | GUT2-0.5ex | pPTLip2-Gut2-0.5ex | JME816 |
| | | Sens 2 | JME807 | | | |
| ***URA3*** | pPTUra3 | Sens 1 | JME809 | LEU2nex | pPTUra3-Leu2nex | JME817 |
| | | Sens 2 | JME810 | | | |
| ***LEU2*** | pPTLeu2 | Sens 1 | JME811 | ADE2ex | pPTLeu2-Ade2ex | JME818 |
| | | | | URA3ex | pPTLeu2-Ura3ex | JME958 |
| | | Sens 2 | JME812 | | | |
| ***ADE2*** | pPTAde2 | Sens 1 | JME813 | GUT2-0.5ex | pPTAde2-Gut2-0.5ex | JME819 |
| | | | | URA3ex | pPTAde2-Ura3ex | JME844 |
| | | Sens 2 | JME814 | JME814 | | |

### 7. Principe de construction des vecteurs d'intégrations pour l'expression de protéine d'intérêt.

Le clonage d'un gène d'intérêt est réalisé seulement en 2 étapes de clonage. La première étape est un sous clonage dans le vecteur d'expression pVC pour obtenir le vecteur pVC-gène d'intérêt sous le contrôle du promoteur POX2 (d'autre promoteur fort peuvent être utilisé constitutif comme par exemple le promoteur hp4d ou des promoteurs inductible). Ce vecteur pVC-gène d'intérêt est digéré par I-CeuI pour isoler et purifier la cassette d'expression. La deuxième étape est le clonage de cette cassette d'expression I-CeuI dans les vecteurs d'intégrations. La cassette d'intégration/cassette d'expression est isolée et purifiée après digestion par NotI avant transformation dans *Y. lipolytica* (Figure 3).

### Exemple 2 : Construction et mise en oeuvre de cassette intégration/expression pour l'insertion ciblée d'un gène codant pour une protéine d'intérêt thérapeutique non produite par Y. lipolytica.

Nous avons pris comme exemple la protéine L-asparaginase *d'Erwinia chrysanthemi.*

On introduit le gène codant pour cette protéine dans le vecteur pVC-pPOX2 au site HindIII/AvrII. Pour ce faire, une fusion par PCR est réalisée entre la séquence preproLIP2 et la partie de la séquence du gène codant pour la forme mature de la protéine L-asparaginase. Les PCR sont réalisés avec les primers preproLip2 et Lip2KR pour amplifier la séquence pre-pro-LIP2 et avec les primers Laspsens et Lasprev pour amplifier la séquence codante pour la protéine mature. Puis en mélangeant les 2 produits de PCR obtenus, on réalise la fusion avec les primers preproLip2 et Lasprev. Le primer preproLip2 contient le site HindIII et le primer Lasprev le site AvrII pour le clonage du produit de la fusion dans le pCV-pPOX2. On obtient alors le vecteur pVC-pPOX2-preproLip2-LAsp (JME898) contenant la cassette d'expression de la protéine L asparaginase (Tableau 8).

A partir de ce vecteur, la cassette d'expression est extraite grâce à une digestion par l'enzyme de restriction I-CeuI et purifiée. Cette cassette est ensuite clouée dans les différents vecteurs d'intégration au site I-CeuI. Les différents vecteurs d'intégration/expression obtenus sont décrits dans le Tableau 8.

**Tableau 8 : Vecteur d'intégration/expression obtenus :**

| **Descriptifs** | **Nom du vecteur** | **N° collection** |
|---|---|---|
| **Vecteur d'expression** | pVC₌pPOX2-LAsp | JME898 |
| | | |
| **Vecteur Int. locus Ura3** | pPTUra3-Leu2nEx-LAsp | JME923 |
| **Vecteur Int. locus Ade2** | pPTAde2-Gut2-0.5Ex-LAsp | JME924 |
| **Vecteur Int. locus Ade2** | pPTAde2-Ura3Ex-LAsp | JME925 |
| **Vecteur Int. locus Leu2** | pPTLeu2-Ade2Ex-LAsp | JME926 |
| **Vecteur Int. locus Lip2** | pPTLip2-Ura3Ex-LAsp | JME927 |
| **Vecteur Int. locus Ade2** | pPTAde2-Gut2-10Ex(s1)-LAsp | JME941 |
| **Vecteur Int. locus Ade2** | pPTAde2-Gut2-1.0Ex(s2)-LAsp | JME942 |
| **Vecteur Int. locus Ade2** | pPTAde2-Gut2-1.5Ex(s1)-LAsp | JME943 |
| **Vecteur Int. locus Ade2** | pPTAde2-Gut2-l .SEx(s2)-LAsp | JME944 |

Les cassettes d'intégration/expression sont obtenues par digestion de l'enzyme de restriction NotI des différents vecteurs d'intégration/expression. Ces cassettes sont ensuite extraites et purifiées afin d'éliminer les séquences d'ADN exogène *E coli.*

### Exemple 3 : Transformation des cassettes intégration/expression de la protéine L-asparaginase chez Y. lipolytica.

On peut introduire successivement par exemple 4 copies de la cassette d'expression de la L-asparaginase dans les 4 loci précités dans la souche FF-luga.

### 1 Insertion de la première copie au locus ADE2.

La première cassette d'expression a été introduite par transformation de la souche FF-luga au locus ADE2 en utilisant la cassette d'intégration/expression PTAde2-Gut2-1.OEx-LAsp issue du vecteur JME941. La sélection des transformant est réalisée sur milieu sélectif YNBcasa supplémenté d'uracile et d'adénine et contenant comme seule source de carbone 1% de glycérol. Les transformants obtenus sont insoles sur ce même milieux sélectif et l'intégration au locus est vérifiée par PCR en utilisant les primers Verlade2 et Ver2ade2 se trouvant en amont et en aval des séquences d'intégration.

Le taux de transformation obtenu est de 8,2 10³ transformants par µg d'ADN transformé. La vérification par PCR de l'intégration au locus Ade2 a montré que 100% des transformants ont intégré la cassette d'expression LAsp à ce locus.

Nous avons conservé 2 transformants nommés 5LAsp1 et 6LAsp1 (Tablreau 5). Les premiers chiffres au début du nom donnent le numéro du transformant et le deuxième chiffre à la fin du nom donne le nombre de copie de la cassette d'expression LAsp.

### 2 Insertion de la Deuxième copie au locus LIP2.

La deuxième cassette d'expression a été introduite par transformation du transformant 5LAsp1 au locus LIP2 en utilisant la cassette d'intégration/expression PTLip2-Ura3Ex-LAsp issue du vecteur JME927. La sélection des transformant est réalisée sur milieu sélectif YNBcasa supplémenté d'adénine et contenant comme seule source de carbone 1% de glucose. Les transformants obtenus sont isoles sur ce même milieux sélectif et l'intégration au locus est vérifiée par PCR en utilisant les primers Ver1lip2 et Ver2lip2 se trouvant en amont et en aval des séquences d'intégration.

Le taux de transformation obtenu est de 1,1 10⁴ transformants par µg d'ADN transformé. La vérification par PCR de l'intégration au locus LIP2 a montré que 75% des transformants ont intégré la cassette d'expression LAsp à ce locus. Alternativement, la vérification de l'intégration de la cassette d'expression au locus LIP2 peut être réalisée par mesure de l'activité lipase comme démontrée dans la Figure 7

Nous avons conservé 5 transformants nommés 14LAsp2, 15LAsp2,16LAsp2, 17LAsp2 et 21 LAsp2 (Tableau 5).

### 3 Insertion de la troisième copie au locus LEU2.

La troisième cassette d'expression a été introduite par transformation du transformant 15LAsp2 au locus LEU2 en utilisant la cassette d'intégration/expression PTLeu2-Ade2Ex-LAsp issue du vecteur JME926. La sélection des transformants est réalisée sur milieu sélectif YNBcasa contenant comme seule source de carbone 1% de glucose. Les transformants obtenus sont isoles sur ce même milieux sélectif et l'intégration au locus est vérifiée par PCR en utilisant les primers Ver1leu2 et Ver2leu2 se trouvant en amont et en aval des séquences d'intégration.

Le taux de transformation obtenu est de 1,6 10⁴ transformants par µg d'ADN transformé. La vérification par PCR de l'intégration au locus LEU2 a montré que 20% des transformants ont intégré la cassette d'expression LAsp à ce locus.

Nous avons conservé 1 transformant nommé 1LAsp3 (Tableau 5).

### 4 Insertion de la quatrième copie au locus URA3.

La quatrième cassette d'expression a été introduite par transformation du transformant 1LAsp3 au locus URA3 en utilisant la cassette d'intégration/expression PTUra3-Leu2nEx-LAsp issue du vecteur JME923. La sélection des transformants est réalisée sur milieu sélectif YNB contenant comme seule source de carbone 1% de glucose. Les transformants obtenus sont isoles sur ce même milieux sélectif et l'intégration au locus est vérifiée par PCR en utilisant les primers Ver1Ura3 et Ver2Ura3 se trouvant en amont et en aval des séquences d'intégration.

Le taux de transformation obtenu est de 5,25 10³ transformantes par µg d'ADN transformé. La vérification par PCR de l'intégration au locus URA3 a montré que 87% des transformants ont intégré la cassette d'expression LAsp à ce locus, 87% des transformants sont Ura- (figure 6).

Nous avons conservé 1 transformant nommé 2LAsp4 (Tableau 5).

Cette dernière souche contient bien au moins une copie supplémentaire (voir Figure 5 (gel électrophorèse) et Figure 8 quantification par RT-qPRC.

### 5 Variante du procédé d'insertion :

L'invention peut aussi être mise en oeuvre selon les étapes suivantes : dans un 1er temps, les 4 (par exemple) transformations sont réalisées les unes après les autres. Dans un deuxième temps, on réalise le criblage des insertions à l'aide des différents marqueurs. D'où l'intérêt, si on ne met pas en oeuvre le système Cre-lox, de détenir autant de système de criblage différents que de copies à intégrer.

### Exemple 4 : Analyse de l'expression de L-Asparaginase en fonction du nombre de copie intégré.

L'expression de L-Asparaginase a été analysée en production à partir des différents transformants obtenus contenant 1 à 4 copies. La production a été réalisée en fiole de 250 mL contenant 25 mL de milieu riche compose de Yeast Extract 1%, de Bacto Tryptone 2%, Glucose 1%, tampon phosphate de sodium 50 mM pH6.8 et d'une émulsion acide oléique 2% (nommé Y1T2D1O2). L'acide oléique sert d'inducteur du promoteur pPOX2. L'expression est analysé par gel d'électrophorèse en conditions dénaturante et réductrice à partir du surnageant des cultures. Le gel d'électrophorèse est révélé par coloration au bleu colloïdal et par western blot en utilisant des anticorps polyclonaux de lapin dirigés contre L-Asparaginase. Les résultats de cette analyse sont présentés Figure 5.

### Exemple 5 : Criblage sur boite des transformants obtenus par insertion de la cassette intégration/expression au locus URA3.

Comme décrit précédemment, l'insertion au locus URA3 permet d'éliminer le gène hétérologue SUC2 codant pour invertase de *S. cerevisiae*. Les transformants ayant intégrés la cassette d'expression deviennent alors Suc2-. Ils ne sont plus capables de pousser sur saccharose comme seule source de carbone. Les transformants sont étalés en strie sur 2 milieux riches : YPD (glucose 1%) et YPS (saccharose 1%). Les résultats de cette analyse sont présentés Figure 6. Par ce criblage, nous confirmons les résultats obtenus par analyse par PCR avec les primers Ver1Ura3 et Ver2Ura3.

### Exemple 6 : Criblage des transformants obtenus par insertion de la cassette intégration/expression au locus LIP2.

Comme décrit précédemment, l'insertion au locus LIP2 permet d'éliminer le gène LIP2 codant pour la lipase lip2p extracellulaire de *Y. lipolytica.* Les transformants ayant intégrés la cassette d'expression deviennent alors Lip2-. Lors d'une production en milieu contenant un inducteur comme l'acide oléique, les transformants ne secrètent plus la lipase dans le surnageant de culture. Le criblage est effectue par culture en milieu de production Y1T2D1O2 pendant 48 heures à) 28°C et une agitation de 180 rpm. La présence de la lipase dans le surnageant est détecté par un test enzymatique en microplaque à partir de 20 µL de surnageant. Les résultats de cette analyse sont présentés Figure 7. Par ce criblage, nous confirmons les résultats obtenus par analyse par PCR avec les primers Ver1Lip2 et Ver2Lip2.

### Exemple 7 : Analyse des transformants obtenus par RT-qPCR.

Le nombre de copies intégrées peut être analyses par une PCR quantitative en temps réel par amplification spécifique d'un amplicon (150 pb) du gène codant pour la L-Asparaginase. A partir d'ADNg des transformants obtenus, nous avons effectue une RT-qPCR à l'aide des primers LAsp1 et LAsp2 spécifique du gène codant pour L-Asparaginase et des primers Act4 et Act5 spécifique du gène codant pour l'actine de Y. lipolytica. Cette RT-qPCR a été réalisée à l'aide du kit LightCycler Faststart DNA Master SyBR Green I (Roche). L'amplicon de l'actine permet d'être un gène dit de ménage dont l'expression est connue pour ne pas être modifiée entre les conditions testées et dans notre cas un gène présent en seul copie dans le génome. Ainsi, les résultats sont normalisés avec cet amplicon. Nous avons effectue aussi une RT-qPCR a l'aide des primers Lip2-1 et Lip2-2 spécifique du gène codant pour la lipase Lip2p de *Y. lipolytica* pour vérifier l'absence du gène à partir de la deuxième cassette d'expression intégré au locus LIP2. Les résultats de cette analyse sont présentés Figure 8.

**Tableau 9 : Listes des primers utilisés pour les cassettes de disruption, les vérifications et la construction des nouveaux marqueurs.**

| | Primer | 5'-3' | S/R | Sites |
|---|---|---|---|---|
| **Leu2EX** | LEU2-L1 | GATCTGCTAGTGTATAAGACTCTATAAAAAGGGCC | S | ΔBamHI |
| | LEU2-L2 | CTTTTTATAGAGTCTTATACACTAGCA | R | ΔBamHI |
| **Marque ur GUT2** | PG3PTS | CCGGAATTCCTGACCAGTCTCACATCCGACC | S | EcoRI |
| | PG3PTR | CCGGAATTCTAAAGCAGATACTCAACAACTCAGCAATAGTC | R | EcoRI |
| | G3PB1S | GGAGCTACCGGCTCCGGTATCGC | S | ΔBamHI |
| | G3PB1R | GCGATACCGGAGCCGGTAGCTCC | R | ΔBamHI |
| | G3PB2S | CGGAAACGCTGGATCTTTCAACATCAAGGCC | S | ΔBamHI |
| | G3PB2R | GGCCTTGATGTTGAA:4GATCCAGCGTTTCCG | R | ΔBamHI |
| | G3PES | GGAGCAGGAGTTCAACACCGGTGTCG | S | ΔEcoRI |
| | G3PER | CGACACCGGTGTTGAACTCCTGCTCC | R | ΔEcoRI |
| **ΔGUT2** | G3PD-P1 | GCAGATCCACTGTCAAGCCG | S | |
| | G3PD-P2 | GCTAGGGATAAACAGGGTAATGCGGTAGGAAAGAGAAGTTCCGCG | R | I-SceI |
| | G3PD-T1 | GCATTACCCTGTTATCCCTAGCCGGACTATTTCCCCGCAGC | S | I-SceI |
| | G3PD-T2 | GCAGCCAGCAGCACGTAGTAG | R | |
| | G3PD-Ver1 | GAATGACGGGGGCAACGCAG | S | |
| | G3PD-Ver2 | CAGCAGCCACAAATAGCAGACTGCC | R | |
| **pGUT2-1.0 et 1.5** | P1KB | CCGGAATTCCGTGACAACGGATGATGCTGTCACATGACG | S | EcoRI |
| | P15KB | CCGGAATTCCTGTTGGCATGCACAGCTCCACTCAACG | S | EcoRI |
| | PNHE1 | TCCCGCTAGCCGAGGTATGCAAGGACGAGTGC | R | NheI |
| **Locus ADE2** | P1ADE2 | ATAAGAATGCGGCCGCGCGAGCTGAGAGCCGATACCAAGGGATGCGAG | S | NotI |
| | P2ADE2 | CATTACCCTGTTATCCCTACAGACACAGGTCCCAGGCGTCGGTTCTCG | R | I-SceI |
| | T1ADE2 | | S | I-SceI /I-CeuI |
| | T2ADE2 | ATAGTTTAGCGGCCGCGGAAGCGTGTCAACGACATGTTCCCTCTTCATACC | R | NotI |
| | Ver1Ade2 | CGACGATAGAGCAGGTCTCACTOTTGGGAATGCTG | S | |
| | Ver2Ade2 | CTACAGTGACGAAGTGGACATCCCGGCTTGGACTG | R | |
| **Marque** ur **ADE2** | ADE2S | CGCGAATTCGCCTGCTTGAAAGAAGTGAGTGGTATGCTCGG | S | EcoRI |
| | ADE2R | CGCGAATTCCATTGCCACGACCTGTTAAAAGACAAGATGACC | R | EcoRI |
| | ADE2ES | GCTGGCCATCCGAATCCTGGCTGCTTACGACGCC | S | ΔEcoRI |
| | ADE2ER | GGCGTCGTAAGCAGCCAGGATTCGGATGGCCAGC | R | ΔEcoRI |
| **Locus Lip2** | P1Lip2 | ATAAGAATGCGGCCGCGTGGTACGTTTCGTCGCATCGGACGAG | S | NotI |
| | P2Lip2 | CATTACCCTGTTATCCCTAGAGAGCTGGTACTTGGGTATCAATTGAGG | R | I-SceI |
| | T1Lip2 | | S | I-SceI /I-CeuI |
| | T2Lip2 | ATAGTTTAGCGGCCGCGTGGACACAAGGAAGTATGCGGTCGTCG | R | NotI |
| | Lip2Ver1 | CTCTGTCAGTGTTCGGATAAGTCCTTAGATCACC | S | |
| | Lip2Ver2 | CCTCACTTCTGTCACAGATGATGCATTCAACAC | R | |
| **Locus Leu2** | P1Leu2 | ATAAGAATGCGGCCGCGACACTACTCTGGCTACAGCTTGCGGTACTG | S | NotI |
| | P2Leu2 | CATTACCCTGTTATCCCTAGCTCGAATAACATTCACAGGCTTGGTG | R | I-SceI |
| | T1Leu2 | CTAGGGATAACAGGGTAATTAACTATAACGGTCCTAAGGTAGCGAGGTGT GTTTGTAGTGGAGGACAGTGGTACG | S | I-Scel /I-CeuI |
| | T2Leu2 | ATAGTTTAGCGGCCGCGTACTGAACCGACTTTGGTGCTTGCACTC | R | NotI |
| | Leu2Ver1 | CTCCACGCTAATGCCCATCATACTCTGTTTGGC | S | |
| | Leu2Ver2 | CCTGGCGTTACAAAGCCGAGGGAGACAGCCTTGAC | R | |
| **Locus Ura3** | P1Ura3 | ATAAGAATGCGGCCGCGGTGACACTGCACTATTGGTTTGCTTCTGATG | S | NotI |
| | P2Ura3 | CATTACCCTGTTATCCCTAGTCGAGCTTCGTAGGAGGGCATTTTGGTGG | R | I-SceI |
| | T1Ura3 | | S | I-SceI /I-CeuI |
| | T2Ura3 | ATAGTTTAGCGGCCGCCTATGCTACGAGCGTCGGATTCACCACAG | R | NotI |
| | Ura3Ver1 | GGCACACTGCTCACTATCGCAGGCTGCAACAATG | S | |
| | Ura3Ver2 | CCTGACTCGTCTCGATACTCAAGACCTCATTGACGC | R | |

**Tableau 10 : Liste des primers utilisés pour la construction du vecteur pVC et la PCR quantitative, RT-qPCR.**

| | Primer | 5'-3' | S/R | Sites |
|---|---|---|---|---|
| **Poly linker pVC** | Link1 | GGCCGCTAACTATAACGGTCCTAAGGTAGCG | S | |
| | Link11 | CCGAATCGATTCGCTACCTTAGGACCGTTATAGTTAGC | R | |
| | Link2 | AATCGATTCGGATCCCACAATGAAGCTTCCCCGCGGTAC | S | |
| | Link22 | CTAGGTACCGCGCGGAAGCTTCATTGTGGGAT | R | |
| | Lip2Thc | TATCAAATGCGGCCGCTTCGCTACCTTAGGACCGTTATAGT TAACACGATTCGATTTGTCTTAGAGGAACGC | R | NotI |
| | POX2Xh o | GAGTAATGCTCGAGTATCGAAGTCTTGTACC | S | XhoI |
| **JMP62 ISceI** | 62claS | | S | |
| | 62claR | CACTTGTTCGTCTTGTGGGAATCGATTCTAGGGATAACAGGGTAATATCGC | R | |
| **Gene LAsp** | preproLi p2 | CGCGGATCCCACAATGAAGCTTTCCACCATCCTTTTCACAGCCTGCGCTAC | S | BamHI HindIII |
| | Lip2KR | TCGCTTCTGGAGAACTGCGGCCTC | R | |
| | LAspsen s | | S | |
| | LAsprev | CCACCTAGGCTAGTAGGTGTGGAAGTACTCCTGGATG | R | AvrII |
| **qPCR Lip2** | LIP2-1 | TACTCTTGGTCAGCCCAT | S | |
| | LIP2-2 | AGAAGGGCACTTGAGGGA | R | |
| **qPCR Act** | Act4 | TATTGCCGAGCGAATGC | S | |
| | Act5 | CTTGGAGATCCACATCTGC | R | |
| **qPCR LAsp** | LAsp1 | GCTGAACGACCGAATTGG | S | |
| | LAsp2 | GTGGTGTGCAGCTTGTC | R | |

**Tableau 11 : Liste des marqueurs excisables construits.**

| **Marqueur** | **Nom du vecteur** | **N° collection** |
|---|---|---|
| **ADE2** | pKS-LPR-Ade2 (sens1) | JME798 |
| | pKS-LPR-Ade2 (sens2) | JME799 |
| **GUT2-0.5** | pKS-LPR-Gut2-0.5 (sens1) | JME792 |
| **GUT2-1.0** | pKS-LPR- Gut2-1.0 (sens1) | JME919 |
| | pKS-LPR- Gut2-1.0 (sens2) | JME920 |
| **GUT2-1.5** | pKS-LPR-Gut2-1.5 (sens1) | JME921 |
| | pKS-LPR- Gut2-1.5 (sens2) | JME922 |
| **LEU2** | **pKS-LPR-Leu2 (sens2)*** | **JME509** |
| **LEU2n** | pKS-LPR-Leu2ΔBamHI (sens2) | JME790 |
| **URA3** | **pKS-LPR-Ura3 (sens1)*** | **JME507** |
| **HYG** | **pKS-LPR-hph (sens2)*** | **JME508** |

| | | |
|---|---|---|
| *** Fickers et al., Journal of Microbiological Methods 55 (2003) 727-737 | | |

**Tableau 12 : Liste des vecteurs d'intégration aléatoire, séries JMP61 et JMP62.**

| **Nom du vecteur** | **N° collection** |
|---|---|
| **JMP61 I-SceI** | JME793 |
| **JMP61 Leu2nEx** | JME794 |
| **JMP61 Ura3Ex** | JME795 |
| **JMP61 HygEx** | JME796 |
| **JMP61 Gut2-0.5Ex** | JME797 |
| **JMP61 Ade2Ex** | JME861 |
| | |
| **JMP62 I-Scel** | JME801 |
| **JMP62 Leu2nEx** | JME802 |
| **JMP62 Ura3Ex** | JME803 |
| **JMP62 HygEx** | JME804 |
| **JMP62 Gut2-0.5Ex** | JME805 |
| **JMP62 Ade2Ex** | JME862 |

## Revendications

1. Procédé d'intégration ciblée d'au moins trois copies d'un gène d'intérêt dans le génome d'une souche de *Yarrowia* comprenant les étapes de :
a) mise en culture d'une souche de *Yarrowia* comprenant au moins trois délétions choisies parmi Ura3-302, Leu2-270, Gut2-744 et Ade2-844, le phénotype associé à chacune de ces délétions correspond à une auxotrophie pour ladite souche, indépendamment l'un de l'autre de ces au moins trois gènes ;
b) transformation de ladite souche de *Yarrowia* auxotrophe avec au moins trois vecteurs recombinants qui comprennent des marqueurs de sélection permettant, pour ladite souche de *Yarrowia,* la complémentation de l'auxotrophie résultant de chacune desdites délétions, lesquels vecteurs recombinants comprenant chacun :
i) la séquence dudit gène d'intérêt,
ii) un marqueur de sélection, et
iii) deux séquences d'ADN encadrant la séquence dudit gène d'intérêt et ledit marqueur de sélection, lesquelles deux séquences d'ADN sont homologues aux séquences correspondant aux extrémités du site d'intégration ciblé dans le génome de ladite souche de *Yarrowia* de sorte de permettre l'intégration ciblée dudit vecteur recombinant par recombinaison homologue ;
sélection sur un milieu minimal, des levures ayant intégré lesdits au moins trois vecteurs recombinants.

2. Procédé d'intégration ciblée selon la revendication 1, **caractérisé en ce que** ladite souche comprend en outre au moins une délétion d'un gène associé à un caractère dominant choisi parmi le gène de résistance à l'hygromycine HPH (HYG), et les gènes MDR3, KanMX, et Tn5ble, le gène HYG étant le plus préféré.

3. Procédé d'intégration ciblée selon l'une des revendications 1 ou 2, **caractérisé en ce qu'**à l'étape b), le site d'intégration ciblé dans le génome de ladite souche de *Yarrowia* est choisi parmi les gènes URA3, LEU2, ADE2, LIP2, LIP7, LIP8, AXP, GUT2 et XPR2.

4. Procédé d'intégration ciblée selon la revendication 3, **caractérisé en ce qu'**à l'étape b), le site d'intégration ciblé dans le génome de ladite souche de *Yarrowia* est choisi parmi les gènes URA3, LEU2, ADE2, LIP2, LIP8 et AXP.

5. Procédé selon l'une quelconque des revendications 1 à 4, **caractérisé en ce que** les étapes b) de transformation de la souche de *Yarrowia* et c) de sélection sur milieu minimal sont effectuées séparément pour chacun desdits au moins trois vecteurs recombinants.

6. Procédé d'intégration ciblée selon l'une des revendications 1 à 4, **caractérisé en ce qu'**à l'étape b), les transformations avec au moins les trois vecteurs recombinants sont réalisées de manière indépendante et successivement avec chacun des vecteurs recombinants, ou simultanément avec l'ensembles desdits vecteurs recombinants, et **en ce que** l'ensemble de ces transformations est suivie d'une étape c) de sélection sur un milieu minimal des levures ayant intégré l'ensemble desdits vecteurs recombinants.

7. Procédé selon l'une des revendications 1 à 6, **caractérisé en ce que** ladite souche est une souche de *Yarrowia lipolytica.*

8. Procédé selon l'une quelconque des revendications 1 à 6, **caractérisé en ce que** ladite souche de *Yarrowia* comprend quatre gènes présentant indépendamment l'un de l'autre une délétion associée à un phénotype d'auxotrophie ou de caractère dominant, de préférence choisis parmi les gènes URA3, LEU2, GUT2 et ADE2.

9. Procédé selon l'une quelconque des revendications 1 à 7, **caractérisé en ce que** l'étape b) de transformation est effectuée avec trois à dix vecteurs recombinants.

10. Procédé selon l'une quelconque des revendications 1 à 9, **caractérisé en ce que** le marqueur de sélection est encadré par des séquences permettant son excision après l'intégration ciblée dudit vecteur recombinant.

11. Procédé de production d'un polypeptide codé par un gène d'intérêt, **caractérisé en ce qu'**il comprend les étapes suivantes:
d) de culture de la souche de *Yarrowia* modifiée obtenue par le procédé selon l'une quelconque des revendications 1 à 10 dans un milieu de culture liquide comprenant des sources assimilables de carbone, d'azote et de sels inorganiques de sorte de permettre la croissance de ladite souche transformée de *Yarrowia;* et
e) la récupération dudit polypeptide d'intérêt exprimé à partir des cellules *Yarrowia* ou du milieu de culture obtenus à l'étape d).

12. Procédé d'obtention d'une souche modifiée de *Yarrowia,* **caractérisée en ce qu'**il comprend une étape dans laquelle on transforme ladite souche de *Yarrowia* avec au moins trois vecteurs permettant d'aboutir à une souche de *Yarrowia* comprenant au moins trois délétions choisies parmi Ura3-302, Leu2-270, Gut2-744 et Ade2-844, le phénotype associé à chacune de ces délétions correspond à une auxotrophie ou à un caractère dominant pour ladite souche, indépendamment l'un de l'autre de ces au moins trois gènes.

13. Procédé selon la revendication 12, **caractérisé en ce que** ladite souche comprend en outre au moins un gène associés à un phénotype de caractère dominant choisi parmi le gène de résistance à l'hygromycine HPH (HYG), et les gènes MDR3, KanMX, et Tn5ble, le gène HYG étant le plus préféré.

14. Souche de *Yarrowia* auxotrophe susceptible d'être obtenue par le procédé selon l'une des revendications 12 et 13, laquelle souche comprend au moins trois délétions choisies parmi Ura3-302, Leu2-270, Gut2-744 et Ade2-844, le phénotype associé à chacune de ces délétions correspond à une auxotrophie ou à un caractère dominant pour ladite souche, indépendamment l'un de l'autre de ces au moins trois gènes.

15. Souche de *Yarrowia* selon la revendication 14 , **caractérisée en ce qu'**elle est choisie dans le groupe consistant en la souche de *Yarrowia lipolytica* déposées le 4 février 2008 conformément au traité de Budapest auprès de la Collection nationale de Culture de Microorganismes (CNCM), INSTITUT PASTEUR, 25 rue du Docteur Roux, 75724 PARIS CEDEX 15, France sous les numéros CNCM 1-3911 et CNCM I-3912 et la souche de *Yarrowia lipolytica* déposée le 4 février 2008 conformément au traité de Budapest auprès de la Collection nationale de Culture de Microorganismes (CNCM), INSTITUT PASTEUR, 25 rue du Docteur Roux, 75724 PARIS CEDEX 15, France sous le numéro CNCM 1-3913.

## Patentansprüche

1. Verfahren zur gezielten Integration von mindestens drei Kopien eines bestimmten Gens in das Genom eines Yarrowia-Stamms, umfassend die folgenden Schritte:
a) Kultivieren eines *Yarrowia*-Stamms, umfassend mindestens drei Deletionen, ausgewählt aus Ura3-302, Leu2-270, Gut2-744 und Ade2-844, wobei der Phänotyp, der mit jeder dieser Deletionen assoziiert ist, einer Auxotrophie für den Stamm entspricht, untereinander unabhängig von diesen mindestens drei Genen;
b) Transformieren des auxotrohphen Yarrowia-Stamms mit mindestens drei rekombinanten Vektoren, die Selektionsmarker umfassen, die, für den Yarrowia-Stamm, die Komplementierung der Auxotrophie ermöglichen, die sich aus jeder dieser Deletionen ergibt, wobei die rekombinanten Vektoren jeweils Folgendes umfassen:
i) die Sequenz des bestimmten Gens,
ii) einen Selektionsmarker; und
iii) zwei DNA-Sequenzen, die die Sequenz des bestimmten Gens und den Selektionsmarker umgeben, wobei die zwei DNA-Sequenzen homolog zu den Sequenzen sind, die den Enden der Ziel-Integrationsstelle im Genom des Stammes von *Yarrowia*-Stamms entsprechen, um die gezielte Integration des rekombinanten Vektors durch homologe Rekombination zu ermöglichen;
Auswählen, auf einem minimalen Medium, der Hefen, in die die mindestens drei rekombinanten Vektoren integriert sind.

2. Verfahren zur gezielten Integration nach Anspruch 1, **dadurch gekennzeichnet, dass** der Stamm außerdem mindestens eine Deletion eines Gens umfasst, das mit einem dominanten Merkmal assoziiert ist, ausgewählt aus dem Hygromycin HPH (HYG)-resistenten Gen und den Genen MDR3, KanMX und Tn5ble, wobei das Gen HYG am bevorzugtesten ist.

3. Verfahren zur gezielten Integration nach einem der Ansprüche 1 oder 2, **dadurch gekennzeichnet, dass** in Schritt b) die Ziel-Integrationsstelle im Genom des Yarrowia-Stamms ausgewählt ist aus den Genen URA3, LEU2, ADE2, LIP2, LIP7, LIP8, AXP, GUT2 und XPR2.

4. Verfahren zur gezielten Integration nach Anspruch 3, **dadurch gekennzeichnet, dass** in Schritt b) die Ziel-Integrationsstelle im Genom des *Yarrowia-*Stamms ausgewählt ist aus den Genen URA3, LEU2, ADE2, LIP2, LIP8 und AXP.

5. Verfahren nach einem der Ansprüche 1 bis 4, **dadurch gekennzeichnet, dass** die Schritte b) des Transformierens des *Yarrowia*-Stamms und c) des Auswählens auf dem minimalen Medium getrennt für jeden der mindestens drei rekombinanten Vektoren durchgeführt werden.

6. Verfahren zur gezielten Integration nach einem der Ansprüche 1 bis 4, **dadurch gekennzeichnet, dass** in Schritt b) die Transformationen mit mindestens den drei rekombinanten Vektoren auf unabhängige Weise und aufeinander folgend mit jedem der rekombinanten Vektoren oder gleichzeitig mit den Einheiten der rekombinanten Vektoren durchgeführt werden, und dadurch, dass die Einheit dieser Transformationen von einem Schritt c) des Auswählens auf einem minimalen Medium der Hefen, in denen die Einheit der rekombinanten Vektoren integriert ist, gefolgt wird.

7. Verfahren nach einem der Ansprüche 1 bis 6, **dadurch gekennzeichnet, dass** der Stamm ein *Yarrowia-*Stamm ist.

8. Verfahren nach einem der Ansprüche 1 bis 6, **dadurch gekennzeichnet, dass** der Yarrowia-Stamm vier Gene umfasst, die untereinander unabhängig eine Deletion aufweisen, die mit einem Phänotyp mit Auxotrophie oder mit dominantem Merkmal assoziiert ist, vorzugsweise ausgewählt aus den Genen URA3, LEU2, GUT2 und ADE2.

9. Verfahren nach einem der Ansprüche 1 bis 7, **dadurch gekennzeichnet, dass** der Schritt b) des Transformierens mit drei bis zehn rekombinanten Vektoren durchgeführt wird.

10. Verfahren nach einem der Ansprüche 1 bis 9, **dadurch gekennzeichnet, dass** der Selektionsmarker von Sequenzen umgeben ist, die seine Exzision nach der gezielten Integration des rekombinanten Vektors ermöglichen.

11. Verfahren zur Herstellung eines Polypeptids, das durch ein bestimmtes Gen codiert ist, **dadurch gekennzeichnet, dass** es die folgenden Schritte umfasst:
d) Kultivieren des modifizierten Yarrowia-Stamms, erhalten durch das Verfahren nach einem der Ansprüche 1 bis 10, in einem flüssigen Kulturmedium, umfassend assimilierbare Quellen aus Kohlenstoff, Stickstoff und anorganischen Salzen, um das Wachstum des transformierten Yarrowia-Stamms zu ermöglichen; und
e) Rückgewinnung des bestimmten Polypeptids, exprimiert auf der Grundlage der *Yarrowia*-Zellen oder des Kulturmediums, erhalten in Schritt d).

12. Verfahren zum Erhalt eines modifizierten Yarrowia-Stamms, **dadurch gekennzeichnet, dass** es einen Schritt umfasst, in dem der Yarrowia-Stamm mit mindestens drei Vektoren transformiert wird, wodurch ermöglichet wird, einen Yarrowia-Stamm zu erhalten, der mindestens drei Deletionen umfasst, ausgewählt aus Ura3-302, Leu2-270, Gut2-744 und Ade2-844, wobei der Phänotyp, der mit jeder dieser Deletionen assoziiert ist, einer Auxotrophie oder einem dominanten Merkmal für den Stamm entspricht, untereinander unabhängig von diesen mindestens drei Genen.

13. Verfahren nach Anspruch 12, **dadurch gekennzeichnet, dass** der Stamm außerdem mindestens ein Gen umfasst, das mit einem Phänotyp mit dominantem Merkmal assoziiert ist, ausgewählt aus dem Hygromycin HPH (HYG)-resistenten Gen und den Genen MDR3, KanMX und Tn5ble, wobei das Gen HYG am bevorzugtesten ist.

14. Auxotropher Yarrowia-Stamm, der durch das Verfahren nach einem der Ansprüche 12 und 13 erhalten werden kann, wobei der Stamm mindestens drei Deletionen umfasst, ausgewählt aus Ura3-302, Leu2-270, Gut2-744 und Ade2-844, wobei der Phänotyp, der mit jeder dieser Deletionen assoziiert ist, einer Auxotrophie oder einem dominanten Merkmal für den Stamm entspricht, untereinander unabhängig von diesen mindestens drei Genen.

15. *Yarrowia*-Stamm nach Anspruch 14, **dadurch gekennzeichnet, dass** er ausgewählt ist aus der Gruppe, bestehend aus dem *Yarrowia lipolytica*-Stamm, eingetragen am 4. Februar 2008 gemäß dem Vertrag von Budapest in die Collection nationale de Culture de Microorganismes (CNCM), INSTITUT PASTEUR, 25 rue du Docteur Roux, 75724 PARIS CEDEX 15, Frankreich unter den Nummern CNCM I-3911 und CNCM I-3912, und dem *Yarrowia lipolytica*-Stamm, eingetragen am 4. Februar 2008 gemäß dem Vertrag von Budapest in die Collection nationale de Culture de Microorganismes (CNCM), INSTITUT PASTEUR, 25 rue du Docteur Roux, 75724 PARIS CEDEX 15, Frankreich unter der Nummer CNCM I-3913.

## Claims

1. Method for the targeted integration of at least three copies of a gene of interest in the genome of a *Yarrowia* strain including the following steps:
a) cultivation of a *Yarrowia* strain comprising at least three deletions chosen from Ura3-302, Leu2-270, Gut2-744 and Ade2-844, the phenotype associated with each of these deletions corresponds to an auxotrophy for this strain, independently of these at least three genes;
b) transformation of this auxotrophic and/or dominant character *Yarrowia* strain with at least three recombinant vectors that include selection markers allowing, for this *Yarrowia* strain, complementation of the auxotrophy resulting from each of these deletions, these recombinant vectors each including:
i. the sequence of said gene of interest,
ii. a selection marker, and
iii. two DNA sequences framing the sequence of said gene of interest and said selection marker, these two DNA sequences are homologous to the sequences corresponding to the ends of the targeted integration site in the genome of said *Yarrowia* strain in order to allow the targeted integration of said recombinant vector via homologous recombination;
selection on minimum medium of the yeasts having integrated the at least three recombinant vectors.

2. Targeted integration method according to claim 1, **characterised in that** said strain further comprises at least one deletion of a gene associated with a dominant character chosen from among the hygromycin-resistance gene HPH (HYG), and the MDR3, Kan MX and Tn5ble genes, the HYG gene being the most preferred.

3. Targeted integration method according to any of claims 1 to 2, **characterised in that** in step b), the targeted integration site in the genome of this *Yarrowia* strain is chosen from among the genes URA3, LEU2, ADE2, LIP2, LIP7, LIP8, AXP, GUT2 and XPR2.

4. Targeted integration method according to claim 3, **characterised in that** in step b), the targeted integration site in the genome of said *Yarrowia* strain is chosen from among the genes URA3, LEU2, ADE2, LIP2, LIP8 and AXP.

5. Method according to any of claims 1 to 4, **characterised in that** the steps b) transformation of the *Yarrowia* strain and c) selection on minimum medium are carried out separately for each of the at least three recombinant vectors.

6. Targeted integration method according to any of claims 1 to 4, **characterised in that** in step b), the transformations with at least the three recombinant vectors are carried out independently and successively with each of the recombinant vectors or simultaneously with all of these recombinant vectors and **in that** all of these transformations are followed by a step c) selection on a minimum medium of the yeasts having integrated all said recombinant vectors.

7. Method according to any of claims 1 to 6, **characterised in that** this strain is a strain of *Yarrowia lipolytica.*

8. Method according to any of claims 1 to 6, **characterised in that** said *Yarrowia* strain includes four genes having independent of one another a deletion associated with an auxotrophic or dominant character phenotype, preferably chosen from among the genes URA3, LEU2, GUT2 and ADE2.

9. Method according to any of claims 1 to 7, **characterised in that** step b) transformation is carried out with three to ten recombinant vectors.

10. Method according to any of claims 1 to 9, **characterised in that** the selection marker is framed by sequences allowing the excision thereof after the targeted integration of said recombinant vector.

11. Method for producing a polypeptide encoded by a gene of interest, **characterised in that** it includes the following steps:
d) cultivation of the modified *Yarrowia* strain obtained by means of the method according to any of claims 1 to 10 in a liquid culture medium containing assimilable sources of carbon, nitrogen and inorganic salts in order to allow the growth of said transformed *Yarrowia* strain; and
e) the recovery of this expressed polypeptide of interest from the *Yarrowia* cells or from the culture medium obtained in step d).

12. Method for obtaining a modified *Yarrowia* strain, **characterised in that** it includes a step wherein said *Yarrowia* strain is transformed with at least three vectors making it possible to arrive at a *Yarrowia* strain comprising at least three deletions chosen from among Ura3-302, Leu2-270, Gut2-744 and Ade2-844, the phenotype associated with each of these deletions corresponds to an auxotrophy or to a dominant character for said strain, independently of these at least three genes.

13. Method according to claim 12, **characterised in that** said strain further comprises at least one gene associated with a dominant character phenotype chosen from among the hygromycin-resistance gene HPH (HYG), and the MDR3, Kan MX and Tn5ble genes, the HYG gene being the most preferred.

14. Auxotrophic *Yarrowia* strain likely to be obtained by means of the method according to any of claims 12 and 13, said strain comprising at least three deletions chosen from among Ura3-302, Leu2-270, Gut2-744 and Ade2-844, the phenotype associated with each of these deletions corresponds to a dominant character for said strain, independently of these at least three genes.

15. *Yarrowia* strain according to claim 14, **characterised in that** it is chosen from the group consisting of the *Yarrowia lipolytica* strain deposited on February 4, 2008 in accordance with the Budapest Treaty with the Collection nationale de Culture de Microorganismes (CNCM), PASTEUR INSTITUTE, 25 rue du Docteur Roux, 75724 PARIS CEDEX 15, France, under the numbers CNCM I-3911 and CNCM I-3912 and the *Yarrowia lipolytica* strain deposited on February 4, 2008 in accordance with the Budapest Treaty with the Collection nationale de Culture de Microorganismes (CNCM), PASTEUR INSTITUTE, 25 rue du Docteur Roux, 75724 PARIS CEDEX 15, France under the number CNCM I-3913.


### SEQUENCE LISTING

<110> INSTITUT NATIONAL DE LA RECHERCHE AGRONOMIQUE (INRA) NICAUD, Jean-Marc FUDALEJ, Franck NEUVEGLISE, Cecile BECKERICH, Jean-Marie
<120> PROCEDE D'INTEGRATION CIBLEE DE MULTICOPIES D'UN GENE D'INTERET DANS UNE SOUCHE DE YARROWIA
<130> 354141D26198
<150> FR 0850736
   <151> 2008-02-05
<160> 77
<170> Patent n version 3.3
<210> 1
   <211> 4844
   <212> DNA
   <213> Yarrowia lipolytica
<400> 1
   acagtatcat taggaaaggg gtgacactgc actattggtt tgcttctgat gatacgtatg      60
   aatacgatgg tatcttgata cagagtcttg catacattaa tttcgaaact agagcacagc     120
   aacagctccc aaatgagcat tcccctgaag cccatcaaaa ctgcaccaca aatttgatcc     180
   catattacca ttttaaagca attgttaaca agatactact gtagaaaact gattaaacat     240
   tcgtaacttg cctgtggctc gacttacagc caaatgttcg cggcaaaatt tgaatatttt     300
   gcacacttat tgtcccaaaa ccaaataatg ccaacaaaat ccgatatagt gtaggggcta     360
   tcacatcacg ctctcatcaa gaatacttct tgagaaccgt ggagaccggg gttcgattcc     420
   ccgtatcgga gtgtttattt tttgctcaac cataccctgg ggtgtgttct gtggagcatt     480
   ctcacttttg gtaaacgaca ttgcttcaag tgcagcggaa tcaaaaagta taaagtgggc     540
   agcgagtata cctgtacaga ctgtaggcga taactcaatc caattacccc ccacaacatg     600
   actggccaaa ctgatctcaa gactttattg aaatcagcaa caccgattct caatgaaggc     660
   acatacttct tctgcaacat tcacttgacg cctaaagttg gtgagaaatg gaccgacaag     720
   acatattctg ctatccacgg actgttgcct gtgtcggtgg ctacaatacg tgagtcagaa     780
   gggctgacgg tggtggttcc caaggaaaag gtcgacgagt atctgtctga ctcgtcattg     840
   ccgcctttgg agtacgactc caactatgag tgtgcttgga tcactttgac gatacattct     900
   tcgttggagg ctgtgggtct gacagctgcg ttttcggcgc ggttggccga caacaatatc     960
   agctgcaacg tcattgctgg ctttcatcat gatcacattt ttgtcggcaa aggcgacgcc    1020
   cagagagcca ttgacgttct ttctaatttg gaccgatagc cgtatagtcc agtctatcta    1080
   taagttcaac taactcgtaa ctattaccat aacatatact tcactgcccc agataaggtt   1140
   ccgataaaaa gttctgcaga ctaaatttat ttcagtctcc tcttcaccac caaaatgccc   1200
   tcctacgaag ctcgagctaa cgtccacaag tccgcctttg ccgctcgagt gctcaagctc   1260
   gtggcagcca agaaaaccaa cctgtgtgct tctctggatg ttaccaccac caaggagctc   1320
   attgagcttg ccgataaggt cggaccttat gtgtgcatga tcaaaaccca tatcgacatc   1380
   attgacgact tcacctacgc cggcactgtg ctccccctca aggaacttgc tcttaagcac  1440
   ggtttcttcc tgttcgagga cagaaagttc gcagatattg gcaacactgt caagcaccag   1500
   taccggtgtc accgaatcgc cgagtggtcc gatatcacca acgcccacgg tgtacccgga   1560
   accggaatca ttgctggcct gcgagctggt gccgaggaaa ctgtctctga acagaagaag   1620
   gaggacgtct ctgactacga gaactcccag tacaaggagt tcctagtccc ctctcccaac   1680
   gagaagctgg ccagaggtct gctcatgctg gccgagctgt cttgcaaggg ctctctggcc   1740
   actggcgagt actccaagca gaccattgag cttgcccgat ccgaccccga gtttgtggtt   1800
   ggcttcattg cccagaaccg acctaagggc gactctgagg actggcttat tctgaccccc   1860
   ggggtgggtc ttgacgacaa gggagacgct ctcggacagc agtaccgaac tgttgaggat   1920
   gtcatgtcta ccggaacgga tatcataatt gtcggccgag gtctgtacgg ccagaaccga   1980
   gatcctattg aggaggccaa gcgataccag aaggctggct gggaggctta ccagaagatt   2040
   aactgttaga ggttagacta tggatatgta atttaactgt gtatatagag agcgtgcaag   2100
   tatggagcgc ttgttcagct tgtatgatgg tcagacgacc tgtctgatcg agtatgtatg   2160
   atactgcaca acctgtgtat ccgcatgatc tgtccaatgg ggcatgttgt tgtgtttctc   2220
   gatacggaga tgctgggtac agtgctaata cgttgaacta cttatactta tatgaggctc   2280
   gaagaaagct gacttgtgta tgacttattc tcaactacat ccccagtcac aataccacca   2340
   ctgcactacc actacaccaa aaccatgatc aaaccaccca tggacttcct ggaggcagaa   2400
   gaacttgtta tggaaaagct caagagagag aagccaagat actatcaaga catgtgtcgc   2460
   aacttcaagg aggaccaagc tctgtacacc gagaaacagg cctttgtcga cgaagctcta   2520
   aagctgaaac gggacatcga aaagttggag aagtcaatct ggaaactaca cgtagaaaac   2580
   agacaccatc tccaacaagt acaggacttc aaatttgcac tgaagacatt ctgtgggtgg   2640
   agccataagt aacaatcgcc tgctccagga acttaaaaaa gaggcattat aggccccttc   2700
   atctgaatat tctttgagta ccaactgccg ccaactcccc aatcgttaat tagtgttatc   2760
   tattctatct atgctattag cacatattta tcatgtgctg ctgttatgta cgggttcaat   2820
   atacaaatgc ttagtcctca aacaccttca taggtgggtc gtcttcctgg ccaaatcgaa   2880
   ttctcatgat ttcaaacacg aacagaccga tagacgtaga cggaagcgca ctgagggtcg 2940
   tccagccaca accatggtat aggtatctgg gccaactcaa ccgcagaccc tttcggatgt 3000
   tattggctgt atgcagagtc tcaacgtagg ctcggtagta gagatggata cccgggaaga 3060
   ttctgtatct aagagtagta aaacatctaa tagaaaagtt gacggtctta ctccatccct 3120
   taccaaactt cacaagaagt gaatgcgttc tgggaagagt ctcctccagc gtttcagaca 3180
   gttccaccat aagagggttt ctgtggtgaa tccgacgctc gtagcataga aatgttgact 3240
   ggaaaggaga ggcgggactg gcctgatgtt ttattcgctc tccaagtttg ttctgtgtct 3300
   tttgctcctt ggatcggtta gcttccagtt gttttcttgc gtcaatgagg tcttgagtat 3360
   cgagacgagt caggtagatg ccctgcagct tctgaagagg aaactgaacc acatgcagtg 3420
   agatggtggc tgctgctcca gctacaagga tgctgaaagg tgctaggacc ttgttgacga 3480
   tgctttttcc attctcatca aagcctgaag gatgttcctt gagacgatca gcatacagcc 3540
   acttggcata ggacctgtac cacgatccct taaccatttc gaacgtggca aaaaagaccc 3600
   caaaacccag actctctttg acaactgaca acgtaaagcc agcaaagaca cctggtgcac 3660
   ctgtttggcg caacttctcc attccatagg accacataga gggcaactgt ttggtcttga 3720
   gcacttctgc tatcgaagaa cgcctgtaga tggcgtcgat tggtgctgca gctactgcct 3780
   gggctgctcc agcgactgct ccggcacaaa acgtctgttt gaacgtgtat cgatgaggca 3840
   tgagttcatc gatgatgtat ttcacatgcg agccgtccat tccctgggta tcataccatg 3900
   atttccttag cagcagttgt cgttggtgtt cgtcttcttc atcctccttt tgtcggtctt 3960
   tctctgattt gaataatggt agacatccca gatatgtgga gtacaatacc agtcctacgg 4020
   ccgagttcgc caataagggg ggcagcaatt gatttggaat aaaaccccag ccatgctgtt 4080
   tgactgcgtt ggcaatcaga gccggactgc tgtgtgtttt gaagctccaa ggtttgtcgc 4140
   tgtttggaga cagcgcccgt ggaagcgcta agtagtcgac tctggtaggt cgaaagagct 4200
   tgacaggcac acggatgtaa aatgtgaaga gctgggatgc gagcgcccga gtaccggccg 4260
   tcgtagctcc aataatccgg gccgagcttg gtttccatgc ttcgttattg ttttcgtgcg 4320
   gttcggtgaa ttccggaacc tcgggcagaa atcccggaac gtcgccgggc tcgattttct 4380
   gcatgaggcc tagaaacact ctccagtgtc gcgggacgtg aggaacgggc ttctatctgc 4440
   tgagcgatat caggtactgg tcgtgcttgc ggttgtccag agatccaaaa cttttgcgat 4500
   gcgtcaggga cgcacagttt accgacaacg gaggagtgca aaacgtgggg gaacagggca 4560
   gtttgtgtgg gtgggtcggc gagtccgaat gatgattttg tatcgttaca gattgcgtgg 4620
   ggtggagatt aatatggctg ctcgaaaatt gctcgtgtcg cgttactgag aggcagagta 4680
   catgtacgcg agctctcctg tgtctactcc ttcacctctt taatgttgct gtacatatac 4740
   gcggtatgat agatgtggtc aggtggtgga tttgggaatg aaggtcggtt tgggctcggt 4800
   agtggtaatg agtgtcatgg gtgaggctta agtggcgaga gatc 4844
<210> 2
   <211> 968
   <212> DNA
   <213> Yarrowia lipolytica
<400> 2
   gaattccgtc gtcgcctgag tcatcattta tttaccagtt ggccacaaac ccttgacgat 60
   ctcgtatgtc ccctccgaca tactcccggc cggctgggta cgttcgatag cgctatcggc 120
   atcgacaagg tttgggtccc tagccgatac cgcactacct gagtcacaat cttcggaggt 180
   ttagtcttcc acatagcacg ggcaaaagtg cgtatatata caagagcgtt tgccagccac 240
   agattttcac tccacacacc acatcacaca tacaaccaca cacatccaca atggaacccg 300
   aaactaagaa gaccaagact gactccaaga agattgttct tctcggcggc gacttctgtg 360
   gccccgaggt gattgccgag gccgtcaagg tgctcaagtc tgttgctgag gcctccggca 420
   ccgagtttgt gttcgaggac cgactcattg gaggagctgc cattgagaag gagggcgagc 480
   ccatcaccga cgctactctc gacatctgcc gaaaggctga ctctattatg ctcggtgctg 540
   tcggaggcgc tgccaacacc gtatggacca ctcccgacgg acgaaccgac gtgcgacccg 600
   agcagggtct cctcaagctg cgaaaggacc tgaacctgta cgccaacctg cgaccctgcc 660
   agctgctgtc gcccaagctc gccgatctct cccccatccg aaacgttgag ggcaccgact 720
   tcatcattgt ccgagagctc gtcggaggta tctactttgg agagcgaaag gaggatgacg 780
   gatctggcgt cgcttccgac accgagacct actccgttcc tgaggttgag cgaattgccc 840
   gaatggccgc cttcctggcc cttcagcata acccccctct tcccgtgtgg tctcttgaca 900
   aggccaacgt gctggcctcc tctcgacttt ggcgaaagac tgttactcga gtcctcaagg 960
   acgaattc 968
<210> 3
   <211> 1698
   <212> DNA
   <213> Yarrowia lipolytica
<400> 3
   atggactcga aaacaattgg tattcttgga ggaggtcagc tgggccgaat ggtcgtggag 60
   gctgcccatc gactcaacgt caagactgtc attctagacg cccccaactc tcccgccaaa 120
   cagattcatg ctggagagca tgtcgatggc tccttcaagg accctgccga cattgctaag 180
   cttgcctcca agtgcgacgt cataaccgtt gaaattgagc atgtgaacgc cgacactctg   240
   gccgatctcc agaagactgg tgtcgaggtt cacccttctc ctgagaccat ccgaattatc   300
   caggacaagt accgacagaa ggagcacctg atctcccacg gcattcccgt tgccgaatcc 360
   tacgaggtca ccgagtctac tgaggctgcc cttgtcgttg ctgctgaaaa ggtcggctat 420
   ccttgtgttg tcaaggccaa gactcttgct tacgatggta gaggcaactt cgttcttaag 480
   ggtccagaga atgtcaagga agctcttgag ttcctctccg accgaccttt gtacgtagag 540
   aaatggtgtc ccttcgtcaa ggagctcgct gttatggttg tccgatctac cgacggaacc 600
   actgttgctt accccaccgt cgagactgtt cagaaaaaca acatctgcca tactgtctac 660
   gcccccgctc gagacgtttc tccttccacc ctgtacgagg ctcagctcgt tgccgagaac 720
   gccattaagt ccttcactgg cgctggtatc tttggtgtcg agatgttcct gctcgaaaac 780
   ggcacgattg ttctcaatga gattgctcct cgaccccaca actccggtca ttacacaaca 840
   gatgcctgct ccacctcgca gtttgaggcc catgtccgag ccattcttgg tctgcctctt 900
   ttccccgaga tggcccagat gcgaacagca gacactcatg ccattatgct gaacattcta 960
   ggaggtgaca agcccgacag cgagcttgag tactgcaagc ggtcactttc cgtacctggg 1020
   tccaccatcc atctctacgg caagaccacg agacccgagc gaaagatggg acacatcaac 1080
   attgttgcta gcagcatgga cgaatgcgag cgacggctgg ccaaaatatc tggagaggac 1140
   atcgaggagg caccaaagac tgtcgagggt gtagcaggaa cctctaagac ccccctcgtg 1200
   tctatcatta tgggctcgga ctccgatctt aaggtcatgt ctgccggagc cgacatcctg 1260
   cggaaatttg acattccctt cgaactgacc attgtgtctg cccaccgaac cgcccaccga 1320
   atggctaaat ttgccgctga ggttgccagc cgaggtgtca aggctgtcat tgccggagct 1380
   ggaggagccg ctcaccttcc cggaatggtt gcctcggaaa ccactctgcc agtcatcggt 1440
   gtccccgtta agggctcctc tctggacgga gttgactctc tctactccat tgtgcagatg 1500
   cctcgaggca tccccgtggc caccatgggt gtcaacaact ccaccaatgc tgctctgctg 1560
   gccatccgaa ttctggctgc ttacgacgcc accctacaca ttcgactggc cgagtacatc 1620
   aagaacatgg agaaggaggt tcttgccaag gccgacaagc tcgagaccat tggatacgag 1680
   cagtacctca accaatag 1698
<210> 4
   <211> 1839
   <212> DNA
   <213> Yarrowia lipolytica
<400> 4
   ttatttgtcc ttgggggtaa ggcccatgga gtagaggtac tcgacaccgg tgttgaattc 60
   ctgctccttt cgagcctcgt cccactggag ctccttggcc atgatctcaa tgacctcagg 120
   gagagcctcg taggcggcag cggcgttaag gaaagcaaga cgagttcgtc gagcaaggaa 180
   gtcgatggcg gtggtggcat actcgtactt catagagtac ttgcactcgg catcgaggta 240
   ggggtagggg tacaggattc gcttaccctt tgtgagagta gactggggga tcacaccggg 300
   ttcgggggtg ggcatctcag caagagaagc aacggtgaaa gctcgagatc cgtagttctc 360
   agaaaggtgc ttagcaacct cggggtcaag gtcctcctgc tggatcagat caatgtaagt 420
   gagaggagtc cagtccttag caccgatgag cttgacgtct cgggtgacgg ccttggcgga 480
   gatttcaggc ttgagaccga acttggcaat gcaggcatcg acagtctcct cagccatctg 540
   tcggtaagtg gtccactttc cgccagcaat ggtgacaaga ccagactcgg agtaggtgat 600
   gagatggttt cggacaagag actcggtgtt cttggcgtgg gggtcccgga caaggggtcg 660
   gattccggac caggcggcca gaacgtcctc tcgtcgcaca tcaaccttgc cctcaacgta 720
   gtgtcggacc tcgttgagaa tgaagtcaat gtcctcctcg gaggggatag ggttagcagt 780
   gatcttggta ggctggtcag tagtaccggc aagggtgttt ccctgccagg ggaggaagaa 840
   gataactcgg ccgtcagaag tagcggggtc aaggagtccc atcttcttgg gggagtagta 900
   accggggaga atgatgtgaa caccggagga aggagcacag atctccttgg tgttcttgtc 960
   gtccatctgt cgcagagagt cagtgaaggg tccagtagca ttaacgacac acttggcctt 1020
   gatgttgaag gatccagcgt ttccgtcagt atccttggca acaacaccgt tgagctggcc 1080
   attggcgccc ttggtgagct cggtgacctc gcaatggttc aggatggtgg cacccttctc 1140
   aacagcagtc ataataagag aaacgttcat tcgagagtcg ttctgggagc catcatagta 1200
   gacaatggcg cccttgagct tgtcatcgga aagcatgggg aaggcatcga gagcacggga 1260
   tcgggagagc atgtaagagg actcgaggtt ctgtcggccg gcaagcagat cgtagcactt 1320
   gacacccatc cagaagtagg gaagctgcca ccaggtgtag acggggatca tgatgggcag 1380
   agcaaaggtg aggtggggag caatgtcgag gaagaccttt cgctcgtgca gggcctcctt 1440
   gaccagctcg tactggttgt agtcgaggtt ccacacagcc ttctcgaggt atcggacacc 1500
   tccgtggatg agcttggtgg atcgggacga ggttccgcag gagaaatcgt ctcgctcgac 1560
   cagagcaacc ttgaggcctc gtgtgacagc gtcgagggcg ataccggatc cggtagctcc 1620
   tccaccaaca acgacgagat caaactcctc ggtcttcatc ttctcgagct gggcggctcg 1680
   agaggggggc tcggcggcca cggggggcac tgtgaatttg tgcttcttgt ggagctcctc 1740
   ctgggcctgg gcaggcacag acagggcgac ggctccagcg ccagcggctg ccacggcggc 1800
   ggcagcagcc cacgcgggtt ttcgaatggt tctgaacat 1839
<210> 5
   <211> 5304
   <212> DNA
   <213> Yarrowia lipolytica
<400> 5
   gatatcggta gcaaacagac acgaagaaga ggccttggag aatttggaaa caacatccat    60
   tcgtgctcct tgtttctgct tgccatgcaa atgcaacagg gggataccgg gctgcagagt   120
   tcggaaggtc tcgtagacgt atcgcacctg ttttgatgaa ctgaagaaaa cgatgatctt   180
   gaacttggtg tgtgttctca ggaaacccca cagtgtgtcc agcttgtctt gcagctccac   240
   gcagacatag ttctgttcga ggttcttggg tgtgctggaa gtggtgtccg ggttggcgga   300
   gatgtacttg ggtcggcgga gtgacaaacg ggccagatca ctgacgctct tggtctgtgt   360
   tgcgctgaaa agcagagtct gtctgtccac aggcaggttt tccaaaatgg catccatggt   420
   cttcttgaaa cccatatcga ggattcgatc ggcctcgtcc agcaccagca tcttcacgtt   480
   agaaagatca aacccgctag tttggtccat gtgttgtaac agtcgtccgg gtgtacaaat   540
   gagaatgttc agcttggcca gtcggtcagc ctccatggca acgtcctttc ctccaataac   600
   caaaccagca gagaagctgt gacaacggcc gatttttcgc agaacctgga aaatctggac    660
   cgccagttct cgagtcggac taataaccag ggctcctaaa ccatccacat cactccactt   720
   gtttcgaaac aggcactcca acacaggcac cagaaacgcc agagtcttac cacttccggt   780
   acgcgctgct ccaagcagat cgtggccttg cagagcggga gggatggccc gcttctgaac   840
   atcggtacag gtgacataat gtgaattttt tagaccctca atagtagcct cagacaaggg   900
   cagctcctca aacttggaca cctccttgtc ggcctcgtcc tctccaaagt tgtcgacgcg   960
   ctcctgcagc ttggagagct tctgtttgtg ttcctctcgt ttgattttac gaggatcggc  1020
   tcgtttcttc attttcgcca tatctcaagc tgtcggataa atttcttgga caaaaaaatc  1080
   taacttttcg tgcgccttca cttttatcag tctgaaaatt acacatgcat gcagcttgga  1140
   gtatggaggc gacagaggtg catgccagag tgcatgaatt agggcaacgg gggtgtgaag  1200
   aagggcgcaa aagatgtgac gacggtgcaa gacgacggta tactgcagac aaggtctttc  1260
   actgcaaaac tcctggttaa atcgtgggtc ccgcgtctcc gtatcatatg actttgggtc  1320
   acgtgactgc aagattttta aggtttcgaa ctcccaaatt gagtcacgtg tcgtctctct  1380
   gcaacatgag gtatcgtaaa ttaggaatga ctcgacctga caggccatca aggaccctga  1440
   tgcaactctg aaattagagg gaggaggtaa tgagaaacag tgctggattg tggtgagaca  1500
   ttgaagcggg attggttaac ctgtttcttt tttgtcgttt gctcttcatt attgcgattt  1560
   ttgtagtcat ttgattagat cagatcagca gtcccctcat gctgtcgttg atgggcgcgt  1620
   aatcagtgta acgctttgaa tgaggatggt gtgagtaata ctggtttcat tggggggggg  1680
   ggggggggta ctgttactcg aaaaacaaat catatgcatc tctgaacgtg ccggggtgtg   1740
   gcatgtttct cgcttcgaaa aataaatgtt ggctgttcca ggcttgtctc agacctgcaa 1800
   gaatcatact ggcgtaatct ccgccaggtc tggtcaggaa caagagagta aaagaaatgt 1860
   ttgagtctcg gctttcacat cctcggcact agataagcag caatatcaat gtcacattgc 1920
   ttgcttgtgc aggtcagggg gagggggatg tatatgatac aagcaggttc gcatctacag 1980
   aatctacagt atgtatctga tgatagcctt acacagcgca cgcagatgta gtttttgggt 2040
   tgaacagtgt cagcggaagc ggcgtagtgg ggtactgtag tacagaatca acggaatgaa 2100
   gtttacaact gcttctgtta ctgtagcctt tcactgttca aatgataacg ttggagaaga 2160
   gtgcaccctc tgttagatag gattacagtc cagacacaca ttggcaccga agtctgtagt 2220
   caaacattgg tctcagttgg tgtaatgaca ttcaatagac tgattctgca actcctacac 2280
   cttgcattgt acacccgaga ccccttgtac ggcccgcatt tctccttcac atgtcggcat 2340
   gtgtcttgtc tgtagccact tcaatgggat gcagtattag cactgtcgga cacacctcag 2400
   tcaatgggaa tggaatgcca tgctcctagc ggctcattgt ttgctaggaa atacagttaa 2460
   tagcacagat catagagtct aggctctata cgtcttagaa caaccattat ttgtttaaac 2520
   gacaacttgg taagcgaatt acagtaagta gctattgttt ctatctctgt cagtgttcgg 2580
   ataagtcctt agatcaccat actcaaagtg gtacgtttcg tcgcatcgga cgagaagtgt 2640
   gtctatcacc gtttcttttt gccgctctca tttttgcggt cggaataatt actgtggacc 2700
   ctcggcgccg accgttttgc agggctcatc aacctaaaac ttctccgagt ctgtgccttc 2760
   aggtgggcat agttgatggg tgttttgaag ttaatagtgg ggaagaacta tggcaaacaa 2820
   gcagatgcag gcaccttgta actgcagacc ggttcttgtc taccgactcc gctgcacctg 2880
   tgccgcggta catgtcgtca caggctgcgg ggttcggagg cccccttgca acctcctttg 2940
   atagttgcta tggccccaaa gagttatacg agatagaccc acagatctac ttgactgttg 3000
   tcacagaacc tgctaggttt gcttattgta cccgctttgt agctactgta caacgacaac 3060
   gtctaaaatt gagacgcgaa caaactccag atgcagaacc caaacctctc tctcagagtt 3120
   tcgagtgctt ctacctcaca gtaaagtgga ggtggacctg caagggaatt cagtcacaag 3180
   gccccgaatg tctccgaaac tccaatcgga ccgtttaaac agactaatat cacgtcattg 3240
   attgatatta gcatccggca agagccgcaa ggttatctcc tcaccaatga gcctgttgta 3300
   cggctcattc cgcatctgcg gctgattcag tttcgagtgg ggatggtaga cttcattgca 3360
   gcattcctaa ccttctactt ggtccgtgga gatgtcatgg acatcgattt tgggctgaga 3420
   agccttttga cgatgtcgat atcactgacc gctaatttac tctggcagtt tctccggctc 3480
   tcgaggcatc gtcgatcacc aaacactatc tgctagtcta aatgtccgac acgacagctt 3540
   ttgatcgccg tgaacggcgc agacctcatg caccatgcac cagggccaaa tcaattacgg 3600
   gtcgcttagc gttgcagtcg gggcattatg gtggaagttc cgatacggca cagacacatt 3660
   ccatagtggg gggattggat tataaaaggg ccatagaaag ccctcaattg atacccaagt 3720
   accagctccc ctcactatga agctttccac catccttttc acagcctgcg ctaccctggc 3780
   tgccgccctc ccttccccca tcactccttc tgaggccgca gttctccaga agcgagtgta 3840
   cacctctacc gagacctctc acattgacca ggagtcctac aacttctttg agaagtacgc 3900
   ccgactcgca aacattggat attgtgttgg tcccggcact aagatcttca agcccttcaa 3960
   ctgtggcctg caatgtgccc acttccccaa cgttgagctc atcgaggagt tccacgaccc 4020
   ccgtctcatc tttgatgttt ctggttacct cgctgttgat catgcctcca agcagatcta 4080
   ccttgttatt cgaggaaccc actctctgga ggacgtcata accgacatcc gaatcatgca 4140
   ggctcctctg acgaactttg atcttgctgc taacatctct tctactgcta cttgtgatga 4200
   ctgtcttgtc cacaatggct tcatccagtc ctacaacaac acctacaatc agatcggccc 4260
   caagctcgac tctgtgattg agcagtatcc cgactaccag attgctgtca ccggtcactc 4320
   tctcggagga gctgcagccc ttctgttcgg aatcaacctc aaggttaacg gccacgatcc 4380
   cctcgttgtt actcttggtc agcccattgt cggtaacgct ggctttgcta actgggtcga 4440
   taaactcttc tttggccagg agaaccccga tgtctccaag gtgtccaaag accgaaagct 4500
   ctaccgaatc acccaccgag gagatatcgt ccctcaagtg cccttctggg acggttacca 4560
   gcactgctct ggtgaggtct ttattgactg gcccctgatc caccctcctc tctccaacgt 4620
   tgtcatgtgc cagggccaga gcaataaaca gtgctctgcc ggtaacactc tgctccagca 4680
   ggtcaatgtg attggaaacc atctgcagta cttcgtcacc gagggtgtct gtggtatcta 4740
   agctatttat cactctttac aacttctacc tcaactatct actttaataa atgaatatcg 4800
   tttattctct atgattactg tatatgcgtt cctctaagac aaatcgaaac cagcatgcga 4860
   tcgaatggca tacaaaagtt tcttccgaag ttgatcaatg tcctgatagt caggcagctt 4920
   gagaagattg acacaggtgg aggccgtagg gaaccgatca acctgtctac cagcgttacg 4980
   aatggcaaat gacgggttca aagccttgaa tccttgcaat ggtgccttgg atactgatgt 5040
   cacaaactta agaagcagcc gcttgtcctc ttcctcgaaa ctctcaaaca cagtccagag 5100
   gtcctttata gcttgatctg tatccagata gcctccgtaa ttggtgtgtg tcttcaaatc 5160
   ccagacgtcc acattggcat gtcctccact gataagcatt tgaagttcat ctgcgttgaa 5220
   cattgagacc cacgaagggt caatgagctg gtatagaccg cccaagaatg catctgtctg 5280
   tgttctgata ctggtgttaa gctt 5304
<210> 6
   <211> 2092
   <212> DNA
   <213> Yarrowia lipolytica
<400> 6
   acgcgtgtga tcgcgagcga gtaacgtcca ccagtcttct gggagcccct ttcctccaaa    60
   aggcctgaca ttgacgcttt aaaaaagttg acatctgaaa caagcagccg ttgccggggg   120
   tctcattggt ataagacagg ataccgttga ttcaccggtc atggttcacg ctagtttgca   180
   accccccccc cgcagagatg caacctccac ctaaggaata tcaagacaaa agactcaagc   240
   acaagttgaa gcatggcaca agctccagcc cacatgccaa ggttcacagg tggatatatt   300
   gactggacct acattctttg tctgcgtgac agtttcgaga atgtgaagat acccaaagtg   360
   cggtgattat gatgctggca gggcctccca gggtctgttt caaatcgcta caaccacatg   420
   gctggattat ctactcattg cagatcagga acactttctg ttggctcccg cgtgcagata   480
   ttatcatcta actagtagct aaagtcgcgc tagcgcctct ctagcacagt catgcacgtt   540
   tagatctgtt cgggtcatgt caggtatacc tgtttattgg tatattgagg ggattaccag   600
   ttgaacaagc atgacggctg tggcggtaga tacacaaaga gaacgacttc tataagaggg   660
   actattcttg ccctctgggt catcatcaag cagcagcttt ccacccttct ctctcccaat   720
   ggtatccctc tctgctcgaa tcaaagactt tttttcggtc ctcctcctcg gagctgcaac   780
   catcactccc tccacacaga ccgcaggcgt gtctcaaggg ttctatgatt ttgctcggga   840
   ctttgcccat ctgtccaaca ttgcctactg tgtcaatgct cccatcactc cactgaaccc   900
   ggacttcacc tgtggcaact cgtgcaagca ctttccggaa attgagcttg tgaagacatt   960
   tggaggcaac ttcttcaaga cctccattac gggctacctg gctgtcgatc atgtcaagaa  1020
   ggagaagtac gttgtcttcc gaggaacctt ttcgctggca gacgcgatca cggacatgca  1080
   gttccagctg tctcctttcc tggtcgatgt gcctgccctg aacactttct cagctaatga  1140
   caccaccgca gaggcccaga cgcactgtga gggctgcaaa attcacgacg gcttctccaa  1200
   ggcctttacc gagacctggg gtaacattgg tgaggatctg cagaaacacc tggacgctaa  1260
   cccggactac cagctgtacg tgactggcca ttctctggga gctgctatgg cccttcttgg  1320
   agctacttcc atcaagctca agggctacga tcccattctc atcaactacg gacagccccg  1380
   agtcggaaac aagcccttcg ctgagttcat taacaagttg tggtttggag aaggcaacgg  1440
   tctggaaatc acccccgaga gaaagctgta ccgaatgacc cactggaacg acatctttgt  1500
   tggcctgccc aactgggagg gatacaccca ctctaacggt gaagtataca tcaacaaccg  1560
   gttcatcaac cctcctctca aggatgtcat ctcttgtgct ggaggcgaaa actcgaagtg  1620
   ctaccgatcc tcgttcagcc tgctgtccca gatcaatctg ctccaaaacc acctggctta  1680
   cattgattac attggatact gcgctctgaa cattggtcga cgagagcttg ccgatcagga 1740
   acattacact ggtccttatt actatggtca tcgatctgag gaggacttta agaagttggg 1800
   cttggagcta tccaccccac aagttgagaa ctgaacgagt tgacatgcct gcactactca 1860
   acaactaact acttgtagtc gtcactttac tattatctag ctgtaattgt atattaatat 1920
   atctaacgaa tataacagac ggctatagag tacaaccttc atcatacaac tcatgactga 1980
   gttcatttcc cgactaatga gactgacaaa tgttgggctt ggagggttgt tgggcttgga 2040
   ggactgttgg gattggagga ctgttgggct cgaagaggtt gatgtgtttt ct 2092
<210> 7
   <211> 3478
   <212> DNA
   <213> Yarrowia lipolytica
<400> 7
   tcctcgtcat aatgacgata aaatttatgc tatggtataa aatcactgtt ttacgtcagc     60
   gctgcccagc acaggtattc acatgtagct atgacaaacc acacggcata tgaacatgcg    120
   ataaggcaac aggaacattt ttttcagttg ttcctctcga atagggtcac tttatgctag    180
   gcttgtacta ttaacaactc ggcacctgca caagtctgaa ccccgacttg caacgaattg    240
   tggggtgcga gagggactac tacaggtaca acctgaacag taaaagaggt agttgagcag    300
   gcaacataac gtgatatcaa ccaggccaca ttagagaccc caagctgcgg gtgatctcta    360
   taggtgtcct tcgatatcac tcatcacttt tttggagtgt tggtcgaaag ggatgcacca    420
   ccggttacga gtacctggta aatttttccc gaggctgaca ttcctagatc agagttgtgt    480
   ctcattgata agagaagtga actacttgta gcagtgacag ttccctatac tgcttttcga    540
   actgtacgag ggcacacacg gcctccgaag aagcaaaagt gaacgccgaa gaagctcatt    600
   cgaggctatc agtgttctaa agatgttgat ctccttgtgc caatatgaac ggcaccagct    660
   ggttaatgtg gggttgagag tgcggaacct tgttcgaagg cccattctac tctgtacacg     720
   atttgcaacg gccagcgaga tatcaatgag tacggcgtgt atatgtctca gggagaaaaa    780
   aactccttcg gggtacggaa cctcaaacac cactgagctc ctaacggctc tagaggagag      840
   tcctcttgag cgattgagtc actgaggagc agggttccat caagtcgcag ataaagaaac    900
   aagcaaacaa cgatgccgtg tatctcacac ccacctccaa gacccttgga agaccagcca    960
   tgaaccttag cttccaccaa agcctgaacg gcacgaggcg atcgtggcaa tccacgtgca   1020
   agtgtaaatc agtgtttaat ctcgaagctt gcatggttta gcagggggcc gtcctatagt    1080
   aactgcacga tagcgccctg ccaagcgatg ccacgacggt gccaagaaac atggaatgtc   1140
   ctagttttgg agtggtgcgt tgcggattat gttagcgctg tgtgttactg ggggaataga   1200
   gatacgctcc ctcgcagctc agacgtcaag tgcacccata tactcggtcg agacccattt 1260
   ctcgtccttg tccaaagttt gaccctggct aaggcacatc agggcctatc gtgataaaca 1320
   agtactgtac atctgtatgt acgtgggggt actgcgaaaa ctgataacgt tgaaatacaa 1380
   tggcttgaaa tcggtgtcgg tctgagtgaa ttctagcgat tgtacggcaa gatatatatt 1440
   ggagtctctc aataacatat tgcttttgga tcaatggttt tgttggcttc tgctggacca 1500
   aatacacgtc ccttggtgag acaaacaaac acaccacgcc gttatcgccc accttatttg 1560
   ccttctaata ccgcactata ttggaacttt cgactcattg accatgcatc tcaccagagg 1620
   caatgactga ggaccaggtc gaagtagctg gcggaaaagt ggttgtacca gaaaacagat 1680
   tcacaagtac ctataaatca ctttgtgtgt gaggactctg gaactgctca cgagtggttc 1740
   caggctactg gtagttctat tcgagctcca aagcacatta agttacgctt ggtgaactat 1800
   agcttctggt cctctgcttg caatgaagct gtgggtggag taaacggtgc cgcttaatac 1860
   agggatggtg cgtgagatag gagatttgga gccgtctact ctgtcggcca acgacataaa 1920
   tagaccccct cagtcacctt agacacagca gaattccacc agatcagctt ccaattctaa 1980
   agatgcagtt ctctcttgcc acccttacca ccctcttggc cttcgttgcc gcagcccccg 2040
   caaacaaggg atttgttcat gcccctatca agaagcagtc cctccaggcg gcccagagca 2100
   agatccccaa ctttgcctcc tccggcccca tcactgccga gctctacaat gagctcatgg 2160
   cttaccaggt tcaaatttct cttggtggtc agactatctc tgccagcatc gataccggat 2220
   ccgagattct gtgggtttgg gagaacgact ctattgcttg ccaggttgac cagcaggact 2280
   gcgacactga cggctcttac aaccccaaga agagttccac gagcaaggac accggagtgc 2340
   ctttcaacat caactatggc aagggacatg ctgatggtta cctctacact gataatgctg 2400
   tcattggtgg agcttccgct cctggtttca agtttggtgt caactctggt gatctcagct 2460
   ccggcggctt ctccatggtc ttcggtatcg gtgtgaacag cgacgcctct acttccatct 2520
   ctgctcagct ccaaaagtcc ggcgagatct cccgaaacct ttacggtatg tcattcagcg 2580
   acgccaacct tgctggcacc agcaacgaca actctgagat cacctttggt gctatcaaca 2640
   ctggtcgata cactggctcg cttaagacca ttccccgagt tgcgacccaa ggcggatacc 2700
   agcacttttc cgtttctgcc tccggaaagt ttggagatgt cgacctcttc gacaatgacc 2760
   tcgtcattct agactctgga accaccatga cttacctcaa gtcggactac tacaacgcct 2820
   tcctcggcgg cctcgaggac cttgatatca ccctcagtga ctactctgga ggatggcacg 2880
   gatacccttg ctctgaaaac tcgaagatca acttcactta caacttcagc ggtaaggaaa 2940
   tcactgtgac tggacacgac ctggccattc ccggcaacgc tgtcaactcc aatgttgact 3000
   cctcagtgtg tttcatgggt gttgacgatg gtggaaacat gaacctgttc ggtgacacct 3060
   tcctgcgagc catttactcc gtctacgatc tcgagcgaga tgaggtctct attgcccagg 3120
   ctgcccatgg caagcccgac aactacgtgg tcatcactgg tgatgtcccc aactagacta 3180
   ttattatcta gcatttcttc tacaagaagc cttatggcaa ctatataatt taattcatta 3240
   aaaatgttct atgctaaatg gtgaggcagt gattcaattg caggttcaac attgttggat 3300
   gcaattcaag ttttcaatgc gcgcggtgtt gtattcggcc cagaggaggt ccaaagacga 3360
   gtggaacatg atcgccaggt ggagcctttg gactgaaata atcaaagagc tcattcaatt 3420
   ggttttgtag ctgctcaaac tccttttgaa cggtctcaag ctgcttctcc accccaca 3478
<210> 8
   <211> 2966
   <212> DNA
   <213> Yarrowia lipolytica
<400> 8
   gttcgtgccg agctcttgtt ctaccgattc agacgtacgg tcgagcttat tgaccgaaag     60
   aacgaggaca gacgcaactc agcggacggc tccgagcctg tttccatcac agaggacctg    120
   cgggaattgt tatctcggaa agtcattgtt gtgcgtgagg gtgagcgtcc tgaaggcgta    180
   atgggtgggt aggtaatgca gtttgcatgc atgaagacac taaacaagcc aaccatacag    240
   cagaagtatg tagccttgca catgatttat tgacaggcca cccaaacagg cgtatgtata    300
   gtactgtacc ttcagtagac tattgtagct aacatgtcgt tgcgtggcgt atgtaccaag    360
   ccacagaaat tatgtcagag ataaggtcgc gacagttaga gcagcaacgc gtggagagtt    420
   tgggttttgg gttacgtacg tagagccgtt tgatagatgg tacatccacc ggctagcgga    480
   acacagtgtc aagacaagcc tgcaacacag tcataatatt tgcgatattc aggcgtatca    540
   ggtacaatct gaggtgtctc acaagtgccg tgcagtcccg cccccacttg cttctctttg    600
   tgtgtagtgt acgtacatta tcgagaccgt tgttcccgcc cacctcgatc cggggtccta    660
   tgcatccctg aaacattgat tggaaattaa catatgagct gcgtgctttt tgcattcaag    720
   ggcgcagctt atcttgtatc cttaattaca catgacctct tgagcgccac ggtacattcc    780
   tggcgtcagt tcggtggagc ggacactttt ctctcctttg tctgacatgt tggttaagtt    840
   gtagtccagg gacacaaggg gttccaacgg cagtggcagc ctaccccacg ctacccacca    900
   ctggccctgg tctaacttcg acgatcggca tcagggttca tggataggtg gtgtgattta    960
   cgatgtgatg gacaatgtta gagagatccc actacttgta gtcaggccat cttttacgta   1020
   cgcactgtac catgatgtca atggagtatg atgaaccgac tttgagagac tcacatctgc   1080
   acaacaccat gtttcagcgg aatccgactt ccaacccaaa cccaagcccc tgtcagatat   1140
   cgtgagaagg cacggcacca actaatgcac acactccacc tgtattgcac caagataatg   1200
   agggcatcgt cttggcgcgt cttggcgaga gccgtgtttc gtgacgcaat cagagcagtt   1260
   tctggatagt atcttgtcca gaaacacgat ataaacccca tcgacgggcc cgttgaagag   1320
   caccaaccca ctatccaatc ctccaatcca acaatgaagc tcgctaccgc ctttactatt   1380
   ctcactgccg ttctggccgc tcccctggcc gcccctgccc ctgctcctga tgctgcccct   1440
   gctgctgtgc ctgagggccc tgccgccgct gcctactcat ctattctgtc cgtggtcgct   1500
   aagcagtcca agaagtttaa gcaccacaag cgagatcttg atgagaagga tcagttcatc   1560
   gttgtctttg acagtagcgc tactgttgac cagatcgcct ccgaaatcca gaagctggac   1620
   tctctggtcg acgaggactc gtccaacggt atcacctctg ctcttgatct tcctgtctac   1680
   acggatggat ctggctttct cggatttgtt ggaaagttca actccactat cgttgacaag   1740
   ctcaaggagt cgtctgttct gacggtcgag cccgatacca ttgtgtctct ccccgagatt   1800
   cctgcttctt ctaatgccaa gcgagctatc cagactactc ccgtcactca atggggcctg   1860
   tctagaatct ctcataagaa ggcccagact ggaaactacg cctacgttcg agagacagtt   1920
   ggcaagcacc ccaccgtttc ttacgttgtt gactctggta tccgaaccac ccactccgag   1980
   ttcggaggcc gagctgtctg gggagccaac ttcgctgaca cacagaacgc tgatcttctc   2040
   ggtcacggca ctcacgttgc aggtaccgtg ggaggaaaga catacggagt cgacgccaac   2100
   accaagctgg tggccgtcaa ggtgtttgca ggccgatccg cagctctctc cgtcatcaac   2160
   cagggcttca cctgggctct caacgactac atctccaagc gagacactct gcctcgagga   2220
   gtgctgaact tctctggagg aggacccaag tccgcttccc aggacgccct atggtctcga   2280
   gctacccagg agggtctgct tgtcgccatc gctgcgggaa acgatgccgt ggacgcctgt   2340
   aacgactctc ccggtaacat tggaggctcc acctctggta tcatcactgt gggttccatt   2400
   gactctagcg ataagatctc cgtctggtcc ggtggacagg gatccaacta cggaacttgt   2460
   gttgatgtct ttgcccccgg ctccgatatc atctctgcct cttaccagtc cgactctggt   2520
   actttggtct actccggtac ctccatggcc tgtccccacg ttgccggtct tgcctcctac   2580
   tacctgtcca tcaatgacga ggttctcacc cctgcccagg tcgaggctct tattactgag   2640
   tccaacaccg gtgttcttcc caccaccaac ctcaagggct ctcccaacgc tgttgcctac   2700
   aacggtgttg gcatttaggc aattaacaga tagtttgccg gtgataattc tcttaacctc   2760
   ccacactcct ttgacataac gatttatgta acgaaactga aatttgacca gatattgttg   2820
   taaatagaaa atctggcttg taggtggcaa aatgcggcgt ctttgttcat caattccctc   2880
   tgtgactact cgtcatccct ttatgttcga ctgtcgtatt tcttattttc catacatatg   2940
   caagtgagat gcccgtgtcc gaattc                                        2966
<210> 9
   <211> 173
   <212> PRT
   <213> Erwinia chrysanthemi
<400> 9
   Met Thr Glu Pro Ile Phe Met Val Gly Ala Arg Gly Cys Gly Lys Thr
   1               5                   10                  15
   Thr Val Gly Arg Glu Leu Ala Arg Ala Leu Gly Tyr Glu Phe Val Asp
               20                  25                  30
   Thr Asp Ile Phe Met Gln His Thr Ser Gly Met Thr Val Ala Asp Val
           35                  40                  45
   Val Ala Ala Glu Gly Trp Pro Gly Phe Arg Arg Arg Glu Ser Glu Ala
       50                  55                  60
   Leu Gln Ala Val Ala Thr Pro Asn Arg Val Val Ala Thr Gly Gly Gly
   65                  70                  75                  80
   Met Val Leu Leu Glu Gln Asn Arg Gln Phe Met Arg Ala His Gly Thr
                   85                  90                  95
   Val Val Tyr Leu Phe Ala Pro Ala Glu Glu Leu Ala Leu Arg Leu Gln
               100                 105                 110
   Ala Ser Pro Gln Ala His Gln Arg Pro Thr Leu Thr Gly Arg Pro Ile
           115                 120                 125
   Ala Glu Glu Met Glu Ala Val Leu Arg Glu Arg Glu Ala Leu Tyr Gln
       130                 135                 140
   Asp Val Ala His Tyr Val Val Asp Ala Thr Gln Pro Pro Ala Ala Ile
   145                 150                 155                 160
   Val Cys Glu Leu Met Gln Thr Met Arg Leu Pro Ala Ala
                   165                 170
<210> 10
   <211> 348
   <212> PRT
   <213> Escherichia coli
<400> 10
   Met Glu Phe Phe Lys Lys Thr Ala Leu Ala Ala Leu Val Met Gly Phe
   1               5                   10                  15
   Ser Gly Ala Ala Leu Ala Leu Pro Asn Ile Thr Ile Leu Ala Thr Gly
               20                  25                  30
   Gly Thr Ile Ala Gly Gly Gly Asp Ser Ala Thr Lys Ser Asn Tyr Thr
           35                  40                  45
   Val Gly Lys Val Gly Val Glu Asn Leu Val Asn Ala Val Pro Gln Leu
       50                  55                  60
   Lys Asp Ile Ala Asn Val Lys Gly Glu Gln Val Val Asn Ile Gly Ser
   65                  70                  75                  80
   Gln Asp Met Asn Asp Asn Val Trp Leu Thr Leu Ala Lys Lys Ile Asn
                   85                  90                  95
   Thr Asp Cys Asp Lys Thr Asp Gly Phe Val Ile Thr His Gly Thr Asp
               100                 105                 110
   Thr Met Glu Glu Thr Ala Tyr Phe Leu Asp Leu Thr Val Lys Cys Asp
           115                 120                 125
   Lys Pro Val Val Met Val Gly Ala Met Arg Pro Ser Thr Ser Met Ser
       130                 135                 140
   Ala Asp Gly Pro Phe Asn Leu Tyr Asn Ala Val Val Thr Ala Ala Asp
   145                 150                 155                 160
   Lys Ala Ser Ala Asn Arg Gly Val Leu Val Val Met Asn Asp Thr Val
                   165                 170                 175
   Leu Asp Gly Arg Asp Val Thr Lys Thr Asn Thr Thr Asp Val Ala Thr
               180                 185                 190
   Phe Lys Ser Val Asn Tyr Gly Pro Leu Gly Tyr Ile His Asn Gly Lys
           195                 200                 205
   Ile Asp Tyr Gln Arg Thr Pro Ala Arg Lys His Thr Ser Asp Thr Pro
       210                 215                 220
   Phe Asp Val Ser Lys Leu Asn Glu Leu Pro Lys Val Gly Ile Val Tyr
   225                 230                 235                 240
   Asn Tyr Ala Asn Ala Ser Asp Leu Pro Ala Lys Ala Leu Val Asp Ala
                   245                 250                 255
   Gly Tyr Asp Gly Ile Val Ser Ala Gly Val Gly Asn Gly Asn Leu Tyr
   260 265 270
   Lys Ser Val Phe Asp Thr Leu Ala Thr Ala Ala Lys Thr Gly Thr Ala
   275 280 285
   Val Val Arg Ser Ser Arg Val Pro Thr Gly Ala Thr Thr Gln Asp Ala
   290 295 300
   Glu Val Asp Asp Ala Lys Tyr Gly Phe Val Ala Ser Gly Thr Leu Asn
   305 310 315 320
   Pro Gln Lys Ala Arg Val Leu Leu Gln Leu Ala Leu Thr Gln Thr Lys
   325 330 335
   Asp Pro Gln Gln Ile Gln Gln Ile Phe Asn Gln Tyr
   340 345
<210> 11
   <211> 109
   <212> DNA
   <213> artificial sequence
<220>
   <223> PCR fragment
<400> 11
   tgcagctttc gagaaccgac gcctgggacc tgtgtctgta gggataacag ggtaattcgc 60
   ttcggataac tcctgctata acgaagttat gtagggataa cagggtaat 109
<210> 12
   <211> 98
   <212> DNA
   <213> artificial sequence
<220>
   <223> PCR fragment
<400> 12
   tagggataac agggtaatta tcgcttcgga taactcctgc tatacgaagt tatgtaggga 60
   taacagggta attaactata acggtcctaa ggtagcga 98
<210> 13
   <211> 35
   <212> DNA
   <213> artificial sequence
<220>
   <223> amorce PCR
<400> 13
   gatctgctag tgtataagac tctataaaaa gggcc 35
<210> 14
   <211> 27
   <212> DNA
   <213> artificial sequence
<220>
   <223> amorce PCR
<400> 14
   ctttttatag agtcttatac actagca 27
<210> 15
   <211> 31
   <212> DNA
   <213> artificial sequence
<220>
   <223> amorce PCR
<400> 15
   ccggaattcc tgaccagtct cacatccgac c 31
<210> 16
   <211> 41
   <212> DNA
   <213> artificial sequence
<220>
   <223> amorce PCR
<400> 16
   ccggaattct aaagcagata ctcaacaact cagcaatagt c 41
<210> 17
   <211> 23
   <212> DNA
   <213> artificial sequence
<220>
   <223> amorce PCR
<400> 17
   ggagctaccg gctccggtat cgc 23
<210> 18
   <211> 23
   <212> DNA
   <213> artificial sequence
<220>
   <223> amorce PCR
<400> 18
   gcgataccgg agccggtagc tcc 23
<210> 19
   <211> 31
   <212> DNA
   <213> artificial sequence
<220>
   <223> amorce PCR
<400> 19
   cggaaacgct ggatctttca acatcaaggc c 31
<210> 20
   <211> 31
   <212> DNA
   <213> artificial sequence
<220>
   <223> amorce PCR
<400> 20
   ggccttgatg ttgaaagatc cagcgtttcc g 31
<210> 21
   <211> 26
   <212> DNA
   <213> artificial sequence
<220>
   <223> amorce PCR
<400> 21
   ggagcaggag ttcaacaccg gtgtcg 26
<210> 22
   <211> 26
   <212> DNA
   <213> artificial sequence
<220>
   <223> amorce PCR
<400> 22
   cgacaccggt gttgaactcc tgctcc 26
<210> 23
   <211> 20
   <212> DNA
   <213> artificial sequence
<220>
   <223> amorce PCR
<400> 23
   gcagatccac tgtcaagccg 20
<210> 24
   <211> 45
   <212> DNA
   <213> artificial sequence
<220>
   <223> amorce PCR
<400> 24
   gctagggata aacagggtaa tgcggtagga aagagaagtt ccgcg 45
<210> 25
   <211> 41
   <212> DNA
   <213> artificial sequence
<220>
   <223> amorce PCR
<400> 25
   gcattaccct gttatcccta gccggactat ttccccgcag c 41
<210> 26
   <211> 21
   <212> DNA
   <213> artificial sequence
<220>
   <223> amorce PCR
<400> 26
   gcagccagca gcacgtagta g 21
<210> 27
   <211> 20
   <212> DNA
   <213> artificial sequence
<220>
   <223> amorce PCR
<400> 27
   gaatgacggg ggcaacgcag 20
<210> 28
   <211> 25
   <212> DNA
   <213> artificial sequence
<220>
   <223> amorce PCR
<400> 28
   cagcagccac aaatagcaga ctgcc 25
<210> 29
   <211> 39
   <212> DNA
   <213> artificial sequence
<220>
   <223> amorce PCR
<400> 29
   ccggaattcc gtgacaacgg atgatgctgt cacatgacg 39
<210> 30
   <211> 37
   <212> DNA
   <213> artificial sequence
<220>
   <223> amorce PCR
<400> 30
   ccggaattcc tgttggcatg cacagctcca ctcaacg 37
<210> 31
   <211> 32
   <212> DNA
   <213> artificial sequence
<220>
   <223> amorce PCR
<400> 31
   tcccgctagc cgaggtatgc aaggacgagt gc 32
<210> 32
   <211> 48
   <212>. DNA
   <213> artificial sequence
<220>
   <223> amorce PCR
<400> 32
   ataagaatgc ggccgcgcga gctgagagcc gataccaagg gatgcgag 48
<210> 33
   <211> 48
   <212> DNA
   <213> artificial sequence
<220>
   <223> amorce PCR
<400> 33
   cattaccctg ttatccctac agacacaggt cccaggcgtc ggttctcg 48
<210> 34
   <211> 77
   <212> DNA
   <213> artificial sequence
<220>
   <223> amorce PCR
<400> 34
   ctagggataa cagggtaatt aactataacg gtcctaaggt agcgacctcg gaaaccactc 60
   tgccagtcat cggtgtc 77
<210> 35
   <211> 51
   <212> DNA
   <213> artificial sequence
<220>
   <223> amorce PCR
<400> 35
   atagtttagc ggccgcggaa gcgtgtcaac gacatgttcc ctcttcatac c 51
<210> 36
   <211> 35
   <212> DNA
   <213> artificial sequence
<220>
   <223> amorce PCR
<400> 36
   cgacgataga gcaggtctca ctgttgggaa tgctg 35
<210> 37
   <211> 35
   <212> DNA
   <213> artificial sequence
<220>
   <223> amorce PCR
<400> 37
   ctacactgac gaagtggaca tcccggcttg gactg 35
<210> 38
   <211> 41
   <212> DNA
   <213> artificial sequence
<220>
   <223> amorce PCR
<400> 38
   cgcgaattcg cctgcttgaa agaagtgagt ggtatgctcg g 41
<210> 39
   <211> 42
   <212> DNA
   <213> artificial sequence
<220>
   <223> amorce PCR
<400> 39
   cgcgaattcc attgccacga cctgttaaaa gacaagatga cc 42
<210> 40
   <211> 34
   <212> DNA
   <213> artificial sequence
<220>
   <223> amorce PCR
<400> 40
   gctggccatc cgaatcctgg ctgcttacga cgcc 34
<210> 41
   <211> 34
   <212> DNA
   <213> artificial sequence
<220>
   <223> amorce PCR
<400> 41
   ggcgtcgtaa gcagccagga ttcggatggc cagc 34
<210> 42
   <211> 43
   <212> DNA
   <213> artificial sequence
<220>
   <223> amorce PCR
<400> 42
   ataagaatgc ggccgcgtgg tacgtttcgt cgcatcggac gag 43
<210> 43
   <211> 48
   <212> DNA
   <213> artificial sequence
<220>
   <223> amorce PCR
<400> 43
   cattaccctg ttatccctag agagctggta cttgggtatc aattgagg 48
<210> 44
   <211> 71
   <212> DNA
   <213> artificial sequence
<220>
   <223> amorce PCR
<400> 44
   ctagggataa cagggtaatt aactataacg gtcctaaggt agcgacctga tccaccctcc 60
   tctctccaac g 71
<210> 45
   <211> 44
   <212> DNA
   <213> artificial sequence
<220>
   <223> amorce PCR
<400> 45
   atagtttagc ggccgcgtgg acacaaggaa gtatgcggtc gtcg 44
<210> 46
   <211> 34
   <212> DNA
   <213> artificial sequence
<220>
   <223> amorce PCR
<400> 46
   ctctgtcagt gttcggataa gtccttagat cacc 34
<210> 47
   <211> 33
   <212> DNA
   <213> artificial sequence
<220>
   <223> amorce PCR
<400> 47
   cctcacttct gtcacagatg atgcattcaa cac 33
<210> 48
   <211> 47
   <212> DNA
   <213> artificial sequence
<220>
   <223> amorce PCR
<400> 48
   ataagaatgc ggccgcgaca ctactctggc tacagcttgc ggtactg 47
<210> 49
   <211> 46
   <212> DNA
   <213> artificial sequence
<220>
   <223> amorce PCR
<400> 49
   cattaccctg ttatccctag ctcgaataac attcacaggc ttggtg 46
<210> 50
   <211> 75
   <212> DNA
   <213> artificial sequence
<220>
   <223> amorce PCR
<400> 50
   ctagggataa cagggtaatt aactataacg gtcctaaggt agcgaggtgt gtttgtagtg 60
   gaggacagtg gtacg 75
<210> 51
   <211> 45
   <212> DNA
   <213> artificial sequence
<220>
   <223> amorce PCR
<400> 51
   atagtttagc ggccgcgtac tgaaccgact ttggtgcttg cactc 45
<210> 52
   <211> 33
   <212> DNA
   <213> artificial sequence
<220>
   <223> amorce PCR
<400> 52
   ctccacgcta atgcccatca tactctgttt ggc 33
<210> 53
   <211> 35
   <212> DNA
   <213> artificial sequence
<220>
   <223> amorce PCR
<400> 53
   cctggcgtta caaagccgag ggagacagcc ttgac 35
<210> 54
   <211> 48
   <212> DNA
   <213> artificial sequence
<220>
   <223> amorce PCR
<400> 54
   ataagaatgc ggccgcggtg acactgcact attggtttgc ttctgatg 48
<210> 55
   <211> 49
   <212> DNA
   <213> artificial sequence
<220>
   <223> amorce PCR
<400> 55
   cattaccctg ttatccctag tcgagcttcg taggagggca ttttggtgg 49
<210> 56
   <211> 70
   <212> DNA
   <213> artificial sequence
<220>
   <223> amorce PCR
<400> 56
   ctagggataa cagggtaatt aactataacg gtcctaaggt agcgatacgg agatgctggg 60
   tacaagtagc 70
<210> 57
   <211> 45
   <212> DNA
   <213> artificial sequence
<220>
   <223> amorce PCR
<400> 57
   atagtttagc ggccgcctat gctacgagcg tcggattcac cacag 45
<210> 58
   <211> 34
   <212> DNA
   <213> artificial sequence
<220>
   <223> amorce PCR
<400> 58
   ggcacactgc tcactatcgc aggctgcaac aatg 34
<210> 59
   <211> 36
   <212> DNA
   <213> artificial sequence
<220>
   <223> amorce PCR
<400> 59
   cctgactcgt ctcgatactc aagacctcat tgacgc 36
<210> 60
   <211> 31
   <212> DNA
   <213> artificial sequence
<220>
   <223> amorce PCR
<400> 60
   ggccgctaac tataacggtc ctaaggtagc g 31
<210> 61
   <211> 38
   <212> DNA
   <213> artificiel sequence
<220>
   <223> amorce PCR
<400> 61
   ccgaatcgat tcgctacctt aggaccgtta tagttagc 38
<210> 62
   <211> 39
   <212> DNA
   <213> artificial sequence
<220>
   <223> amorce PCR
<400> 62
   aatcgattcg gatcccacaa tgaagcttcc ccgcggtac 39
<210> 63
   <211> 32
   <212> DNA
   <213> artificial sequence
<220>
   <223> amorce PCR
<400> 63
   ctaggtaccg cggggaagct tcattgtggg at 32
<210> 64
   <211> 72
   <212> DNA
   <213> artificial sequence
<220>
   <223> amorce PCR
<400> 64
   tatcaaatgc ggccgcttcg ctaccttagg accgttatag ttaacacgat tcgatttgtc 60
   ttagaggaac gc 72
<210> 65
   <211> 31
   <212> DNA
   <213> artificial sequence
<220>
   <223> amorce PCR
<400> 65
   gagtaatgct cgagtatcga agtcttgtac c 31
<210> 66
   <211> 51
   <212> DNA
   <213> artificial sequence
<220>
   <223> amorce PCR
<400> 66
   gcgatattac cctgttatcc ctagaatcga ttcccacaag acgaacaagt g 51
<210> 67
   <211> 51
   <212> DNA
   <213> artificial sequence
<220>
   <223> amorce PCR
<400> 67
   cacttgttcg tcttgtggga atcgattcta gggataacag ggtaatatcg c 51
<210> 68
   <211> 51
   <212> DNA
   <213> artificial sequence
<220>
   <223> amorce PCR
<400> 68
   cgcggatccc acaatgaagc tttccaccat ccttttcaca gcctgcgcta c 51
<210> 69
   <211> 24
   <212> DNA
   <213> artificial sequence
<220>
   <223> amorce PCR
<400> 69
   tcgcttctgg agaactgcgg cctc 24
<210> 70
   <211> 52
   <212> DNA
   <213> artificial sequence
<220>
   <223> amorce PCR
<400> 70
   gaggccgcag ttctgcagaa gcgagccgac aagctgccca acattgtgat tc 52
<210> 71
   <211> 37
   <212> DNA
   <213> artificial sequence
<220>
   <223> amorce PCR
<400> 71
   ccacctaggc tagtaggtgt ggaagtactc ctggatg 37
<210> 72
   <211> 18
   <212> DNA
   <213> artificial sequence
<220>
   <223> amorce PCR
<400> 72
   tactcttggt cagcccat 18
<210> 73
   <211> 18
   <212> DNA
   <213> artificial sequence
<220>
   <223> amorce PCR
<400> 73
   agaagggcac ttgaggga 18
<210> 74
   <211> 17
   <212> DNA
   <213> artificial sequence
<220>
   <223> amorcé PCR
<400> 74
   tattgccgag cgaatgc 17
<210> 75
   <211> 19
   <212> DNA
   <213> artificial sequence
<220>
   <223> amorce PCR
<400> 75
   cttggagatc cacatctgc 19
<210> 76
   <211> 18
   <212> DNA
   <213> artificial sequence
<220>
   <223> amorce PCR
<400> 76
   gctgaacgac cgaattgg 18
<210> 77
   <211> 17
   <212> DNA
   <213> artificial sequence
<220>
   <223> amorce PCR
<400> 77
   gtggtgtgca gcttgtc 17